(19) Europäisches Patentamt
European Patent Office
Office européen des brevets

(11) **EP 4 279 502 A1**

(12) **EUROPEAN PATENT APPLICATION**
published in accordance with Art. 153(4) EPC

(43) Date of publication:
**22.11.2023 Bulletin 2023/47**

(21) Application number: **22739532.4**

(22) Date of filing: **18.01.2022**

(51) International Patent Classification (IPC):
***C07K 16/00*** *(2006.01)* ***A61K 39/395*** *(2006.01)*
***A61K 47/54*** *(2017.01)* ***A61K 47/65*** *(2017.01)*
***A61K 47/68*** *(2017.01)* ***A61P 43/00*** *(2006.01)*
***C07K 1/00*** *(2006.01)* ***C07K 14/00*** *(2006.01)*
***C07K 19/00*** *(2006.01)* ***C12N 15/13*** *(2006.01)*
***C12P 21/08*** *(2006.01)*

(52) Cooperative Patent Classification (CPC):
**A61K 39/395; A61K 47/54; A61K 47/65; A61K 47/68; A61P 43/00; C07K 1/00; C07K 14/00; C07K 16/00; C07K 19/00**

(86) International application number:
**PCT/JP2022/001617**

(87) International publication number:
**WO 2022/154127 (21.07.2022 Gazette 2022/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.01.2021 JP 2021005763**

(71) Applicant: **AJINOMOTO CO., INC.**
**Chuo-ku**
**Tokyo 104-8315 (JP)**

(72) Inventors:
- **FUJII, Tomohiro**
**Kawasaki-shi, Kanagawa 210-8681 (JP)**
- **YAMADA, Kei**
**Kawasaki-shi, Kanagawa 210-8681 (JP)**
- **MATSUDA, Yutaka**
**Kawasaki-shi, Kanagawa 210-8681 (JP)**
- **HIRAMA, Ryusuke**
**Kawasaki-shi, Kanagawa 210-8681 (JP)**
- **HATADA, Noriko**
**Kawasaki-shi, Kanagawa 210-8681 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **COMPOUND OR SALT THEREOF, AND ANTIBODY OBTAINED THEREFROM**

(57) The present invention provides a desired antibody or antibody composition by modifying a thiol group chemically introduced to an antibody. More specifically, the present invention provides an antibody composition comprising
(A) an antibody intermediate or a salt thereof having a bioorthogonal functional group which may be protected, which is represented by the following formula (A):

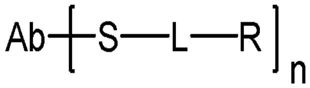 (A)

wherein
Ab is a certain antibody,
S is a sulfur atom,
L is a divalent group,
R is a bioorthogonal functional group which may be protected, and
n is an integer from 1 to 8, and

(B) a thiol group-introduced antibody or a salt thereof, which is represented by the following formula (B):

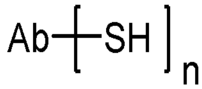 (B)

EP 4 279 502 A1

wherein
Ab and n are the same as formulae (A), and
SH is a thiol group; and the like.

FIG. 1

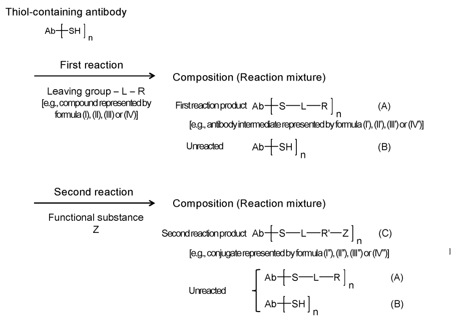

**Description**

TECHNICAL FIELD

The present invention relates to a compound or salt thereof, and antibody obtained by using the same, and the like.

BACKGROUND ART

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)).

An ADC is prepared by binding a functional group in a side chain of a specific amino acid residue present in an antibody to a drug. Examples of such a functional group utilized in the preparation of ADCs are a thiol group in a side chains of a cysteine residue present in the antibody. For techniques for modifying the thiol group in the antibody, techniques using 3-arylpropionitrile compounds (patent literature 1) and techniques using aryl/heteroarylsulfone/sulfoxide compounds (patent literature 2) are known. The above prior art for modifying the thiol group in the antibody is to modify thiol group(s) in the side chain of cysteine residues in the antibody (e.g., thiol group(s) in the side chains of cysteine residue(s) naturally occurring in the antibody and cysteine residue(s) genetically engineered into the antibody).

However, as long as the inventors know, the prior art does not teach or suggest modifying a thiol group chemically introduced into an antibody (e.g., a thiol group introduced into an antibody via a side chain of an amino acid residue other than a cysteine residue in the antibody).

PRIOR ART REFERENCES

Patent Literature

Patent Literature 1: WO 2015/001117
Patent Literature 2: WO 2014/144878

DISCLOSURE OF INVENTION

PROBLEM TO BE SOLVED BY THE INVENTION

The object of the present invention is to provide a desired antibody or antibody composition in which an antibody has modification of a tiol group which is chemically introduced into the antibody.

MEANS FOR SOLVING PROBLEM

As a result of intensive studies, the present inventors have found that a certain compound can efficiently modify a thiol group which is chemically introduced into an antibody. Such a specific compound is believed to be useful for modification of a thiol group which is chemically introduced into an antibody and modification of a thiol group in a cysteine residue in an antibody because of excellent efficiency of a reaction. According to such a specific compound, it is also possible to produce a desired antibody in which a thiol group in the antibody is modified with high efficiency.

The inventors have also found that antibody modified by the specific compound is excellent in desired properties such as low antibody aggregation.

The present inventors have further found that according to the present invention, a special antibody composition comprising a desired antibody having the above characteristics can be obtained, and the present invention has been completed.

That is, the present invention is the following.

[1] An antibody composition comprising

(A) an antibody intermediate or a salt thereof having a bioorthogonal functional group which may be protected, which is represented by formula (A), and

(B) a thiol group-introduced antibody or a salt thereof, which is represented by formula (B),

wherein the sulfur atom (S) adjacent to the antibody (Ab) in formula (A) and the thiol group (SH) adjacent to the antibody (Ab) in formula (B) are directly bonded or bonded via a linker to the atom in the side chain of the same amino acid residue present at the same position in the constant region of the antibody heavy chain,
wherein the partial structure represented by L-R has a molecular weight of 700 or less,
wherein the percentage of the amount of the antibody intermediate or salt thereof relative to the total amount of the antibody intermediate or salt thereof and the thiol group-introduced antibody or salt thereof [100 (%) × (the amount of the antibody intermediate or salt thereof)/(the total amount of the antibody intermediate or salt thereof and the thiol group-introduced antibody or salt thereof) ] is 80% or more,
wherein the antibody intermediate or salt thereof and the thiol group-introduced antibody or salt thereof have an aggregation rate of 5% or less.

[2] The antibody composition of [1], wherein the antibody intermediate is represented by formula (I'), (II'), (III'), or (IV').
[3] The antibody composition of [1] or [2], wherein the amino acid residue is an amino acid residue other than a cysteine residue.
[4] The antibody composition of any of [1] to [3], wherein the sulfur atom (S) adjacent to the antibody (Ab) in formula (A) and the thiol group (SH) adjacent to the antibody (Ab) in formula (B) are regioselectively bonded to a nitrogen atom in a side chain of a lysine residue present at the same position in the constant region of the heavy chain of the antibody (Ab) via a linker which does not contain a peptide.
[5] The antibody composition of any of [1] to [4], wherein the antibody is an IgG antibody.
[6] The antibody composition of [4] or [5], wherein the lysine residue is present at one or more positions selected from the group consisting of the positions 246/248, 288/290, and 317 of a heavy chain of a human IgG of EU numbering.
[7] The antibody composition of any of [1] to [6], wherein the antibody composition is selected from the group consisting of antibody intermediates represented by formulae (1')-(10').
[8] The antibody composition of any of [1] to [7], wherein the bioorthogonal functional group which may be protected is an alkyne residue or azide.
[9] The antibody composition of [8], wherein the alkyne residue is a ring group having a triple bond between carbon atoms, which may be substituted.
[10] A reagent for derivatizing a thiol group-introduced antibody or a salt thereof, which comprises a compound represented by formula (I), (II), (III), or (IV).
[11] The reagent of [10], wherein the reagent is a reagent for derivatizing the thiol group-introduced antibody or salt thereof by reacting with a thiol group introduced via a side chain of an amino acid residue other than a cysteine residue in a thiol group-introduced antibody or a salt thereof.
[12] The reagent of [10] or [11], wherein the thiol group in the thiol group-introduced antibody is regioselectively bonded to a nitrogen atom in a side chain of a lysine residue in a constant region of a heavy chain in the antibody via a linker which does not contain a peptide.
[13] The reagent of any of [10] to [12], wherein the thiol group-introduced antibody is an IgG antibody.
[14] The reagent of [12] or [13], wherein the lysine residue is present in one or more positions selected from the group consisting of the positions 246/248, 288/290, and 317 of a heavy chain of a human IgG of EU numbering.
[15] The reagent of any of [10] to [14], wherein the divalent group is one group selected from the group consisting of alkylene, arylene, -C(=O)-, $-NR_2-$, $-C(=O)-NR_2-$, $-NR_2-C(=O)-$, -O-, and $-(O-R_3)_m$, or

the divalent group is a divalent group in which two or more groups selected from the group consisting of alkylene, arylene, -C(=O)-, $-NR_2-$, $-C(=O)-NR_2-$, $-NR_2-C(=O)-$, -O-, and$-(O-R_3)_m$ are linked,
$R_2$ is a hydrogen atom or alkyl,
$R_3$ is alkylene or arylene, and
m is an integer from 1 to 5.

[16] The reagent of any of [10] to [15], wherein the reagent is selected from the group consisting of compounds represented by formulae (1)-(10).
[17] A compound or a salt thereof, wherein the compound is represented by formula (I), (II), (III), or (IV).
[18] The compound or salt thereof of [17], wherein the compound is selected from the group consisiting of the compounds represented by formulae (2)-(10).
[19] An antibody intermediate or salt thereof having a bioorthogonal functional group which may be protected, wherein the antibody intermediate is represented by formula (I'), (II'), (III'), or (IV').

[20] The antibody intermediate or salt thereof of [19], wherein the sulfur atom (S) adjacent to the antibody (Ab) in formula (I'), (II'), (III'), or (IV') is directly bonded or bonded via a linker to an atom in a side chain of an amino acid residue other than a cysteine residue in a constant region of a heavy chain of the antibody (Ab).
[21] The antibody intermediate or salt thereof of [20], wherein the sulfur atom (S) adjacent to the antibody (Ab) in formula (I'), (II'), (III'), or (IV') is regioselectively bonded to a nitrogen atom in a side chain of a lysine residue in the constant region of the heavy chain of the antibody (Ab) via a linker which does not contain a peptide.
[22] The antibody intermediate or salt thereof of any of [19] to [21], wherein the antibody intermediate is an IgG antibody.
[23] The antibody intermediate or salt thereof of [21] or [22], wherein the lysine residue is present at one or more positions selected from the group consisting of the positions 246/248, 288/290, and 317 of a heavy chain in a human IgG of EU numbering.
[24] The antibody intermediate or salt thereof of any of [19] to [23], wherein the antibody intermediate is selected from the group consisting of antibody intermediates represented by formulae (1')-(12').
[25] A method for producing an antibody intermediate or a salt thereof having a bioorthogonal functional group which may be protected, comprising reacting a compound represented by formula (I), (II), (III), or (IV) or a salt thereof with a thiol group-introduced antibody or a salt thereof to give the antibody intermediate or salt thereof having a bioorthogonal functional group which may be protected, which is represented by formula (I'), (II'), (III'), or (IV').
[26] A conjugate of an antibody and a functional substance or a salt thereof, which is represented by formula (I''), (II"), (III"), or (IV").
[27] The conjugate or salt thereof of [26], wherein the conjugate is selected from the group consisting of conjugates represented by formulae (1")-(12").
[28] A method for producing a conjugate of an antibody and a functional substance or a salt thereof, comprising reacting an antibody intermediate or salt thereof having a bioorthogonal functional group which may be protected, which is represented by formula (I'), (II'), (III'), or (IV') with a functional substance having a bioorthogonal functional group capable of reacting with a bioorthogonal functional group possessed by the antibody intermediate

to give the conjugate of the antibody and the functional substance or salt thereof wherein the conjugate is represented by formula (I"), (II"), (III"), or (IV"),
when the antibody intermediate or salt thereof has an alkyne residue, the antibody intermediate or salt thereof is reacted with a functional substance having an azide, or
when the antibody intermediate or salt thereof has an azide, the antibody intermediate or a salt thereof is reacted with a functional substance having an alkyne residue.

[29] The method of [28], further comprising reacting a compound or salt thereof, wherein the compound is represented by formula (I), (II), (III), or (IV) with a thiol group-introduced antibody or salt thereof
to give the antibody intermediate or salt thereof having a bioorthogonal functional group which may be protected, which is represented by the above formula (I'), (II'), (III'), or (IV').

Effect of Invention

The antibody composition of the present invention can efficiently produce a conjugate of an antibody and a functional substance or a salt thereof in a low aggregation state.
The reagent of the present invention can derivatize a thiol group-introduced antibody or a salt thereof.
The compound or salt thereof of the present invention can be used to derivatize a thiol-introduced antibody or a salt thereof.
The antibody intermediate or salt thereof of the present invention can be used to prepare a conjugate of an antibody and a functional substance or a salt thereof.
The conjugate or salt thereof of the present invention can be used as a pharmaceutical or reagent (e.g., diagnostic agent, research reagent).

BRIEF DESCRIPTION OF DRAWINGS

FIG. 1 is an overview of an antibody composition obtained by a reaction between a thiol group-introduced antibody and a compound represented by "leaving group-L-R" (L is a divalent group, and R is a bioorthogonal functional group which may be protected) or a salt thereof. An antibody composition obtained by such a reaction comprises an antibody intermediate or a salt thereof having a bioorthogonal functional group which may be protected, which

is represented by formula (A), and a thiol group-introduced antibody or a salt thereof, which is represented by formula (B).

FIG. 2 is an overview of modification of a thiol group-introduced antibody by a compound or salt thereof of the present invention, which is represented by formula (I).

FIG. 3 is an overview of modification of a thiol group-introduced antibody by a compound or salt thereof of the present invention, which is represented by formula (II).

FIG. 4 is an overview of modification of a thiol group-introduced antibody by a compound or salt thereof of the present invention, which is represented by formula (III).

FIG. 5 is an overview of modification of a thiol group-introduced antibody by a compound or salt thereof of the present invention, which is represented by formula (IV).

FIG. 6 shows (1) amino acid sequence of a heavy chain of trastuzumab (SEQ ID NO: 1) and (2) amino acid sequence of a light chain of trastuzumab (SEQ ID NO: 2).

FIG. 7 shows MS spectrum (found: m/z 577.03606, theoretical value: 577.03557, tetravalent) of a peptide fragment consisting of 18 amino acid residues FNWYVDGVEVHNAKTKPR (SEQ ID NO: 3) comprising a modification site to lysine residues (a tiol-introduced antibody which has been calbamidomethylated with iodoacetamide (+145.019 Da)) by trypsin digestion of trastuzumab.

FIG. 8 shows CID spectra of product ions of m/z 682.13 (theoretical value: 682.01) corresponding to trivalent y16 which shows modification of lysine residue(s) at the position 288/290 of a human IgG heavy chain according to EU numbering.

FIG. 9 shows results of searching a peptide fragment comprising a modification to lysine residues (a tiol-introduced antibody which has been calbamidomethylated with iodoacetamide (+145.019 Da)) for a trypsin digest of trastuzumab using a BioPharma Finder. The horizontal axis indicates the identified lysine residue, and the vertical axis indicates Intensity.

FIG. 10 shows MS spectrum (found: m/z 577.03571, theoretical value: 577.03557, tetravalent) of a peptide fragment consisting of 18 amino acid residues FNWYVDGVEVHNAKTKPR (SEQ ID NO: 3) comprising a modification site to lysine residues (a tiol-introduced antibody which has been calbamidomethylated with iodoacetamide (+145.019 Da)) by trypsin digestion of trastuzumab.

FIG. 11 shows CID spectra of product ions of m/z 682.41 (theoretical value: 682.01) corresponding to trivalent y16 which shows modification of lysine residue(s) at the position 288/290 of a human IgG heavy chain according to EU numbering.

FIG. 12 shows results of searching a peptide fragment comprising a modification to lysine residues (a tiol-introduced antibody which has been calbamidomethylated with iodoacetamide (+145.019 Da)) for a trypsin digest of trastuzumab using a BioPharma Finder. The horizontal axis indicates the identified lysine residue, and the vertical axis indicates Intensity.

FIG. 13 shows MS spectrum (found: m/z 769.04506, theoretical value: 769.04482, trivalent) of a peptide fragment consisting of 18 amino acid residues FNWYVDGVEVHNAKTKPR (SEQ ID NO: 3) comprising a modification site to lysine residues (a tiol-introduced antibody which has been calbamidomethylated with iodoacetamide (+145.019 Da)) by trypsin digestion of trastuzumab.

FIG. 14 shows CID spectra of product ions of m/z 1022.71 (theoretical value: 1022.51) corresponding to trivalent y16 which shows modification of lysine residue(s) at the position 288/290 of a human IgG heavy chain according to EU numbering.

FIG. 15 shows results of searching a peptide fragment comprising a modification to lysine residues (a tiol-introduced antibody which has been calbamidomethylated with iodoacetamide (+145.019 Da)) for a trypsin digest of trastuzumab using a BioPharma Finder. The horizontal axis indicates the identified lysine residue, and the vertical axis indicates Intensity.

## EMBODIMENTS FOR CARRYING OUT THE INVENTION

### 1.Definitions of general terms

#### (Antibody and terms related thereto)

In the present invention, the term "antibody" is as follows. The term "immunoglobulin unit" corresponds to a divalent monomer unit that is a basic constituent element of such an antibody, and is a unit comprising two heavy chains and two light chains. Therefore, definitions, examples, and preferred examples of the origin, type (polyclonal or monoclonal, isotype, and full-length antibody or antibody fragment), antigen, position of a lysine residue, and regioselectivity of the immunoglobulin unit are similar to those of the antibody described below.

The origin of the antibody is not particularly limited, and for example, the antibody may be derived from an

animal such as a mammal or a bird (e.g., a domestic fowl). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or a variable region, and an antibody fragment (e.g., an antibody fragment comprising a CH1 domain and a CH2 domain) or an antibody which does not comprise a constant region) can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include a protein [which comprises an oligopeptide and a polypeptide, and may be a protein modified with a biomolecule such as a sugar (e.g., a glycoprotein)], a sugar chain, a nucleic acid, and a small compound. The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.

(1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

(2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

(3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

(4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

(5) Hereditary Rare Diseases

amyloid AL, SEMA4D (CD100), insulin receptors, ANGPTL3, IL4, IL13, FGF23, adrenocorticotropic hormone, transthyretin, and huntingtin.

(6) Eye Diseases

Factor D, IGF-1R, PGDFR, Ang2, VEGF-A, CD-105 (Endoglin), IGF-1R, and β amyloid.

(7) Bone and Orthopedic Region

Sclerostin, Myostatin, Dickkopf-1, GDF8, RNAKL, HAS, and Siglec-15

(8) Blood Diseases

vWF, Factor IXa, Factor X, IFNγ, C5, BMP-6, Ferroportin, and TFPI

(9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

The positions of amino acid residues in the antibody and the position of a constant region of a heavy chain (e.g., CH2 domain) are in accordance with EU numbering (see, http://www.imgt.org/IMGTScientificChart/Numbering/Hu_IGHGnb er. html). For example, when human IgG is a target, a lysine residue at position 246 corresponds to an amino acid residue at position 16 of a human IgG CH2 region, a lysine residue at position 248 corresponds to an amino acid residue at position 18 of a human IgG CH2 region, a lysine residue at position 288 corresponds to an amino acid residue at position 58 of a human IgG CH2 region, a lysine residue at position 290 corresponds to an amino acid residue at position 60 of a human IgG CH2 region, and a lysine residue at position 317 corresponds to an amino acid residue at position 87 of a human IgG CH2 region. The notation at position 246/248 indicates that a lysine residue at position 246 or position 248 is a target. The notation at position 288/290 indicates that a lysine residue at position 288 or position 290 is a target.

(Halogen atom)

Examples of the halogen atom include a fluorine atom, a chlorine atom, a bromine atom, and an iodine atom.

(Monovalent hydrocarbon group and terms related thereto)

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in a main chain thereof. Note that the chain structure may be linear or branched. Examples of the monovalent chain hydrocarbon group include alkyl, alkenyl, and alkynyl. The alkyl, alkenyl, and alkynyl may be linear or branched.

The alkyl is preferably $C_{1-12}$ alkyl, more preferably $C_{1-6}$ alkyl, and even more preferably $C_{1-4}$ alkyl. When the alkyl has a substituent, the number of carbon atoms does not comprise the number of carbon atoms of the substituent. Examples of the $C_{1-12}$ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably $C_{2-12}$ alkenyl, more preferably $C_{2-6}$ alkenyl, and even more preferably $C_{2-4}$ alkenyl.

When the alkenyl has a substituent, the number of carbon atoms does not comprise the number of carbon atoms of the substituent. Examples of the $C_{2-12}$ alkenyl include vinyl, propenyl, and n-butenyl.

The alkynyl is preferably $C_{2-12}$ alkynyl, more preferably $C_{2-6}$ alkynyl, and even more preferably $C_{2-4}$ alkynyl. When the alkynyl has a substituent, the number of carbon atoms does not comprise the number of carbon atoms of the substituent. Examples of the $C_{2-12}$ alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

The cycloalkyl is preferably $C_{3-12}$ cycloalkyl, more preferably $C_{3-6}$ cycloalkyl, and even more preferably $C_{5-6}$ cycloalkyl. When the cycloalkyl has a substituent, the number of carbon atoms does not comprise the number of carbon atoms of the substituent. Examples of the $C_{3-12}$ cycloalkyl include cyclopropyl, cyclobutyl, cyclopentyl, and cyclohexyl.

The cycloalkenyl is preferably $C_{3-12}$ cycloalkenyl, more preferably $C_{3-6}$ cycloalkenyl, and even more preferably $C_{5-6}$ cycloalkenyl. When the cycloalkenyl has a substituent, the number of carbon atoms does not comprise the number of carbon atoms of the substituent. Examples of the $C_{3-12}$ cycloalkenyl include cyclopropenyl, cyclobutenyl, cyclopentenyl, and cyclohexenyl.

The cycloalkynyl is preferably $C_{3-12}$ cycloalkynyl, more preferably $C_{3-6}$ cycloalkynyl, and even more preferably $C_{5-6}$ cycloalkynyl. When the cycloalkynyl has a substituent, the number of carbon atoms does not comprise the number of carbon atoms of the substituent. Examples of the $C_{3-12}$ cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably $C_{6-12}$ aryl, more preferably $C_{6-10}$ aryl, and even more preferably $C_6$ aryl. When the monovalent aromatic hydrocarbon group has a substituent, the number of carbon atoms does not comprise the number of carbon atoms of the substituent. Examples of the $C_{6-12}$ aryl include phenyl and naphthyl.

The monovalent aromatic hydrocarbon group is preferably phenyl.

Among these groups, the monovalent hydrocarbon group is preferably alkyl, cycloalkyl, or aryl.

(Monovalent heterocyclic group and terms related thereto)

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom comprised in the heterocyclic group.

The monovalent aromatic heterocyclic group is preferably a $C_{1-15}$ aromatic heterocyclic group, more preferably a $C_{1-9}$ aromatic heterocyclic group, and even more preferably a $C_{1-6}$ aromatic heterocyclic group. When the monovalent aromatic heterocyclic group has a substituent, the number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a $C_{2-15}$ nonaromatic heterocyclic group, more preferably a $C_{2-9}$ nonaromatic heterocyclic group, and even more preferably a $C_{2-6}$ nonaromatic heterocyclic group. When the monovalent nonaromatic heterocyclic group has a substituent, the number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

(Divalent group)

The divalent group is a divalent linear hydrocarbon group, a divalent cyclic hydrocarbon group, a divalent heterocyclic group, one group selected from the group consisting of -C(=O)-, -C(=S)-, $-NR_2-$, $-C(=O)-NR_2-$, $-NR_2-C(=O)-$, $-C(=S)-NR_2-$, $-NR_2-C(=S)-$, -O-, -S-, $-(O-R_3)_m-$, and - $(S-R_3)_m-$, or a group having a main chain structure comprising two or more (e.g., 2 to 10, preferably 2 to 8, more preferably 2 to 6, even more preferably 2 to 5, particularly preferably 2 or 3) of these groups. $R_2$ represents a hydrogen atom or a substituent described later. $R_3$ represents a divalent linear hydrocarbon group, a divalent cyclic hydrocarbon group, or a divalent heterocyclic group. m is an integer of 1 to 10, preferably an integer of 1 to 8, more preferably an integer of 1 to 6, even more preferably an integer of 1 to 5, and particularly preferably an integer of 1 to 3.

The divalent linear hydrocarbon group is a linear alkylene, a linear alkenylene, or a linear alkynylene.

The linear alkylene is a $C_{1-6}$ linear alkylene, and is preferably a $C_{1-4}$ linear alkylene. Examples of the linear alkylene include methylene, ethylene, n-propylene, n-butylene, n-pentylene, and n-hexylene.

The linear alkenylene is a $C_{2-6}$ linear alkenylene, and is preferably a $C_{2-4}$ linear alkenylene. Examples of the linear alkenylene include ethylenylene, n-propynylene, n-butenylene, n-pentenylene, and n-hexenylene.

The linear alkynylene is a $C_{2-6}$ linear alkynylene, and is preferably a $C_{2-4}$ linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a $C_{6-14}$ arylene, more preferably a $C_{6-10}$ arylene, and particularly preferably a $C_6$ arylene. Examples of the arylene include phenylene, naphthylene, and anthracenylene.

The divalent nonaromatic cyclic hydrocarbon group is preferably a $C_{3-12}$ monocyclic or polycyclic divalent nonaromatic cyclic hydrocarbon group, more preferably a $C_{4-10}$ monocyclic or polycyclic divalent nonaromatic cyclic hydrocarbon group, and particularly preferably a $C_{5-8}$ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a $C_{3-15}$ divalent aromatic heterocyclic group, more preferably a $C_{3-9}$ divalent aromatic heterocyclic group, and particularly preferably a $C_{3-6}$ divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a $C_{3-15}$ nonaromatic heterocyclic group, more preferably a $C_{3-9}$ nonaromatic heterocyclic group, and particularly preferably a $C_{3-6}$ nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

The divalent group is preferably a divalent group having a main chain structure comprising one group selected from the group consisting of alkylene, arylene, -C(=O)-, - $NR_2-$, $-C(=O)-NR_2-$, $-NR_2-C(=O)-$, -O-, and $-(O-R_2)_m-$, or

a divalent group having a main chain structure comprising two or more groups selected from the group consisting of alkylene, arylene, -C(=O)-, $-NR_2-$, -C (=O) $NR_2-$, $-NR_2-C(=O)-$, -O-, and $-(O-R_3)_m-$,
$R_{72}$ is a hydrogen atom or an alkyl,
$R_3$ is an alkylene or an arylene, and
m may be an integer of 1 to 5 (that is, 1, 2, 3, 4, or 5) .

The alkylene, the arylene, and the alkyl are similar to those described above.

The main chain structure in the divalent group may have one or more (e.g., 1 to 10, preferably 1 to 8, more preferably 1 to 6, even more preferably 1 to 5, particularly preferably 1 to 3) substituents described later.

(Substituent)

Examples of the substituent include:

(i) a halogen atom;
(ii) a monovalent hydrocarbon group;
(iii) a monovalent heterocyclic group;
(iv) an aralkyl;
(v) $R_a$-O-, $R_a$-C(=O)-, $R_a$-O-C(=O)-, or $R_a$-C(=O)-O-, (where $R_a$ represents a hydrogen atom or a monovalent hydrocarbon group);
(vi) $NR_bR_c$-, $NR_bR_c$-C(=O)-, $NR_bR_c$-C(=O)-O-, or $R_b$-C(=O)-$NR_c$-, (where $R_b$ and $R_c$ are the same as or different from each other, and each represent a hydrogen atom or a monovalent hydrocarbon group); and
(vii) a nitro group, a sulfate group, a sulfonate group, a cyano group, and a carboxyl group.

Definitions, examples, and preferred examples of the halogen atom, the monovalent hydrocarbon group, and the monovalent heterocyclic group in the substituent are similar to those described above.
The aralkyl refers to arylalkyl. Definitions, examples, and preferred examples of the aryl and the alkyl in the arylalkyl are as described above. The aralkyl is preferably $C_{3-15}$ aralkyl. Examples of such an aralkyl include benzoyl, phenethyl, naphthylmethyl, and naphthylethyl.
The substituent may be preferably:

(i) a halogen atom;
(ii) a $C_{1-12}$ alkyl, a $C_{1-12}$ phenyl, or a $C_{1-12}$ naphthyl;
(iii) a $C_{3-15}$ aralkyl;
(iv) a 5- or 6-membered heterocycle;
(v) $R_a$-O-, $R_a$-C(=O)-, $R_a$-O-C(=O)-, or $R_a$-C(=O)-O-, (where $R_a$ represents a hydrogen atom or a $C_{1-12}$ alkyl);
(vi) $NR_bR_c$-, $NR_bR_c$-C(=O)-, $NR_bR_c$-C(=O)-O-, or $R_b$-C(=O)-$NR_c$-, (where $R_b$ and $R_c$ are the same as or different from each other, and each represent a hydrogen atom or a $C_{1-12}$ alkyl); or
(vii) the same groups as listed in the above (vii).

The substituent may be more preferably:

(i) a halogen atom;
(ii) a $C_{1-12}$ alkyl;
(iii) $R_a$-O-, $R_a$-C(=O)-, $R_a$-O-C(=O)-, or $R_a$-C(=O)-O-, (where $R_a$ represents a hydrogen atom or a $C_{1-12}$ alkyl);
(iv) $NR_bR_c$-, $NR_bR_c$-C(=O)-, $NR_bR_c$-C(=O)-O-, or $R_b$-C(=O)-$NR_c$-, (where $R_b$ and $R_c$ are the same as or different from each other, and each represent a hydrogen atom or a $C_{1-12}$ alkyl); or
(v) the same groups as listed in the above (vii).

The substituent may be even more preferably:

(i) a halogen atom;
(ii) a $C_{1-6}$ alkyl;
(iii) $R_a$-O-, $R_a$-C(=O)-, $R_a$-O-C(=O)-, or $R_a$-C(=O)-O-, (where $R_a$ represents a hydrogen atom or a $C_{1-6}$ alkyl);
(iv) $NR_bR_c$-, $NR_bR_c$-C(=O)-, $NR_bR_c$-C(=O)-O-, or $R_b$-C(=O)-$NR_c$-, (where $R_b$ and $R_c$ are the same as or different from each other, and each represent a hydrogen atom or a $C_{1-6}$ alkyl); or
(v) the same groups as listed in the above (vii).

The substituent may be particularly preferably:

(i) a halogen atom;
(ii) a $C_{1-4}$ alkyl;
(iii) $R_a$-O-, $R_a$-C(=O)-, $R_a$-O-C(=O)-, or $R_a$-C(=O)-O-, (where $R_a$ represents a hydrogen atom or a $C_{1-4}$ alkyl);
(iv) $NR_bR_c$-, $NR_bR_c$-C(=O)-, $NR_bR_c$-C(=O)-O-, or $R_b$-C(=O)-$NR_c$-, (where $R_b$ and $R_c$ are the same as or different from each other, and each represent a hydrogen atom or a $C_{1-4}$ alkyl); or
(v) the same groups as listed in the above (vii).

(Bioorthogonal functional group)

The bioorthogonal functional group refers to a group that does not react with biological components (e.g., amino acids, proteins, nucleic acids, lipids, sugars, and phosphoric acids) or has a low reaction rate to the biological components but selectively reacts with components other than the biological components. The bioorthogonal functional group is well known in the technical field concerned (see, e.g., Sharpless K. B. et al., Angew. Chem. Int. Ed. 40, 2004 (2015); Bertozzi C. R. et al., Science 291,2357 (2001); Bertozzi C. R. et al., Nature Chemical Biology 1,13 (2005)).

In the present invention, as the bioorthogonal functional group, a bioorthogonal functional group to a protein is used. This is because a thiol group-introduced antibody to be derivatized with a reagent of the present invention is a protein. The bioorthogonal functional group to a protein is a group that does not react with side chains of 20 types of natural amino acid residues forming proteins, or reacts with a target functional group although having a low reaction rate to the side chain. The 20 types of natural amino acids forming proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Among these 20 types of natural amino acids, glycine, which has no side chain (that is, which has a hydrogen atom as a side chain), and alanine, isoleucine, leucine, phenylalanine, and valine, which each have a hydrocarbon group as a side chain (that is, which each comprise no hetero atom selected from the group consisting of a sulfur atom, a nitrogen atom, and an oxygen atom in a side chain thereof) are inactive to normal reactions. Consequently, the bioorthogonal functional group to a protein is a group that does not react with, in addition to the side chains of these amino acids having side chains inactive to normal reactions, side chains of asparagine, glutamine, methionine, proline, serine, threonine, tryptophan, tyrosine, aspartic acid, glutamic acid, arginine, histidine, and lysin, or reacts with a target functional group although having a low reaction rate.

Examples of such a bioorthogonal functional group include an azide residue, an aldehyde residue, a thiol residue, an alkene residue (in other words, it is only required to have a vinylene (ethenylene) portion, which is a minimum unit having a carbon-carbon double bond. Hereinafter the same), an alkyne residue (in other words, it is only required to have an ethynylene portion, which is a minimum unit having a carbon-carbon triple bond. Hereinafter the same), a halogen residue, a tetrazine residue, a nitrone residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue (e.g., a carbonyl residue having a fluorine atom, a chlorine atom, a bromine atom, or an iodine atom at an α-position. Hereinafter the same), an isonitrile residue, a sydnone residue, and a selenium residue.

More specifically, the bioorthogonal functional group may correspond to any one chemical structure selected from the group consisting of the following:

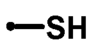 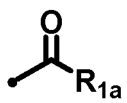 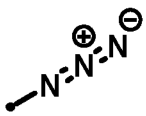 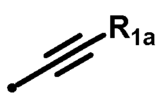 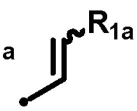 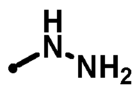 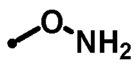

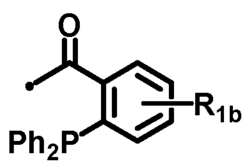 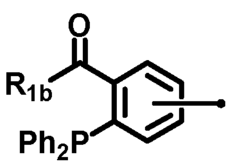 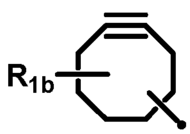 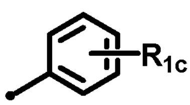 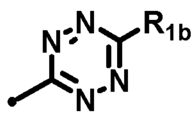

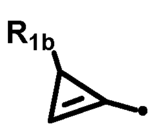 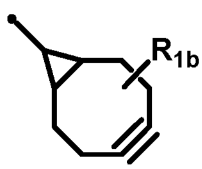 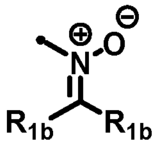 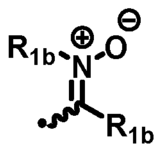 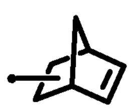 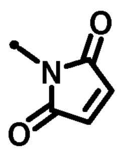

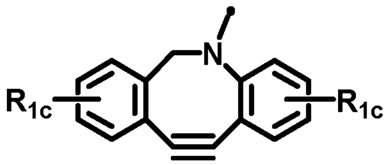 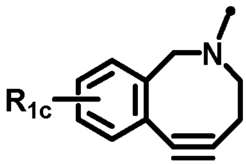 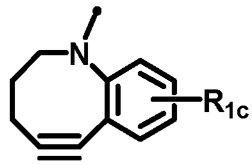

wherein

$R_{1a}$, one or a plurality of $R_{1b}$s, and one or a plurality of $R_{1c}$s are the same as or different from each other, and each represent any one of the substituents described above or an electron-withdrawing group, and
• is a bond.

Examples of the electron-withdrawing group include a halogen atom, an alkyl substituted with a halogen atom (e.g., trifluoromethyl), a boronic acid residue, mesyl, tosyl, triflate, nitro, cyano, a phenyl group, and a keto group (e.g., acyl), and a halogen atom, a boronic acid residue, mesyl, tosyl, and triflate are preferred.

Preferably, the bioorthogonal functional group may be an alkyne residue or an azide residue. The alkyne residue may be a ring group which has a carbon-carbon triple bond and which may be substituted with a substituent as described above.

The bioorthogonal functional group may be protected. The optionally protected bioorthogonal functional group refers to an unprotected bioorthogonal functional group or a protected bioorthogonal functional group. The unprotected bioorthogonal functional group corresponds to the bioorthogonal functional group described above. The protected bioorthogonal functional group is a group that generates a bioorthogonal functional group by cleavage of a protective group. The protective group can be cleaved by a specific treatment under a condition (a mild condition) incapable of causing denaturation or decomposition of proteins (e.g., cleavage of an amide bond). Examples of such a specific treatment include (a) a treatment with one or more substances selected from the group consisting of an acidic substance, a basic substance, a reducing agent, an oxidizing agent, and an enzyme, (b) a treatment with a physical and chemical stimulus selected from the group consisting of light, and (c) leaving a cleavable linker as it is when the cleavable linker comprises a self-degradable cleavable portion. Such a protective group and a cleavage condition therefor are common technical knowledge in the field concerned (e.g., G. Leriche, L. Chisholm, A. Wagner, Bioorganic & Medicinal Chemistry. 20,571 (2012); Feng P. et al., Jounal of American Chemical Society. 132,1500 (2010).; Bessodes M. et al., Journal of Controlled Release, 99,423 (2004).; DeSimone, J.M., Journal of American Chemical Society. 132,17928 (2010); Thompson, D.H., Journal of Controlled Release, 91,187 (2003); and Schoenmarks, R.G., Journal of Controlled Release, 95,291 (2004)).

Examples of the protected bioorthogonal functional group include a disulfide residue, an ester residue, an acetal residue, a ketal residue, an imine residue, and a vicinaldiol residue.

More specifically, the protected bioorthogonal functional group may correspond to any one chemical structure selected from the group consisting of the following:

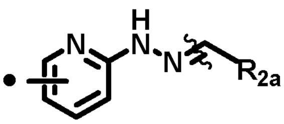 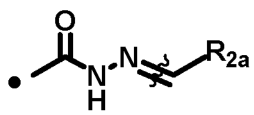 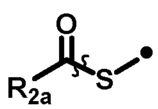 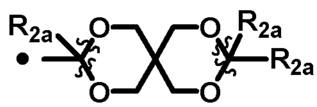

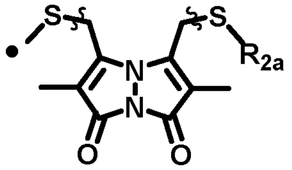 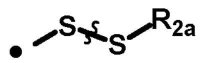 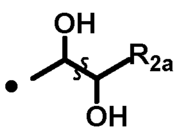 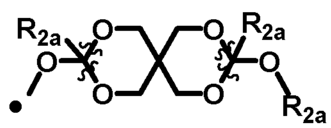

wherein the wavy line orthogonal to the bond indicates a cleavage site,

one or a plurality of $R_{2a}$s are the same as or different from each other, and each represent a hydrogen atom or a group selected from the group consisting of the substituents described above, and
• is a bond.

The optionally protected bioorthogonal functional group is preferably an unprotected bioorthogonal functional group.

(Functional substance)

The functional substance is not limited to a particular substance as long as it is a substance imparting any function to the antibody; examples thereof include drugs, labelling substances, and stabilizers; preferred are drugs and labelling substances. The functional substance may be a single functional substance or a substance in which two or more functional substances are linked with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., $^{3}H$), radioisotopes of a carbon atom (e.g., $^{14}C$), radioisotopes of a phosphorous atom (e.g., $^{32}P$), radioisotopes of a sulfur atom (e.g., $35^{S}$), radioisotopes of yttrium (e.g., $^{90}Y$), radioisotopes of technetium (e.g., $^{99m}Tc$), radioisotopes of indium (e.g., $^{111}In$), radioisotopes of an iodide atom (e.g., $^{123}I$, $^{125}I$, $^{129}I$, and $^{131}I$), radioisotopes of samarium (e.g., $^{153}Sm$), radioisotopes of rhenium (e.g., $^{186}Re$), radioisotopes of astatine (e.g., $^{211}At$), and radioisotopes of bismuth (e.g., $^{212}Bi$). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubricin.

The labelling substance is a substance that makes detection of a target (e.g., a tissue, a cell, or a substance) possible. Examples of the labelling substance include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl) Duthenium, and luminol), and radioisotopes (e.g., those described above) or substances comprising them.

The stabilizer is a substance that makes stabilization of an antibody possible. Examples of the stabilizer include diols, glycerin, nonionic surfactants, anionic surfactants, natural surfactants, saccharides, and polyols.

The functional substance may also be a peptide, a protein, a nucleic acid, a low molecular weight organic compound, a sugar chain, a lipid, a high molecular polymer, a metal (e.g., gold), or a chelator. Examples of the peptide include a cell membrane permeable peptide, a blood-brain barrier permeable peptide, and a peptide medicament. Examples of the protein include enzymes, cytokines, fragment antibodies, lectins, interferons, serum albumin, and antibodies. Examples of the nucleic acid include DNA, RNA, and artificial nucleic acid. Examples of the nucleic acid also include RNA interference inducible nucleic acids (e.g., siRNA), aptamers, and antisense. Examples of the low molecular weight organic compound include proteolysis targeting chimeras, dyes, and photodegradable compounds.

When the functional sutstance does not have a bioorthogonal functional group that is likely to react with the bioorthogonal functional group possessed by the antibody intermediate, the functional sutstance may be derivatized to have such a bioorthogonal functional group. The bioorthogonal functional group possessed by the functional substance or the derivatized functional substance is selected from the bioorthogonal functional group as described above while considering the bioorthogonal functional group possessed by the antibody intermediate. For example, when the bioorthogonal functional group possessed by the antibody intermediate is an alkyne residue (preferably a ring group having a triple bond between carbon atoms, which may be substituted with a substituent as described above), the bioorthogonal

functional group possessed by the functional sutstance or the derivatized functional sutstance may be an azide. When the bioorthogonal functional group possessed by the antibody intermediate is an azide, the bioorthogonal functional group possessed by the functional substance or the derivatized functional substance may be an alkyne residue (preferably, a ring group having a triple bond between carbon atoms, which may be substituted with a substituent as described above). The derivatization is common technical knowledge in the art (e.g., WO2004/010957, US2006/0074008, US2005/02386649). For example, the derivatization may be carried out using any cross-linking agent. Alternatively, the derivatization may be carried out using a specific linker having a desired functional group. For example, such a linker may be capable of separating a functional substance and an antibody in a suitable environment (e.g., intracellular or extracellular) by cleavage of the linker. Such linkers include, for example, peptidyl linkers that are degraded by specific proteases [e.g., intracellular proteases (e.g., proteases present in lysosomes, or endosomes), extracellular proteases (e.g., secretory proteases)] (e.g., USP6,214,345; Dubowchik et al., Pharma. Therapeutics 83: 67-123 (1999)), a linker that can be cleaved at a locally acidic site present in the living body (e.g., USP5,622,929, 5,122,368; 5,824,805). The linker may be self-immolative (e.g., WO02/083180, WO04/043493, WO05/1192919). In the present invention, the derivatized functional substance is also simply referred to as "functional substance".

(Salt)

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

2. Antibody composition

The present invention provide an antibody composition comprising

(A) an antibody intermediate or a salt thereof having a bioorthogonal functional group which may be protected, which is represented by the following formula (A):

$$Ab\left[S-L-R\right]_n \quad (A)$$

wherein

Ab is an antibody comprising an immunoglobulin unit comprising two heavy chains and two light chains and having a disulfide bond between the heavy chains and between the heavy and light chains,
S is a sulfur atom,
L is a divalent group,
R is a bioorthogonal functional group which may be protected, and
n is an integer from 1 to 8, and

(B) a thiol group-introduced antibody or a salt thereof, which is represented by the following formula (B):

$$Ab\left[SH\right]_n \quad (B)$$

wherein

Ab is the same antibody as the antibody in formula (A),
SH is a thiol group, and
n is the same integer as n in formula (A),
wherein the sulfur atom (S) adjacent to the antibody (Ab) in formula (A) and the thiol group (SH) adjacent to the antibody (Ab) in formula (B) are directly bonded or bonded via a linker to the atom in the side chain of the same amino acid residue present at the same position in the constant region of the antibody heavy chain,
wherein the partial structure represented by L-R has a molecular weight of 700 or less,
wherein the percentage of the amount of the antibody intermediate or salt thereof relative to the total amount of the antibody intermediate or salt thereof and the thiol group-introduced antibody or salt thereof [100 (%) × (the amount of the antibody intermediate or salt thereof)/(the total amount of the antibody intermediate or salt thereof and the thiol group-introduced antibody or salt thereof) ] is 80% or more,
wherein the antibody intermediate or salt thereof and the thiol group-introduced antibody or salt thereof have an aggregation rate of 5% or less.

The antibody composition of the present invention can be obtained by reacting a thiol group-introduced antibody with a compound represented by "leaving group-L-R" (FIG. 1). Therefore, the common symbols Ab and n in formulae (A) and (B) are the same between the formulae (A) and (B). Although such an antibody composition can be obtained according to the present invention, there is no motivation to obtain such an antibody composition depending on prior art.

Ab in formulae (A) and (B) is an antibody comprising immunoglobulin unit comprising two heavy chains and two light chains and having a disulfide bond between the heavy chains and between the heavy chain and the light chain. Such antibodies include, for example, an IgG antibody, IgD antibody and IgE antibody comprising an immunoglobulin unit comprising two heavy chains and two light chains and having a disulfide bond between the heavy chains and between the heavy chain and the light chain; an IgA antibody comprising an immunoglobulin unit comprising four heavy chains and four light chains and having disulfide bonds between the heavy chains and between the heavy chain and light chain; and an IgM antibody comprising an immunoglobulin unit comprising eight light chains and eight light chains and having a disulfide bond between the heavy chains and light chains and between the heavy chain and the light chain, and an IgG antiboduy (e.g., IgG1, IgG2, IgG3, IgG4) is preferred. The antibody is preferably a human IgG monoclonal antibody, more preferably a human IgG full-length monoclonal antibody.

The divalent group represented by L in formula (A) is the same as that described above.

The bioorthogonal functional group which may be protected, which is represented by R in the formula (A) is the same as that described above.

n in formulae (A) and (B) is an integer of 1 to 8. n is preferably an integer of 1 to 6, and more preferably an integer of 1 to 4 (i.e., 1, 2, 3 or 4).

The sulfur atom (S) adjacent to the antibody (Ab) in formula (A) and the thiol group (SH) adjacent to the antibody (Ab) in formula (B) are directly linked or linked via a linker to the atoms in the side chain of the same amino acid residue present at the same position in the constant region of the antibody heavy chain. Since the antibody composition of the present invention can be obtained by modifying a thiol group-introduced antibody with a compound represented by the "leaving group-L-R" (FIG. 1), the bonding manner of the sulfur atom (S) and the thiol group (SH) to the antibody is the same. That is, when the sulfur atom (S) is also directly linked to the atom in the side chain, then the thiol group (SH) is also directly linked to the same atom in the same side chain. When the sulfur atom (S) is linked to an atom in the side chain via a linker, the thiol group (SH) is also linked to the same atom in the same side chain via the same linker.

In one embodiment, the amino acid residues in the antibody utilized to link the sulfur atoms (S) and thiol groups (SH) may be amino acid residues other than cysteine residues. For the amino acid residues other than cysteine residues, amino acid residues having a side chain which are easily modified (e.g., amino group, carboxy group, amide group, hydroxy group) (e.g., lysine residue, aspartic acid residue, glutamic acid residue, asparagine residue, glutamine residue, threonine residue, serine residue, tyrosine residue) can be utilized. Preferably, the amino acid residue is a lysine residue having a side chain containing a nitrogen atom (amino group), a tyrosine residue, serine residue, and threonine residue having a side chain containing an oxygen atom (hydroxy group). More preferably, the amino acid residue is a lysine residue.

In another embodiment, the amino acid residue in the antibody utilized to link the sulfur atoms (S) and thiol groups (SH) may be cysteine residues. The compound used in the present invention are believed to be useful for modification of a thiol group chemically introduced into antibody as well as modification of a thiol group in cysteine residue in antibody because of the excellent efficiency of the reaction.

The amino acid residues in the antibody utilized in the bonding of the sulfur atom (S) and the thiol group (SH) may utilize those present at a certaion location in the constant region of the antibody heavy chain (for example, CH1, CH2 or CH3, preferably CH2 or CH3).

In a preferred specific embodiment, the amino acid residue in the antibody utilized to bond the sulfur atom (S) and the thiol group (SH) is a lysine residue having a side chain containing a nitrogen atom (amino group). Examples of

the lysine residue include a lysine residue present at one or more locations selected from the group consisting of the position 246/248, 288/290, and 317 in a human IgG heavy chain.

Methods of producing an antibody containing a specific atom or group at a certain position by regioselectively modifying a specific amino acid residue at the certain position are described, for example, in WO 2018/199337, WO 2019/240288, WO 2019/240287, and WO 2020/090979. In addition, according to the methods described in these references, specific amino acid residues at a certain position can be regioselectively modified without the use of a linker containing a peptide. The peptide moiety have potential immunogenicity and is susceptible to hydrolysis in the blood. Therefore, avoiding the use of a linker containing a peptide moiety is desirable in clinical applications.

Preferably, an antibody containing a thiol group that is regioselectively linked to an atom in the side chain of a specific amino acid residue present at a certain position in the constant region of the heavy chain (preferably a nitrogen atom in the side chain of the lysine residue) as a thiol group-introduced antibody via a linker that does not contain a peptide can be used. By modifying such a thiol group-introduced antibody with a compound represented by the "leaving group-L-R", it is possible to obtain the antibody composition of the present invention wherein the sulfur atom (S) adjacent to the antibody (Ab) in formula (A) and the thiol group (SH) adjacent to the antibody (Ab) in formula (B) are regioselectively directly linked to, or are regioselectively linked via a linker to an atom in the side chain of the same amino acid residue present at the same position in the constant region of the antibody heavy chain. In the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselective binding or retention can be achieved by utilizing an antibody obtained by the above method wherein a specific amino acid residue at a certain position can be modified with a thiol group in a regioselective manner as the thiol group-introduced antibody. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%. Confirmation of position selectivity can be performed, for example, by peptide mapping (see, e.g., International Publication No. 2019/240287 (WO 2019/240287 A1)).

As a linker that does not contain a peptide, the divalent group described above can be utilized. Preferably, when the amino acid residue in the antibody utilized for the bonding of the sulfur atom (S) and the thiol group (SH) is a lysine residue, a carbonyl-containing linker comprising -C(=O)- moiety capable of forming an amide bond with an amino group in the side chain of the lysine residue can be utilized as a linker that does not contain a peptide.

For the carbonyl-containing linker, a group having -C(=O)- moiety at an end and having a main chain structure containing one group selected from the group consisting of a divalent linear hydrocarbon group, a divalent cyclic hydrocarbon group, a divalent heterocyclic group, -C(=O)-, -C(=S)-, $-NR_4-$, $-C(=O)-NR_4-$, $-NR_4-C(=O)-$, $-C\ (=S)-NR_4-$, $-NR_4-C(=S)-$, -O-, -S-, $-(O-R_5)_{m1}-$, and $-(S-R_5)_{m1}-$, or two or more thereof (for example, 2 to 10, preferably 2 to 8, more preferably 2 to 6, even more preferably 2 to 5, particularly preferably 2 or 3) can be used. $R_4$ indicates a hydrogen atom or a substituent described below. $R_5$ indicates a divalent linear hydrocarbon group, a divalent cyclic hydrocarbon group, or a divalent heterocyclic group. m1 is an integer from 1 to 10, preferably an integer from 1 to 8, more preferably an integer from 1 to 6, even more preferably an integer from 1 to 5, more preferably an integer from 1 to 3.

Preferably, the carbonyl-containing linker is a divalent group having -C(=O)- moiety at an end and having a main chain structure comprising one group selected from the group consisting of alkylene, arylene, -C(=O)-, $-NR_2-$, $-C(=O)-NR_4-$, $-NR_4-C(=O)-$, -O-, and $-(O-R_5)_{m1}-$, or alternatively

a divalent group having -C(=O)- moiety at an end and having a main chain structure comprising two or more groups selected from the group consisting of alkylene, arylene, - C(=O)-, $-NR_2-$, $-C(=O)-NR_4-$, $-NR_4-C(=O)-$, -O-, and $-(O-R_5)_m$,

$R_2$ is a hydrogen atom or alkyl,

$R_3$ is alkylene or arylene,

m is an integer from 1 to 5 (i.e., 1, 2, 3, 4, or 5).

The alkylene, arylene, and alkyl are similar to those described above.

More preferably, the carbonyl-containing linker has a-C (= O)-moiety at the end and may be a divalent group having a backbone structure comprising alkylene or arylene or combinations thereof.

Even more preferably, the carbonyl-containing linker may be a divalent group (e.g.,$-C\ (= O)-CH_2-CH_2-$) having a backbone structure comprising-C (= O)--portions and alkylene.

The backbone structure of the carbonyl-containing linker may be substituted with one or more of the substituents

described above (e.g., 1 to 10, preferably 1 to 8, more preferably 1 to 6, even more preferably 1 to 5, particularly preferably 1 to 3).

In the present invention, the molecular weight of the partial structure represented by L-R in formula (A) is 700 or less. When the molecular weight of the partial structure represented by L-R is 700 or less, the antibody intermediate or salt thereof having a bioorthogonal functional group which may be protected, and the thiol group-introduced antibody or salt thereof have a very small ratio of the molecular weight of the partial structure relative to the molecular weight of the whole antibody, thus making it difficult to separate based on the difference in molecular weight. The molecular weight of the partial structure represented by L-R is preferably 600 or less, more preferably 500 or less, even more preferably 400 or less, particularly preferably 300 or less, 250 or less, 200 or less, or 150 or less.

In the present invention, the percentage of the amount of antibody intermediates or salts thereof to the total amount of antibody intermediates or salts thereof and thiol group-introduced antibodies or salts thereof [100 (%) × (amount of antibody intermediates or salts thereof)/(total amount of antibody intermediates or salts thereof and thiol group-introduced antibody or salt thereof) is greater than or equal to 80%. Since the compound or salt thereof used in the present invention have excellent reaction efficiency for thiol group-introduced antibody or salt thereof, good reactivity expressed in such percentages can be achieved. Such percentage is preferably 82% or more, more preferably 84% or more, even more preferably 86% or more, particularly preferably 88% or more, 90% or more, 92% or more, 94% or more, or 96% or more. Such percentage may be determined based on reverse phase HPLC under reduction conditions or value measured by mass spectrometry (see, Examples).

In the present invention, the aggregation rate of an antibody intermediate or a salt thereof and a thiol group-introduced antibody or a salt thereof is 5% or less. Modification of a thiol group-introduced antibody by compounds used in the present invention can be carried out under conditions (mild conditions) which are less likely to cause denaturation and/or degradation of a protein (e.g., cleavage of an amide bond), since the modification is less likely to cause aggregation of the antibody. The aggregation rate is preferably 4.8% or less, more preferably 4.6% or less, even more preferably 4.4% or less, particularly preferably 4.2% or less, 4.0% or less, 3.8% or less, 3.6% or less, or 3.4% or less. The aggregation rate of the antibody can be measured by size exclusion chromatography (SEC)-HPLC (see, Examples and ACS Omega 2020, 5, 7193-7200).

Details of the antibody intermediate or salt thereof contained in the antibody composition are as described below for the antibody intermediate or salt thereof.

The antibody composition of the present invention may further comprise an antibody used as a raw material for preparing a thiol group-introduced antibody, in addition to (A) an antibody intermediate or salt thereof having a bioorthogonal functional group which may be protected, and (B) a thiol group-introduced antibody or salt thereof. In this case, the properties of the antibody used as a raw material for preparing a thiol group-introduced antibody can be taken over to an "antibody intermediate having a bioorthogonal functional group that may be protected" and a "thiol group-introduced antibody". Thus, the antibody used as a raw material for preparing a thiol group-introduced antibody can be defined similar to "antibody" in "antibody intermediate having a bioorthogonal functional group that may be protected" and "thiol group-introduced antibody".

The antibody composition of the present invention can be obtained by reacting a thiol group-introduced antibody with a compound represented by the "leaving group-L-R" (FIG. 1). By such reactions, the thiol group in the thiol group-introduced antibody and the atom bonded to leaving group in L in the compound represented by the "leaving group-L-R" are bonded, and the leaving group is leaved from the atom bonded to the leaving group to obtain an antibody composition comprising an antibody intermediate or salt thereof (a reacted product) and a thiol group-introduced antibody or salt thereof (an unreacted product). The molar ratio of the compound or salt thereof represented by the "leaving group-L-R" to the thiol group-introduced antibody or salt thereof in the reaction (the compound or salt thereof represented by "leaving group-L-R"/thiol group-introduced antibody or salt thereof) is not particularly limited since it vary depending on factors such as types of the compound or salt thereof represented by the "leaving group-L-R" and the thiol group-introduced antibody. The molar ratio is, for example, from 1 to 100, preferably from 2 to 80, and more preferably from 3 to 50.

Such a reaction can be appropriately carried out under a condition that cannot cause protein denaturation/degradation (e.g., cleavage of amide bond) (mild conditions). For example, such a reaction can be carried out at room temperature (e.g., about 15-30°C) in a suitable reaction system such as buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and even more preferably 30 minutes to 8 hours. For details of such a reaction, see, e.g., G.I.J.L. Bernardes et al., Chem. Rev., 115, 2174(2015); J.L. Bernardes et al., Chem. Asian. J., 4, 630 (2009); B. G. Devices et al., Nat. Commun., 5, 4740(2014); Wagner et al., Bioconjugate. Chem., 25, 825(2014).

The determination of the formation of the antibody intermediate or salt thereof, which depends on its specific raw materials and the molecular weight of a product, can be performed by, for example, reverse phase HPLC under a reduction condition or mass spectrometry.

The antibody compositions of the present invention can contain an antibody intermediate or salt thereof having a bioorthogonal functional group that may be protected at a high content in a low rate of aggregation. The ntibody intermediate or salt thereof having a bioorthogonal functional group that may be protected can be used in the preparation of a conjugate of an antibodty and a functional substance or a salt thereof. Thus, the antibody composition of the present invention can efficiently produce the conjugate of an antibody and a functional substance or a salt thereof in a low aggregation state.

3. A reagent for derivatizing a thiol group-introduced antibody or salt thereof.

The present invention provides a reagent for derivatizing a thiol group-introduced antibody or salt thereof

In one embodiment, the reagent of the present invention comprises a compound or salt thereof represented by the following formula (I):

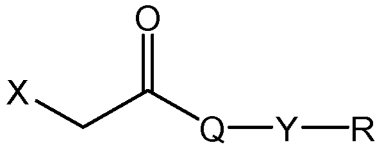 (I)

wherein

X is a halogen atom,
Q is arylene or alkylene,
Y is a bond or a divalent group, and
R is a bioorthogonal functional group which may be protected.

The halogen atom represented by X in formula (I) is the same as that described above.

The arylene represented by Q in formula (I) includes, for example, phenylene, naphthylene, anthracenylene, and phenylene and naphthylene are preferred, and phenylene is preferred. The alkylene represented by Q in formula (I) includes, for example, a linear or branched alkylene, and a linear alkylene is preferred. For the linear alkylene, a linear alkylene having 1 to 6 carbon atoms is preferred, and a linear alkylene having 1 to 4 carbon atoms is more preferred.

The divalent group represented by Y in formula (I) is the same as that described above.

In a specific embodiment, the divalent group represented by Y in formula (I) may be a group having a main chain structure comprising $-(O-R_5)_{m1}$. $R_5$ and m1 are the same as those described above.

The bioorthogonal functional group which may be protected, which is represented by R in formula (I), are the same as that described above.

In another embodiment, the reagent of the present invention comprises a compound or salt thereof represented by formula (II):

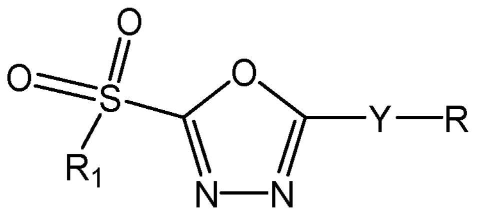 (II)

wherein

$R_1$ is alkyl,
Y is a divalent group, and
R is a bioorthogonal functional group which may be protected.

The alkyl represented by $R_1$ in formula (II) is the same as that described above.

The divalent group represented by Y in formula (II) is the same as that described above.

The bioorthogonal functional group which may be protected, which is represented by R in formula (II), is the same as that described above.

In yet another embodiment, the reagent of the present invention comprises a compound or salt thereof represented by formula (III):

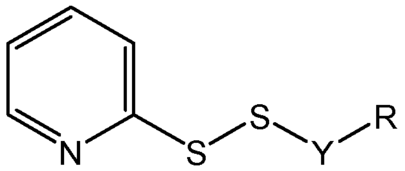

(III)

wherein,

Y is a divalent group, and
R is a bioorthogonal functional group which may be protected.

The divalent group represented by Y in formula (III) is the same as that described above.
The bioorthogonal functional group which may be protected, which is represented by R in formula (III), is the same as that described above.
In yet another embodiment, the reagent of the present invention comprises a compound or salt thereof represented by formula (IV):

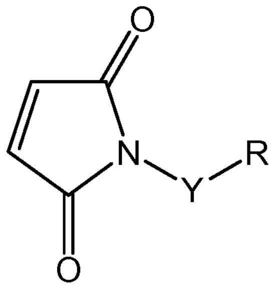

(IV)

wherein

Y is a divalent group, and
R is a bioorthogonal functional group which may be protected.

The divalent group represented by Y in formula (IV) is the same as that described above.
The bioorthogonal functional group which may be protected, which is represented by R in formula (IV), are the same as that described above.
More specifically, the compounds represented by formulae (I)-(IV) may be compounds represented by the following formulae (1)-(12):

(1) 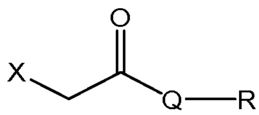

(2) 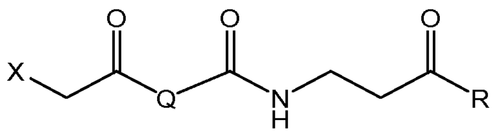

(3) 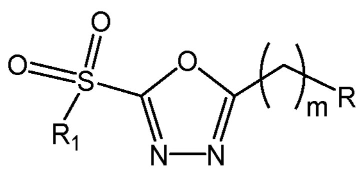

(4)

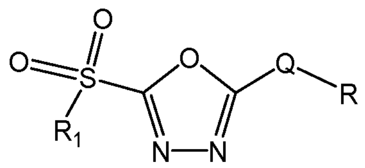

(5)

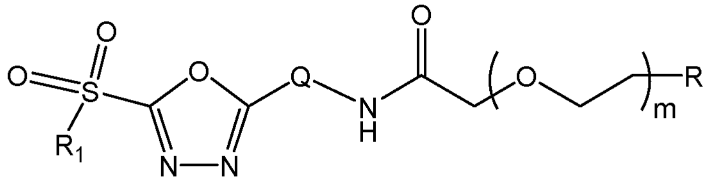

(6)

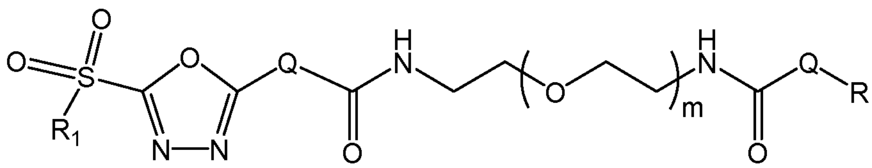

(7)

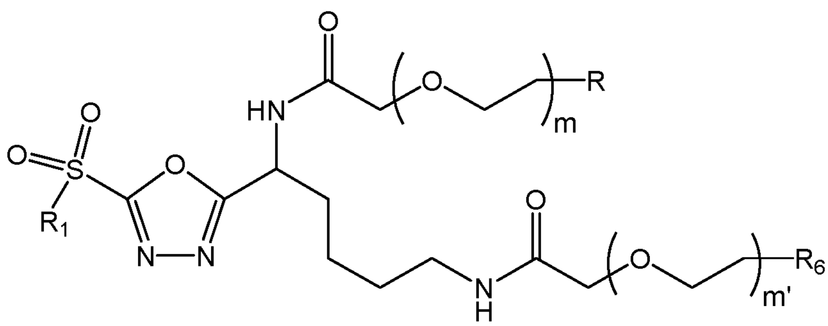

(8)

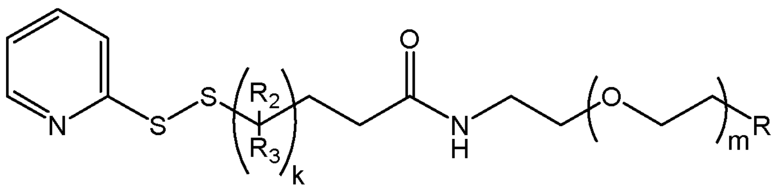

(9)

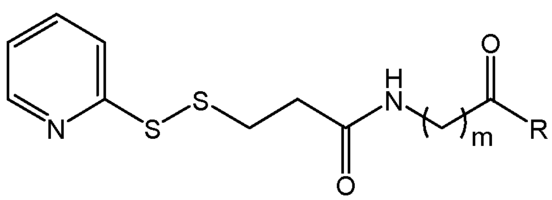

(10)

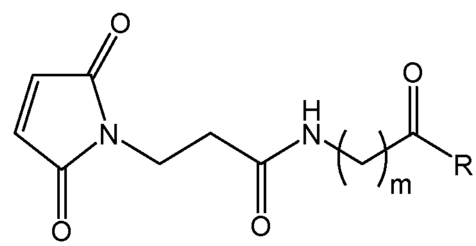

(11) 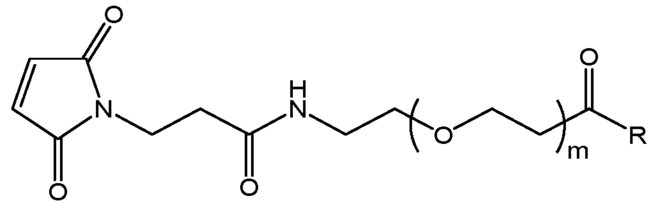

(12) 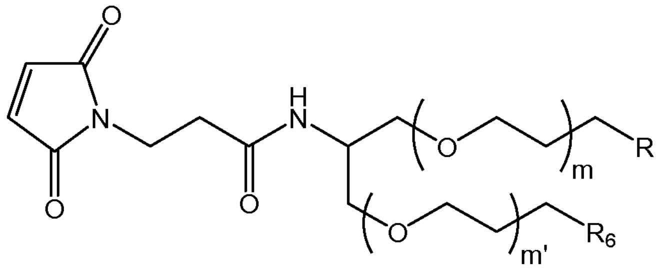

wherein

k is an integer of 0 or 1,
m is an integer from 1 to 5 (preferably an integer from 1 to 3),
m' is an integer from 1 to 5 (preferably an integer from 1 to 3),
$R_2$ and $R_3$ are each independently a hydrogen atom or methyl,
$R_6$ is a bioorthogonal functional group which may be protected, and
X, $R_1$, Q and R are the same as those described above.

The thiol-introduced antibody or salt thereof derivatized with the reagent of the present invention is the same as that described above for a thiol-introduced antibody or antibody. For example, a thiol group in the thiol group-introduced antibody may be regioselectively linked via a linker which does not contain a peptide to an atom in a side chain of an amino acid residue (e.g., an amino acid residue having an easily modified side chain described above) in a constant region of the antibody heavy chain (preferably a nitrogen atom in a side chain of a lysine residue). The thiol group-introduced antibody may also be an IgG antibody. When a human IgG antibody is used as the thiol group-introduced antibody, such lysine residue may be present at one or more positions selected from the group consisting of the position 246/248, the position 288/290, and the position 317 of the human IgG heavy chain according to EU numbering.
The reagent of the present invention can derivatize a thiol group-introduced antibody or salt thereof by reacting with a thiol group in a thiol group-introduced antibody or a salt thereof (preferably a thiol group introduced via a side chain of a lysine residue).
The reagent of the present invention may be provided in the form of a composition further comprising other components. Such other components include, for example, solutions, stabilizers (e.g., antioxidants, preservatives). The solution is preferably an aqueous solution. The aqueous solutions include, for example, water (e.g., distilled water, sterile distilled water, purified water, saline), buffers (e.g., aqueous phosphorate, tris-hydrochloric acid buffer, carbonic acid-bicarbonate buffer, aqueous borate, glycine-sodium hydroxide buffer, citrate buffer), and buffers are preferred. The pH of the solution is, for example, 5.0-9.0, preferably 5.5-8.5. The reagent of the present invention can be provided in a liquid or powdered form (e.g., lyophilized powder)

4. Compound or a salt thereof.

The present invention also provides a compound or salt thereof.
In one embodiment, the compound of the present invention is a compound or salt thereof represented by formula (I):

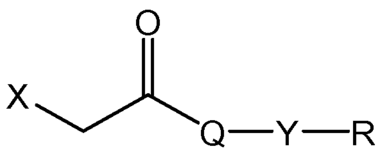 (I)

wherein

X is a halogen atom,
Q is arylene or alkylene,
Y is a divalent group, and
R is a bioorthogonal functional group which may be protected.

In formula (I), the halogen atom represented by X, the arylene or alkylene represented by Q, the divalent group represented by Y, and the bioorthogonal functional group which may be protected, which is represented by R, are the same as those described above.

In another embodiment, the compound of the present invention is a compound or salt thereof represented by formula (II):

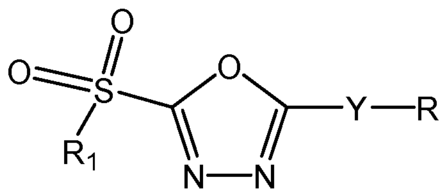
(II)

wherein

$R_1$ is alkyl,
Y is a divalent group, and
R is a bioorthogonal functional group which may be protected.

In formula (II), the alkyl represented by $R_1$, the divalent group represented by Y, and the bioorthogonal functional group which may be protected, which is represented by R, are the same as those described above.

In yet another embodiment, the compound of the present invention is a compound or a salt thereof represented by formula (III):

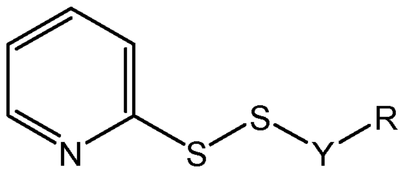
(III)

wherein

Y is a divalent group, and
R is a bioorthogonal functional group which may be protected.

In formula (III), the divalent group represented by Y and the bioorthogonal functional group which may be protected, which is represented by R, are the same as those described above.

In yet another embodiment, the compound of the present invention is a compound or salt thereof represented by formula (IV):

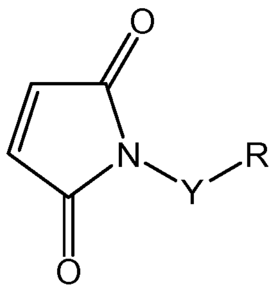

(IV)

wherein

Y is a divalent group, and
R is a bioorthogonal functional group which may be protected.

In formula (IV), the divalent group represented by Y and the bioorthogonal functional group which may be protectged, which is represented by R, are the same as those described above.

More specifically, the compounds represented by formulae (I)-(IV) may be compounds represented by formulae (2)-(12) as above.

The compounds represented by formulae (I)-(IV) can be obtained by synthetic reactions as described in the Examples. For example, such reactions can be carried out at an appropriate temperature (e.g., about 4-90°C) in a suitable organic solvent system. The reaction time is, for example, from 1 minute to 20 hours, preferably from 10 minutes to 15 hours.

The confirmation of the formation of a series of compounds or salts thereof as described above, which depends on its specific materials and the molecular weight of the product, may be performed by, for example, NMR, or mass spectrometry. Such a compound or salt thereof can be suitably purified by any methods such as chromatography, solvent extraction, and recrystallization.

The compound of the present invention or salt thereof can be used to derivatize a thiol-introduced antibody or salt thereof.

5. Antibody intermediate or salt thereof.

The present invention provides an antibody intermediate or salt thereof having a bioorthogonal functional group which may be protected.

In one embodiment, the antibody intermediate of the present invention is an antibody intermediate having a bioorthogonal functional group which may be protected, which is represented by formula (I'):

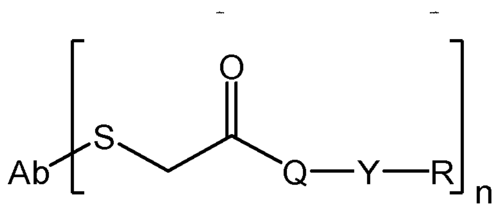

(I')

wherein

Ab is an antibody,
S is a sulfur atom,
Q is arylene or alkylene,
Y is a bond or a divalent group,
R is a bioorthogonal functional group which may be protected, and
n is an integer from 1 to 8.

In formula (I'), the antibody represented by Ab, and the relationship between the antibody and the sulfur atom (S) adjacent to the antibody are similar to those described above for the thiol group-introduced antibody or antibody. For example, a sulfur atom (S) adjacent to an antibody may be regioselectively linked via a linker which does not contain

a peptide to an atom in a side chain of an amino acid residue (e.g., an amino acid residue having an easily modified side chain described above) in a constant region of an antibody heavy chain (perferably a nitrogen atom of the side chain in the lysine residue). The antibody may also be an antibody comprising an immunoglobulin unit comprising two heavy chains and two light chains (preferably an antibody comprising two heavy chains and two light chains and having disulfide bonds between the heavy chains and between the heavy and light chains). Further, the antibody may be an IgG antibody. When a human IgG antibody is used as the antibody, such a lysine residue may be present at one or more positions selected from the group consisting of the position 246/248, the position 288/290, and the position 317 the human IgG heavy chain according to EU numbering. The regioselectivity and degree thereof are similar to those described above. The confirmation of regioselectivity can be confirmed by the method described above.

In formula (I'), n is an integer from 1 to 8, preferably an integer from 1 to 6, more preferably from 1 to 4 (i.e., 1, 2, 3, or 4).

The arylene represented by Q in formula (I') includes, for example, phenylene, naphthylene, anthracenylene, and phenylene and naphthylene are preferred, and phenylene is perfered. The alkylene represented by Q in formula (I') includes, for example, a linear or branched alkylene, and a linear alkylene is preferred. For the linear alkylene, a linear alkylene having 1 to 6 carbon atoms is preferred, and a linear alkylene having 1 to 4 carbon atoms is more preferred.

In formula (I'), the divalent group represented by Y and the bioorthogonal functional group which may be protected, which is represented by R, are the same as those described above.

In a specific embodiment, the divalent group represented by Y in formula (I') may be a group having a main chain structure comprising $-(O-R_5)_{m1}$. $R_5$ and m1 are the same as those described above.

In another embodiment, the antibody intermediate of the present invention is an antibody intermediate having a bioorthogonal functional group which may be protected, which is represented by formula (II'):

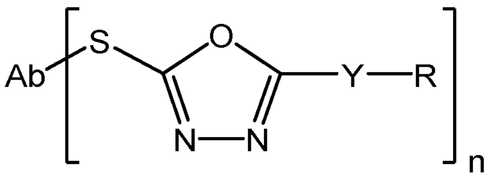

(II')

wherein

Ab is an antibody,
S is a sulfur atom,
Y is a bond or a divalent group,
R is a bioorthogonal functional group which may be protected, and
n is an integer from 1 to 8.

In formula (II'), the antibody represented by Ab, a relationship between the antibody and the sulfur atom (S) adjacent to the antibody, the integer represented by n, the divalent group represented by Y, and the bioorthogonal functional group which may be protected, which is represented by R, are the same as those described above.

In yet another embodiment, the antibody intermediate of the present invention is an antibody intermediate having a bioorthogonal functional group which may be protected, which is represented by formula (III'):

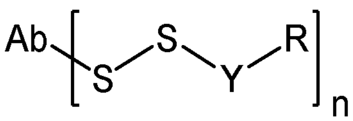

(III')

wherein

Ab is an antibody,
S is a sulfur atom,
Y is a bond or a divalent group,
R is a bioorthogonal functional group which may be protected, and
n is an integer from 1 to 8.

In formula (III'), the antibody represented by Ab, the relationship between the antibody and the sulfur atom (S) adjacent to the antibody, the integer represented by n, the divalent group represented by Y, and the bioorthogonal

functional group which may be protected, which is represented by R, are the same as those described above.

In yet another embodiment, the antibody intermediate of the present invention is an antibody intermediate having a bioorthogonal functional group which may be protected, which is represented by formula (IV'):

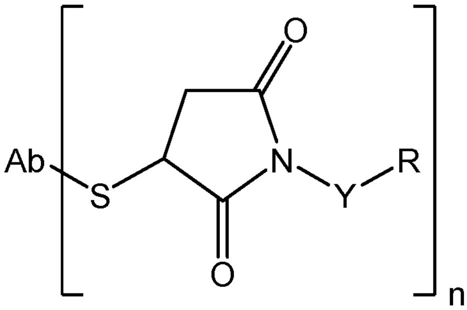

(IV')

wherein

Ab is an antibody,
S is a sulfur atom,
Y is a bond or a divalent group,
R is a bioorthogonal functional group which may be protected, and
n is an integer from 1 to 8.

In formula (IV'), the antibody represented by Ab, the relationship between the antibody and the sulfur atom (S) adjacent to the antibody, the integer represented by n, the divalent group represented by Y, and the bioorthogonal functional group which may be protected, which is represented by R, are the same as those described above.

More specifically, the antibody intermediate represented by formula (I')-(IV ') may be an antibody intermediate represented by formula (1')-(12'):

(1')

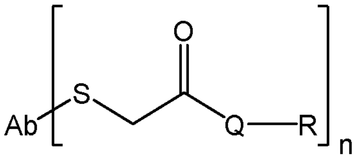

(2')

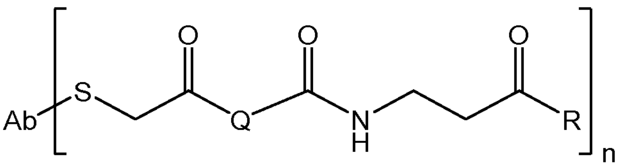

(3')

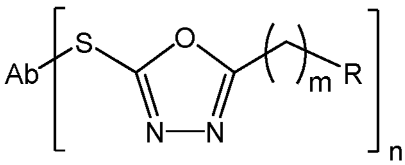

(4')

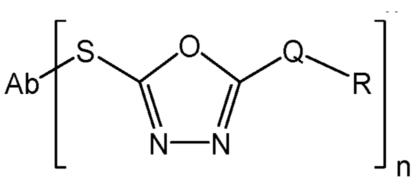

(5') 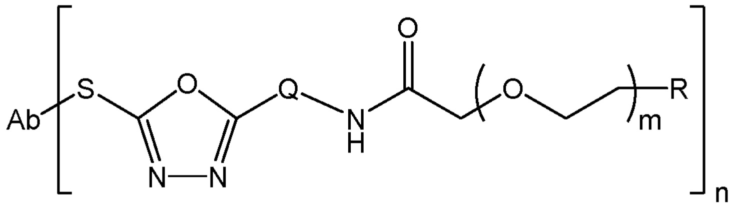

(6') 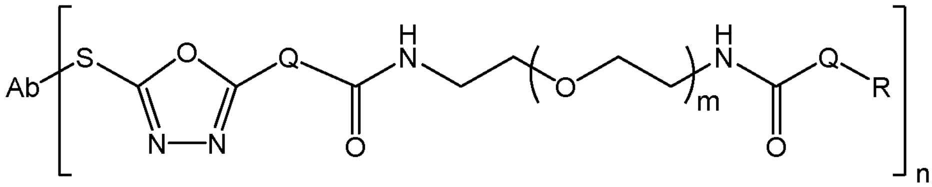

(7') 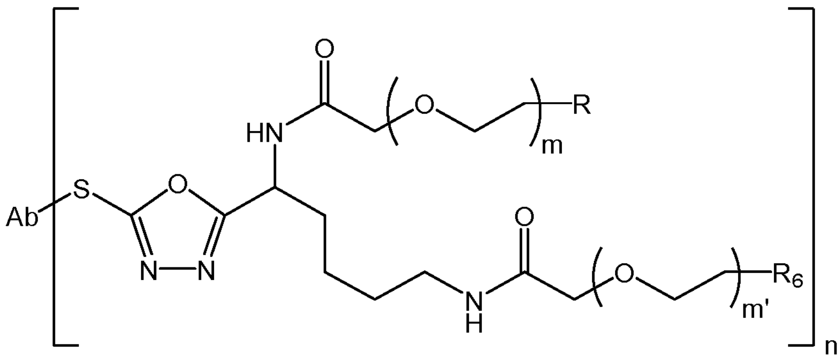

(8') 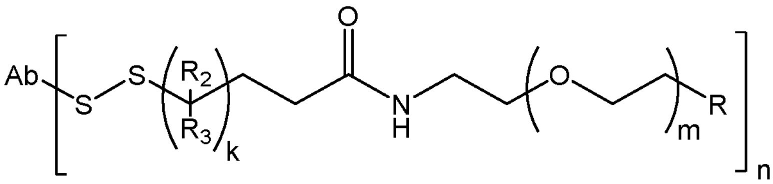

(9') 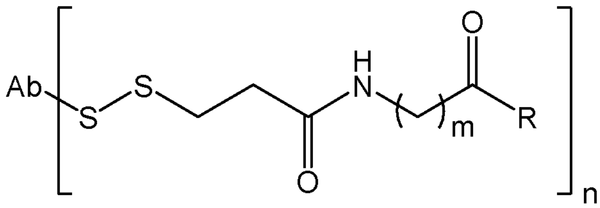

(10') 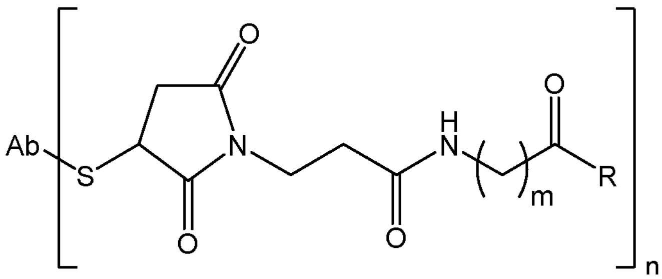

(11') 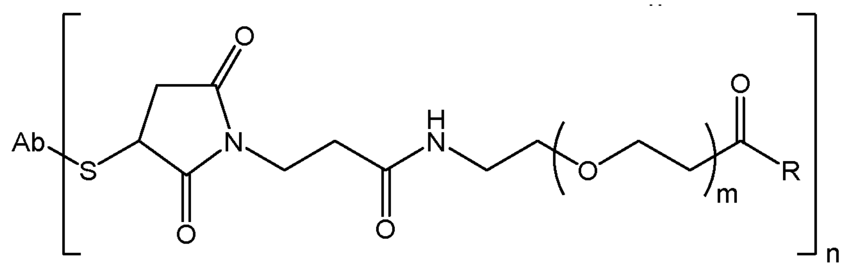

(12') 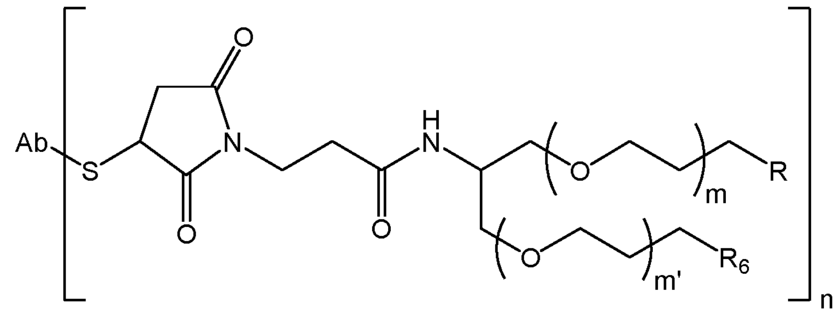

wherein

k is an integer of 0 or 1,
m is an integer from 1 to 5 (perferably an integer from 1 to 3),
m' is an integer from 1 to 5 (perferably an integer from 1 to 3),
$R_2$ and $R_3$ are each independently a hydrogen atom or methyl,
$R_6$ is a bioorthogonal functional group which may be protected, and
Ab, S adjacent to Ab, Q, R and n are the same as those described above.

The aggregation rate of the antibody intermediates or salts thereof of the present invention may be 5% or less. Modification of a thiol group-introduced antibody by compounds used in the present invention can be carried out under conditions (mild conditions) which are less likely to cause denaturation and/or degradation of a protein (e.g., cleavage of an amide bond), since the modification is less likely to cause aggregation of the antibody. The aggregation rate is preferably 4.8% or less, more preferably 4.6% or less, even more preferably 4.4% or less, particularly preferably 4.2% or less, 4.0% or less, 3.8% or less, 3.6% or less, or 3.4% or less. The aggregation rate of the antibody can be measured by size exclusion chromatography (SEC)-HPLC (see, Examples and ACS Omega 2020, 5, 7193-7200).

The antibody intermediate or salt thereof of the present invention can also have high stability in blood.

The antibody intermediate of the present invention can be obtained by reacting a thiol group-introduced antibody with a compound represented by formula (I)-(IV) or a salt thereof (FIG. 1). The molar ratio of a compound represented by formulae (I)-(IV) or a salt thereof to a thiol group-introduced antibody or a salt thereof in a reaction (a compound represented by formula (I)-(IV) or a salt/thiol group-introduced antibody or a salt thereof) is not particularly limited since it varies depending on factors such as the type of the compounds represented by formulae (I)-(IV) or salts thereof and the thiol group-introduced antibody, and is, for example, 1-100, preferably, from 2 to 80, more preferably from 3 to 50.

Such a reaction can be appropriately carried out under a condition that cannot cause protein denaturation/degradation (e.g., cleavage of amide bond) (mild conditions). For example, such a reaction can be carried out at room temperature (e.g., about 15-30°C) in a suitable reaction system such as buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and even more preferably 30 minutes to 8 hours. For details of such a reaction, see, e.g., G.I.J.L. Bernardes et al., Chem. Rev., 115, 2174(2015); J.L. Bernardes et al., Chem. Asian. J., 4, 630 (2009); B. G. Devices et al., Nat. Commun., 5, 4740(2014); Wagner et al., Bioconjugate. Chem., 25, 825(2014).

Confirmation of the production of an antibody intermediate or a salt thereof, depending on its specific materials and the molecular weight of the product, can be carried out, for example, by reverse phase HPLC under reduction conditions or by mass spectrometry.

The antibody intermediate of the present invention can be used to prepare a conjugate of an antibody and a functional substance or a salt thereof.

6. Conjugate of antibody and functional substance or salt thereof

The present invention provides a conjugate of an antibody and a functional substance or a salt thereof.

In one embodiment, the conjugate of the invention is a conjugate of an antibody and a functional substance, which is represented by formula (I"):

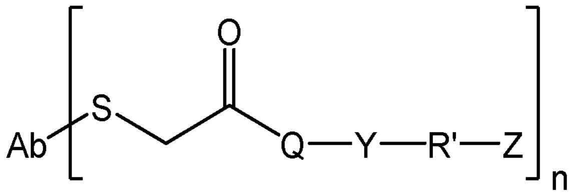

(I")

wherein

Ab is an antibody,
S is a sulfur atom,
Q is arylene or alkylene,
Y is a bond or a divalent group,
R' is a group produced by the reaction between two bioorthogonal functional groups capable of reacting with each other,
Z is a functional substance, and
n is an integer from 1 to 8.

In formula (I"), the antibody represented by Ab, and the relationship between the antibody and the sulfur atom (S) adjacent to the antibody are the same as those described above for the thiol group-introduced antibody or antibody. For example, the sulfur atom (S) adjacent to the antibody may be regioselectively linked via a linker that does not contain a peptide to an atom in a side chain of an amino acid residue (e.g., an amino acid residue having an easily modified side chain described above) in a constant region of an antibody heavy chain (preferably a nitrogen atom of a side chain in a lysine residues). The antibody may also be an antibody comprising an immunoglobulin unit comprising two heavy chains and two light chains (preferably an antibody comprising two heavy chains and two light chains and having disulfide bonds between the heavy chains and between the heavy and light chains). Further, the antibody may be an IgG antibody. When a human IgG antibody is used as the antibody, such a lysine residue may be present at one or more positions selected from the group consisting of the position 246/248, the position 288/290, and the position 317 of the human IgG heavy chain according to EU numbering. The regioselectivity and degree thereof are similar to those described above. Confirmation of regioselectivity can be confirmed by the method described above.

In formula (I"), n is an integer from 1 to 8, preferably an integer from 1 to 6, more preferably an integer from 1 to 4 (i.e., 1 ,2, 3, or 4).

The group produced by the reaction between two bioorthogonal functional groups capable of reacting with each other, which is represented by R' in formula (I"), is a group produced by the reaction of a bioorthogonal functional group possessed by the antibody intermediate and a bioorthogonal functional group possessed by the functional substance (wherein the bioorthogonal functional groups are selected to be a reactive combination with each other). Since the combination of two bioorthogonal functional groups capable of reacting with each other is well known, one of ordinary skill in the art can suitably select such a combination to properly set a divalent group comprising a moiety that is produced by the reaction of two bioorthogonal functional groups capable of reacting with each other. Combinations of the bioorthogonal functional groups capable of reacting with each other include, for example, a combination of a thiol residue and a maleimide residue, a combination of a furan residue and a maleimide residue, a combination of a thiol residue and a halocarbonyl residue (by a substitution reaction, a halogen is substituted with a thiol), a combination of an alkyne residue (preferably a ring group having a carbon atom triple bond, which may be substituted by a substituent as described above) and an azide residue, a combination of a tetrazine residue and an alkene residue, a combination of a tetrazine residue and an alkyne residue, a combination of a thiol residue with another thiol residue (disulfide bond). Thus, said moiety may be a group produced by the reaction of a thiol residue with a maleimide residue, a group produced by the reaction of a furan residue with a maleimide residue, a group produced by the reaction of a thiol residue with a halocarbonyl residue, a group produced by the reaction of an alkyne residue with an azide residue, or a group produced by the reaction of a tetrazine residue with an alkene residue, or a disulfide group produced by the reaction of a thiol residue and another thiol residue. Combinations of the bioorthogonal functional groups capable of reacting with each other include, for example, a combination of an alkyne residue (preferably a ring group having a triple bond between carbon atoms, which

may be substituted by a substituent as described above) and an azide, a combination of a thiol and a maleimide, a combination of a tetrazine and an azide, and a combination of furan and maleimide. Preferably, the combination of the bioorthogonal functional groups capable of reacting with each other may be a combination of an alkyne residue (preferably a ring group having a triple bond between carbon atoms, which may be substituted by a substituent as described above)) and an azide residue. When the combination of an alkyne residue and azide is used as two bioorthogonal functional groups capable of reacting with each other, R’ is a divalent triazole ring group. When the combination of a cyclic group having a triple bond between carbon atoms and an azide is used as two bioorthogonal functional groups capable of reacting with each other, R’ is a divalent cyclic group in which the ring group and the triazole ring are condensed. Since a ring group having a triple bond between carbon atoms may be substituted by a substituent as described above, a divalent ring group in which the ring group and the triazole ring are condensed may also be substituted by a substituent as described above.

In certain embodiments, the group produced by the reaction between two bioorthogonal functional groups capable of reacting with each other may be a divalent group represented by the structural formula of any one of the following structural formulas:

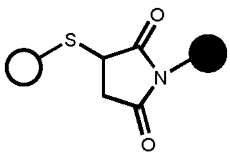
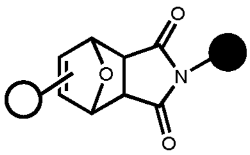
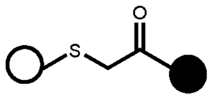
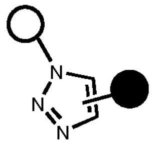
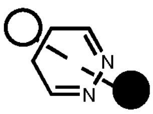
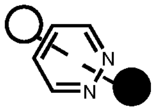
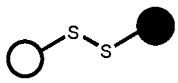

where the open circle and the filled circle represent the bond. When the bond of the white circle is bonded to an atom present on the Ab bonding side, the bond of the filled circle may be bonded to Z. When the bond of the white circle is bonded to Z, the bond of the filled ciercle may be bonded to the atom present on the Ab bonding side.

The arylene represented by Q in formula (I") includes, for example, phenylene, naphthylene and anthracenylene, and phenylene and naphthylene are prefered, and phenylene is prefered. The alkylene represented by Q in formula (I") includes, for example, a linear or branched alkylene, and a linear alkylene is preferred. For the linear alkylene, a linear alkylene having 1 to 6 carbon atoms is preferred, and a linear alkylene having 1 to 4 carbon atoms is more preferred.

In formula (I"), the divalent group represented by Y and the functional substance represented by Z are the same as those described above.

In a specific embodiment, the divalent group represented by Y in formula (I") may be a group having a main chain structure comprising $-(O-R_5)_{m1}$. $R_5$ and m1 are the same as those described above.

In another embodiment, the conjugate of the invention is a conjugate of an antibody and a functional substance, which is represented by formula (II"):

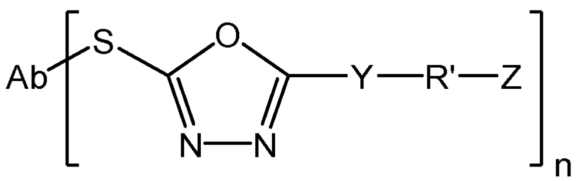

(II")

wherein

- Ab is an antibody,
- S is a sulfur atom,
- Y is a bond or a divalent group,
- R’ is a group produced by the reaction between two bioorthogonal functional groups capable of reacting with each other,
- Z is a functional substance, and
- n is an integer from 1 to 8.

In formula (II"), the antibody represented by Ab, and the relationship between the antibody and the sulfur atom (S) adjacent to the antibody, the integer represented by n, the divalent group represented by Y, the group produced by a reaction of two bioorthogonal functional groups capable of reacting with each other, which is represented by R', and the functional substance represented by Z are the same as those described above.

In yet another embodiment, the conjugate of the invention is a conjugate of an antibody and a functional substance, which is represented by formula (III"):

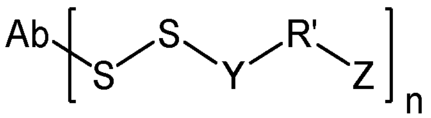

(III")

wherein

Ab is an antibody,
S is a sulfur atom,
Y is a bond or a divalent group,
R' is a group produced by the reaction between two bioorthogonal functional groups capable of reacting with each other,
Z is a functional substance, and
n is an integer from 1 to 8.

In formula (III"), the antibody represented by Ab, and the relationship between the antibody and the sulfur atom (S) adjacent to the antibody, the integer represented by n, the divalent group represented by Y, the group produced by a reaction between two bioorthogonal functional groups capable of reacting with each other, which is represented by R', and the functional substance represented by Z are the same as those described above.

In yet another embodiment, the conjugate of the invention is a conjugate of an antibody and a functional substance represented by formula (IV"):

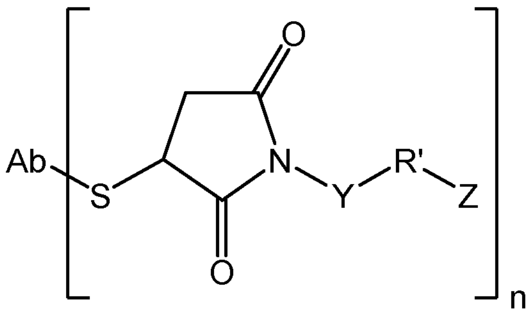

(IV")

wherein

Ab is an antibody,
S is a sulfur atom,
Y is a bond or a divalent group,
R' is a group produced by the reaction between two bioorthogonal functional groups capable of reacting with each other,
Z is a functional substance, and
n is an integer from 1 to 8.

An antibody represented by Ab in formula (IV "), and an antibody and a sulfur atom (S) adjacent thereto, an integer represented by n, a divalent group represented by Y, a group generated by a reaction between two mutually reactive bio-orthogonal functional groups represented by R', and a functional substance represented by Z are the same as those described above.

More specifically, the conjugate represented by formula (1")-(12") may be a conjugate represented by (1")-(12"):

(1'')

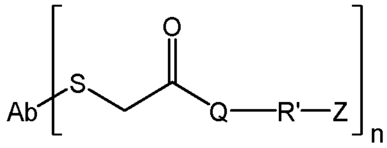

(2'')

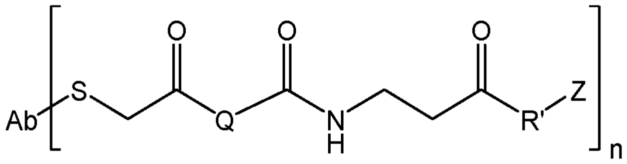

(3'')

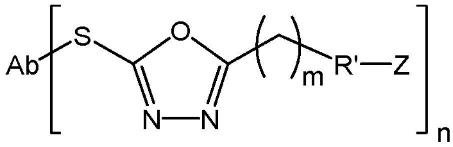

(4'')

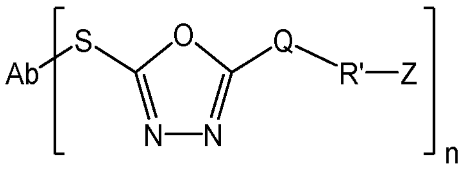

(5'')

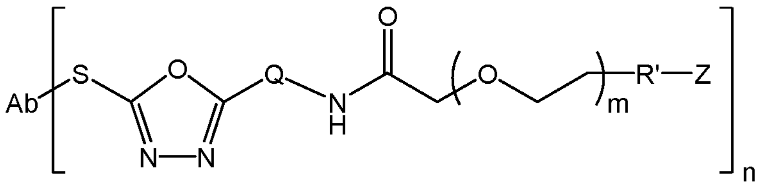

(6'')

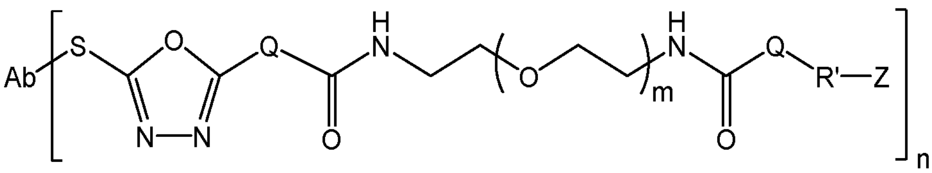

(7'')

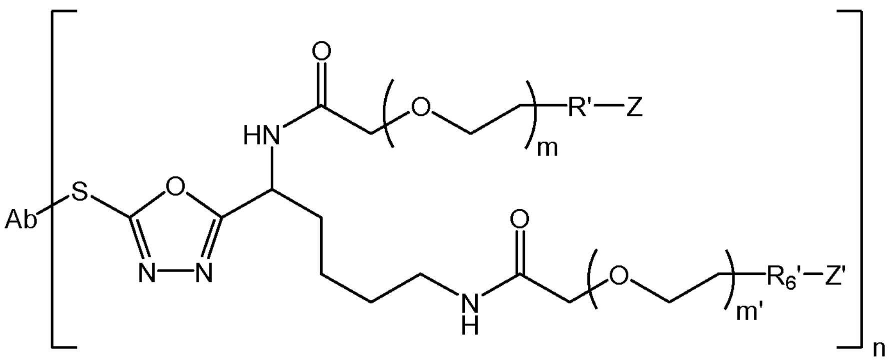

(8'') 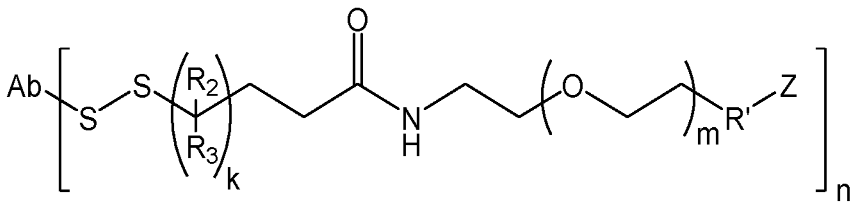

(9'') 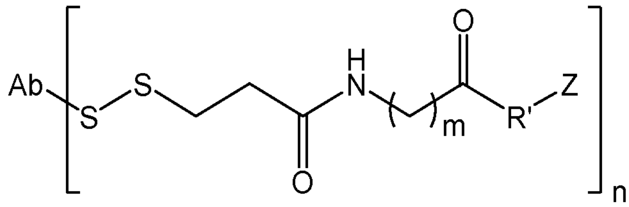

(10'') 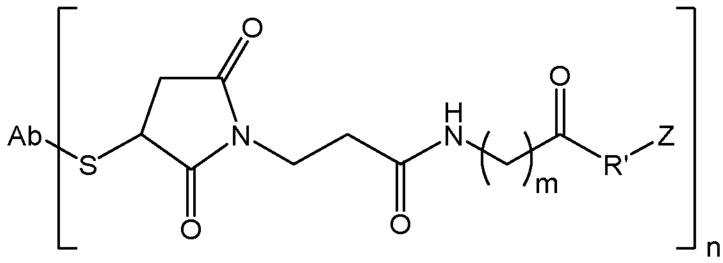

(11'') 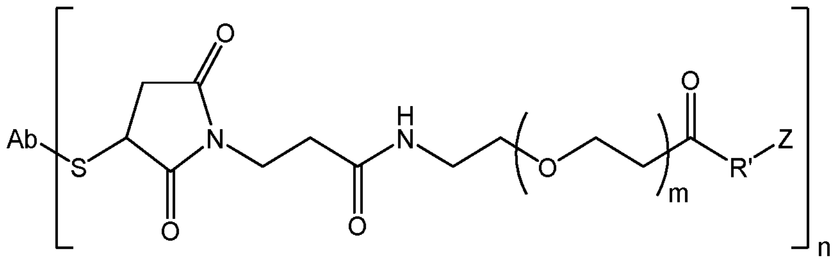

(12'') 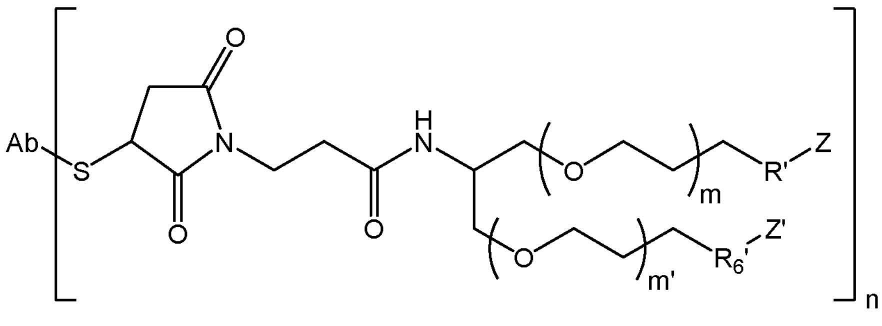

wherein

k is an integer of 0 or 1,
m is an integer from 1 to 5 (preferably an integer from 1 to 3),
m' is an integer from 1 to 5 (preferably an integer from 1 to 3),
$R_2$ and $R_3$ are each independently a hydrogen atom or methyl,
$R_6$' is a group produced by a reaction between two bioorthogonal functional groups capable of reacting with each other,
Z' is a functional substance, and
S, Q, R', Z and n adjacent to Ab, Ab are the same as those described above.

The aggregation rate of the conjugate or salt thereof of the present invention may be 10% or less since the antibody intermediate or salt thereof used in the present invention have an aggregation rate of 5% or less, and since the conjugate or salt thereof of the present invention can be obtained by subjecting the antibody intermediate or salt thereof to a reaction under conditions (mild conditions) that cannot cause denaturation and/or degradation of a protein (e.g., cleavage of an amide bond), which is less likely to cause aggregation of the antibody. The aggregation rate may be preferably 9% or less, more preferably 8.5% or less, even more preferably 8.0% or less, particularly preferably 7.5% or less, 7.0% or less, 6.5% or less, 6.0% or less, 5.5% or less, 5% or less, 4.8% or less, 4.6% or less, 4.4% or less, 4.2% or less, 4.0% or less, 3.8% or less, 3.6% or less, or 3.4% or less. The aggregation rate of the antibody can be measured by size exclusion chromatography (SEC)-HPLC (see Examples and ACS Omega 2020, 5, 7193-7200).

The conjugates or salts thereof of the invention can also have high stability in blood.

The conjugate or salt thereof of the invention can be obtained by reacting an antibody intermediate or a salt thereof of the invention with a functional substance. Such reactions can be carried out under conditions which cannot cause denaturation and/or degradation of a protein (immunoglobulin/antibody) (e.g., the cleavage of an amide bond) (e.g., mild conditions as described above). In the reaction, the molar ratio of the functional substance to the antibody intermediate or salt thereof (functional substance/antibody intermediate or salt thereof) is not particularly limited because it varies depending on factors such as types of antibody intermediate or salt thereof and functional substances and reaction times, and is, for example, 2 or more, preferably 3 or more, and more preferably 5 or more. A sufficient amount (e.g., excess amount) of functional substances to the antibody intermediate or salt thereof can be used to sufficiently react the functional substance with the thiol group of the antibody intermediate in a short reaction time.

Confirmation of the formation of a conjugate or a salt thereof, which depends on its specific raw materials and the molecular weight of the product, can be carried out, for example, by reverse phase HPLC under reduction conditions or by mass spectrometry. The conjugate or salt thereof can be suitably purified by any method such as chromatography (e.g., affinity chromatography).

The conjugate or salt thereof of the present invention can be used as pharmaceuticals or reagents (e.g., diagnostic reagents and reagents for research), for example.

The conjugateor salt thereof of the present invention may be provided in the form of a pharmaceutical composition. The pharmaceutical composition may comprise a pharmaceutically acceptable carrier in addition to the antibody having a functional substance or functional substances, or a salt thereof. Examples of the pharmaceutically acceptable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The conjugate or salt thereof of the present invention may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set as appropriate.

## EXAMPLES

Next, the present invention will be described in more detail with reference to Examples, but the present invention is not limited to the following Examples.

Comparative Example 1: Introduction of an azide group by N-(4-azidophenyl)-2-iodo-acetamide

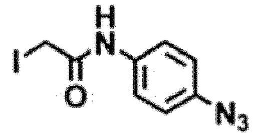

In the following comparative examples and examples, the antibody derivative (thiol-group-introduced trastuzumab) described in Examples 81-7 of WO 2019/240287 (WO2019/240287A1) was used as the thiol-group-introduced antibody. This antibody derivative has the following structure in which a thiol group is regioselectively introduced into trastuzumab (humanized IgG1 antibody) via the amino-group of the side chain of the lysine residue at position 246 or 248 of the antibody heavy chain (the position of the lysine residue follows EU numbering):

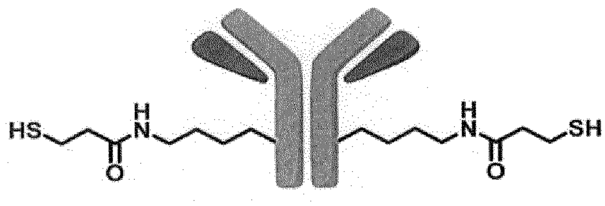

(In the above structure, NH-$CH_2$-$CH_2$-$CH_2$-$CH_2$- extending from the antibody heavy chain corresponds to the side chain of the lysine residue, and the thiol-containing group HS-$CH_2$-$CH_2$-C(=O) is added to the amino group in the side chain of the lysine residue.)

To a solution (50 μM) of a buffer (pH 8.0, HEPES buffer) containing a thiol group-introduced antibody, DMF solution of 20 equivalents of N-(4-azidophenyl)-2-iodoacetamide (12.5 mM) and 50 equivalents of sodium iodide in buffer (pH 8.0, HEPES buffer) (100 mM) were added, and the mixture was allowed to stand at 37°C for 2 hours, and then the product was purified using NAP-5 Columns (GE Healthcare).

To the product was added tris(2-carboxyethyl)phosphine hydrochloride (0.5 mM) and the mixture was stirred at room temperature for 10 minutes and measured by ESI-TOFMS according to the previous report (WO2019/240287A1). The source thiol group-introduced antibody had heavy chain peaks at 50683 and 50845, and a light chain peak at 23439, and the product had heavy chain peaks at 50831, 50993, and 50683 and 50845, respectively, in which the azide group was introduced into the heavy chain, and the light chain peaks at 23439.

Comparative Example 2: Introduction of an azide group by N-[2-[2-(2-azidoethoxy)ethoxy]ethyl]-2-iodoaacetamide)

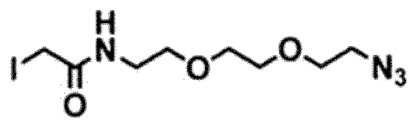

To a thiol-group-introduced buffer (pH 8.0, HEPES buffer) solution (50 μM), 50 equivalents of N-[2-[2-(2-azidoethoxy)ethoxy]ethyl]-2-iodoacetamide (31.3 mM) in DMF solution and 50 equivalents of sodium iodide buffer (pH 8.0, HEPES buffer) solution (100 mM) were added, and the solution was allowed to stand for 2 hours at 37°C, and the product was purified using NAP-5 Columns (GE Healthcare).

To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody had heavy chain peaks at 50683 and 50845, and a light chain peak at 23439, and the product had heavy chain peaks at 50896, 51058 and 50683 and 50845, and light chain peaks at 23439.

Example 1: Introduction of azide groups by azide linker (1)

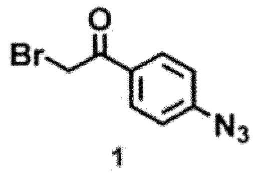

1

To a thiol-group-introduced antibody in buffer (pH 7.4, PBS buffer and 10 mM EDTA) solution (20 μM), an DMF solution (1.25 mM) of 5 equivalent amounts of an azide linker (1, manufactured by Aldrich) was added, and the mixture was allowed to stand at room temperature for 1 hour, followed by purification using a NAP-5 Columns (GE Healthcare).

To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody showed heavy chain peaks at 50683 and 50845 and light chain peaks at 23439, and the products showed heavy chain peaks at 50816 and 50978 in which an azide group(s) was introduced to a heavy chain(s) and a light chain peak at 23439 which were the same as the source.

Example 2: Introduction of azide groups by azide linker (2)

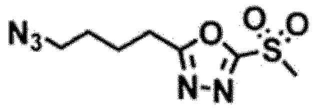

2

(1) Synthesis of Azide Linker (2)

(1-1) Synthesis of 5-azidopentanehydrazide

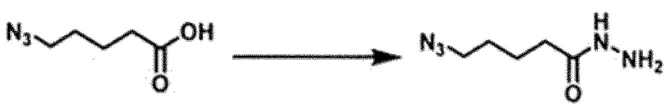

5-azidopentanoic acid (200 mg, 1.4 mmol) in THF solution (10 mL) was cooled to 0°C and N-methylmorpholine (0.32 mL, 2.1 mmol) and isobutyl chloroformate (0.22 mL, 1.7 mmol) were added. After stirring at 0°C for 30 minutes, hydrazine monohydrate (0.087 mL, 2.8 mmmol) and DIPEA (0.71 mL, 4.2 mmol) were added. After stirring at room temperature for 16 hours, concentration was performed under reduced pressure, and the obtained crude product was purified by column chromatography. The fraction containing the product was collected and concentrated under reduced pressure to give 5-(4-azidobutyl)-1,3,4-oxadiazole-2-thiol (111 mg, 0.71 mmol, yield 51%).

MS(ESI) m/z: 158[M+H]

(1-2) Synthesis of 5-(4-azidobutyl)-1,3,4-oxadiazole-2-thiol

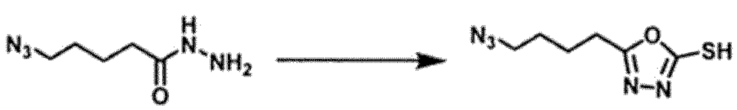

To 5-azidopentanehydrazide (111 mg, 0.71 mmol) in an ethanol solution (7.1 mL) were added carbon disulfide (0.055 mL, 0.92 mmol) and potassium hydroxide (192 mg,3.4 mmol), and the mixture was stirred at 70°C for 16 hours, and then an aqueous solution of 6 M hydrochloric acid was added to adjust pH in the system to 3.0. After the adjustment, the obtained crude product was purified by column chromatography by performing separation extraction with ethyl acetate and concentrating the organic phase under reduced pressure. The fraction containing the product was collected and concentrated under reduced pressure to give 5-(4-azidobutyl)-1,3,4-oxadiazole-2-thiol (35 mg, 0.18 mmol, yield 25%).
MS(ESI) m/z: 200[M+H]

(1-3) Synthesis of 2-(4-azidobutyl)-5-methylsulfanyl-1,3,4-oxadiazole

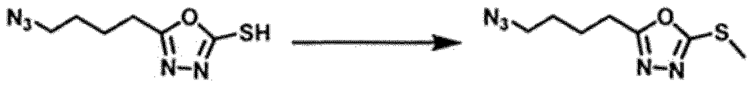

To 5-(4-azidobutyl)-1,3,4-oxadiazole-2-thiol (35 mg, 0.18 mmol) in THF solution (1.8 mL) was added iodomethane (0.033 mL, 0.53 mmol) and triethylamine (0.074 mL, 0.53 mmol) and the mixture was stirred at room temperature for 16 hours. The crude product obtained by concentrating the reaction solution under reduced pressure was purified by column chromatography. The fraction containing the product was collected and concentrated under reduced pressure to give 2-(4-azidobutyl)-5-methylsulfanyl-1,3,4-oxadiazole (33 mg, 0.15 mmol, yield 85%).
MS(ESI) m/z: 214[M+H]

(1-4) Synthesis of Azide Linker (2)

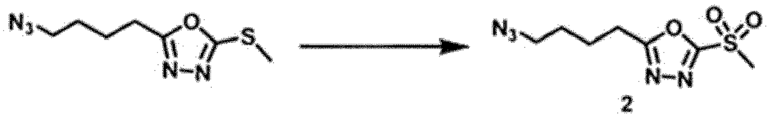

2

2-(4-azidobutyl)-5-methylsulfanyl-1,3,4-oxadiazole (33 mg, 0.15 mmol) in dichloromethane (1.5 mL) was cooled to 0°C and mCPBA (0.311 g, 1.3 mmol) was added. After stirring at room temperature for 20 hours, a saturated aqueous sodium bicarbonate solution was added, and the mixture was extracted with ethyl acetate. The crude product obtained by concentrating the organic layer under reduced pressure was purified by column chromatography. The fraction containing the product was collected and concentrated under reduced pressure to give the azide linker (2) (26 mg, 0.11 mmol, yield 71%).
MS(ESI) m/z: 246[M+H]

(2) Introduction of Azide Group by Azide Linker (2)

To a solution (20 μM) of the thiol-group-introduced antibody in buffer (pH 7.4, PBS buffer, 10 mM EDTA), 5 equivalents of an azide linker (2) in a DMF solution (1.25 mM) were added, and the mixture was allowed to stand at room temperature for 1 hour, followed by purification using a NAP-5 Columns (GE Healthcare).
To the product was added tris(2-carboxyethyl)phosphine hydrochloride (0.5 mM) and the mixture was stirred at room temperature for 10 minutes and the product was measured by ESI-TOFMS according to WO2019/240287 A1. The source thiol group-introduced antibody had heavy chain peaks at 50683 and 50845 and a light chain peak at 23439, and the products had heavy chain peaks at 50846 and 51008 in which an azide group(s) was introduce to a heavy chain(s), and a light chain peak at 23439 which was the same as the source.

Example 3: Introduction of Azide Group by Azide Linker (3)

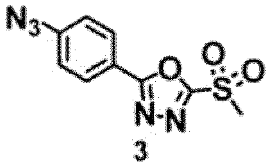

3

(1) Synthesis of Azide Linker (3)

(1-1) Synthesis of 2-(4-azidophenyl)-5-methylsulfanyl-1,3,4-oxadiazole

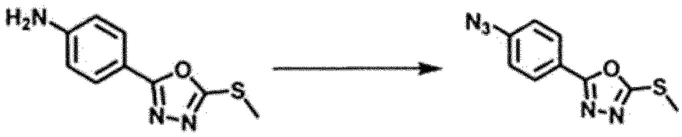

To 4-(5-methylsulfanyl-1,3,4-oxadiazol-2-yl)aniline trifluoroacetate (48 mg, 0.15 mmol) in dichloromethane solution (3.0 mL) was added 2-azido-1,3-dimethylimidazolinium hexafluorophosphate (137 mg, 0.48 mmol) and DMAP (86 mg, 0.70 mmol) and the mixture was stirred at 50°C for 16 h. The crude product obtained by concentrating the reaction solution under reduced pressure was purified by column chromatography. The fraction containing the product was collected and concentrated under reduced pressure to give 2-(4-azidophenyl)-5-methylsulfanyl-1,3,4-oxadiazole (26 mg, 0.11 mmol, yield 73%) .

MS(ESI) m/z: 234[M+H]

(1-2) Synthesis of Azide Linker (3)

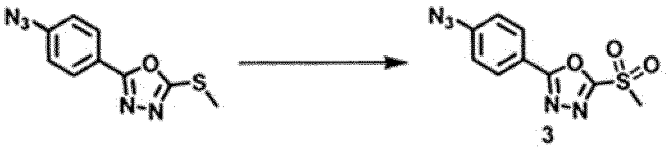

3

2-(4-azidophenyl)-5-methylsulfanyl-1,3,4-oxadiazole (26 mg, 0.11 mmol) in dichloromethane solution (1.1 mL) was cooled to 0°C and mCPBA (113 mg, 0.45 mmol) was added. After stirring at room temperature for 20 hours, a saturated aqueous sodium bicarbonate solution was added, and the product was extracted with ethyl acetate. The crude product obtained by concentrating the organic layer under reduced pressure was purified by column chromatography. The fractions containing the product were collected and concentrated under reduced pressure to give the azide linker (3) (23 mg, 0.086 mmol, yield: 78%).

MS(ESI) m/z: 266[M+H]

(2) Introduction of Azide Group by Azide Linker (3)

The azide linker (3) was reacted with the thiol group-introduced antibody in the same manner as described in Example 2 (2).

To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody had heavy chain peaks at 50683 and 50845 and a light chain peak at 23439, and the products had light chain peaks at 50841 and 51002 in which an azide group(s) was introduce to a heavy chain(s), and a light chain peak at 23439 which was the same as the source.

Example 4: Introduction of azide group by azide linker (4)

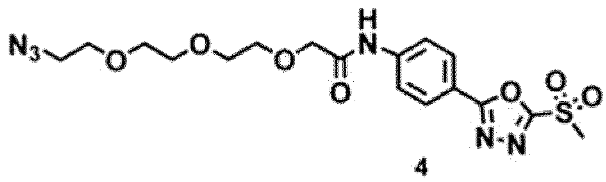

4

(1) Synthesis of Azide Linker (4)

(1-1) Synthesis of tert-butyl N-[4-(hydrazinecarbonyl)phenyl] carbamate

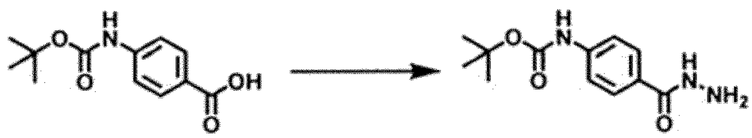

4-(tert-butoxycarbonylamino)benzoic acid (200 mg, 0.84 mmol) in THF solution (8.4 mL) was cooled to 0°C and N-methylmorpholine (0.14 mL, 1.3 mmol) and isobutyl chloroformate (0.13 mL, 1.0 mmol) were added. After stirring at 0°C for 30 minutes, hydrazine monohydrate (0.078 mL, 2.5 mmmol) and DIPEA (0.30 mL, 1.7 mmol) were added. After stirring at room temperature for 16 hours, concentration was performed under reduced pressure, and the obtained crude product was purified by column chromatography. The fraction containing the product was collected and concentrated under reduced pressure to give tert-butyl N-[4-(hydrazinecarbonyl)phenyl]carbamate (187 mg, 0.74 mmol, yield 88%).

MS(ESI) m/z: 252[M+H]

(1-2) Synthesis of tert-butyl N-[4-(5-sulfanyl-1,3,4-oxadiazol-2-yl)phenyl]carbamate

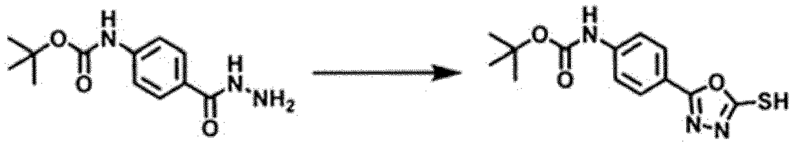

To tert-butyl N-[4-(hydrazinecarbonyl)phenyl] carbamate (187 mg, 0.74 mmol) in ethanol solution (7.4 mL), carbon disulfide (0.058 mL, 0.97 mmol) and potassium hydroxide (125 mg, 2.2 mmol) were added, and the mixture was stirred at 75°C for 16 hours, and then an aqueous solution of 6 M hydrochloric acid was added to adjust pH in the system to 3.0. After the adjustment, the obtained crude product was purified by column chromatography by performing separation extraction with ethyl acetate and concentrating the organic phase under reduced pressure. The fraction containing the product was collected and concentrated under reduced pressure to give tert-butyl N-[4-(5-sulfanyl-1,3,4-oxadiazol-2-yl)phenyl]carbamate (99 mg, 0.34 mmol, yield 45%) .

MS(ESI) m/z: 294[M+H]

(1-3) Synthesis of tert-butyl N-[4-(5-methylsulfanyl-1,3,4-oxadiazol-2-yl)phenyl]carbamate

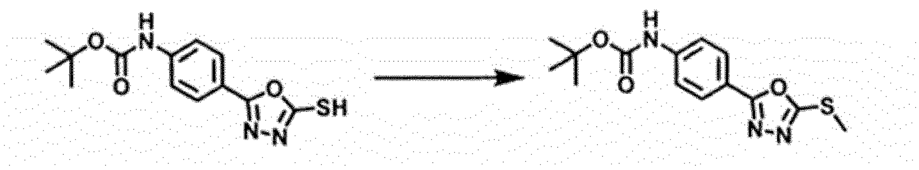

Tert-butyl N-[4-(5-sulfanyl-1,3,4-oxadiazol-2-yl)phenyl]carbamate (99 mg, 0.34 mmol) in THF solution (3.4 mL) was cooled to 0°C, iodomethane (0.063 mL, 1.0 mmol) and triethylamine (0.14 mL, 1.0 mmol) were added, and the mixture was stirred at room temperature for 3 hours. The crude product obtained by concentrating the reaction solution under reduced pressure was purified by column chromatography. The fraction containing the product was collected and concentrated under reduced pressure to give tert-butyl N-[4-(5-methylsulfanyl-1,3,4-oxadiazol-2-yl)phenyl]carbamate (88 mg, 0.29 mmol, yield 84%).

MS(ESI) m/z: 308[M+H]

(1-4) Synthesis of 4-(5-methylsulfanyl-1,3,4-oxadiazol-2-yl)aniline

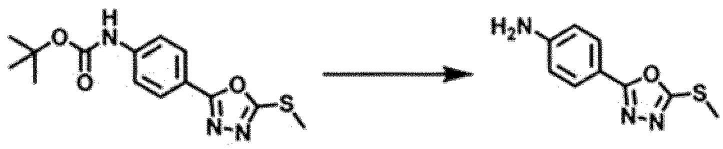

To tert-butyl N-[4-(5-methylsulfanyl-1,3,4-oxadiazol-2-yl)phenyl]carbamate (88 mg, 0.29 mmol) in dichloromethane solution (2.9 mL) was added trifluoroacetic acid (2.9 mL) and the mixture was stirred at room temperature for 1 h. The solution was concentrated under reduced pressure to give 4-(5-methylsulfanyl-1,3,4-oxadiazol-2-yl)aniline trifluoroacetate (93 mg, 0.29 mmol, yield 100%).

(1-5) Synthesis of 2-[2-[2-(2-azidoethoxy)ethoxy]ethoxy]-N-[4-(5-methylsulfanyl-1,3,4-oxadiazol-2-yl)phenyl]acetamide

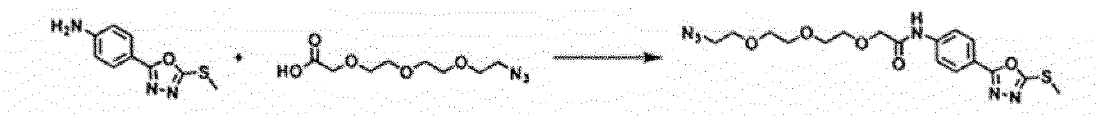

To 4-(5-methylsulfanyl-1,3,4-oxadiazol-2-yl) aniline trifluoroacetate (57 mg, 0.18 mmol) in dichloromethane solution (1.8 mL) was added 2-[2-[2-(2-azidoethoxy)ethoxy]ethoxy]acetic acid (102 mg, 0.43 mmol), EDCI (115 mg, 0.60 mmol), DMAP (9.4 mg, 0.077 mmol) and triethylamine (0.114 mL, 0.82 mmol). After stirring at room temperature for 2 hours, the crude product obtained by concentrating the reaction solution under reduced pressure was purified by column chromatography. The fraction containing the product was collected and concentrated under reduced pressure to give 2-[2-[2-(2-azidoethoxy)ethoxy]ethoxy]-N-[4-(5-methylsulfanyl-1,3,4-oxadiazol-2-yl)phenyl]acetamide (73 mg, 0.17 mmol, yield 94%) .

MS(ESI) m/z: 423[M+H]

(1-6) Synthesis of Azide linker (4)

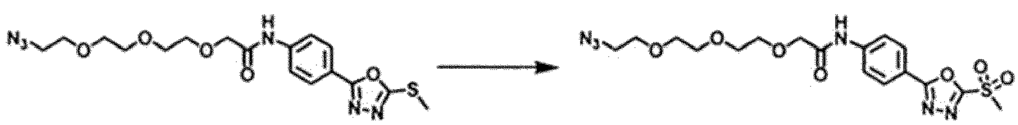

2-[2-[2-(2-azidoethoxy)ethoxy]ethoxy]-N-[4-(5-methylsulfanyl-1,3,4-oxadiazol-2-yl)phenyl]acetamide (73 mg, 0.17 mmol) in dichloromethane solution (1.7 mL) was cooled to 0°C and mCPBA (185 mg, 0.75 mmol) was added. After stirring at room temperature for 20 hours, a saturated aqueous sodium bicarbonate solution was added, and the mixture was subjected to separation extraction with ethyl acetate. The crude product obtained by concentrating the organic layer under reduced pressure was purified by column chromatography. The fractions containing the product were collected and concentrated under reduced pressure to give the azide linker (4) (1.2 mg, 0.0026 mmol, yield 1.8%) .

MS(ESI) m/z: 455[M+H]

(2) Introduction of an Azide Group by Azide Linker (4)

The azide linker (4) was reacted with the thiol group-introduced antibody in the same manner as described in Example 2 (2).

To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody had heavy chain peaks at 50683 and 50845 and a light chain peak at 23439, and the products had light chain peaks at 51057 and 51219 in which an azide group(s) was introduced to a heavy chain(s) and a light chain peak at 23439 which is the same as the source.

Example 5: Introduction of Azide Group by Azide Linker (5)

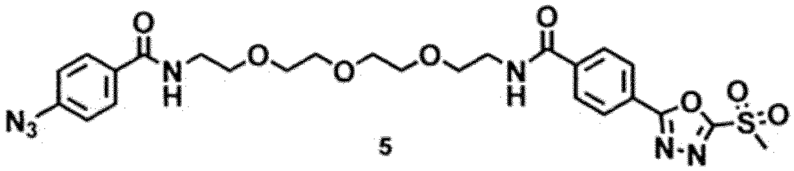

5

(1) Synthesis of Azide Linker (5)

(1-1) Synthesis of 4-(5-sulfanyl-1,3,4-oxadiazol-2-yl)benzoic acid

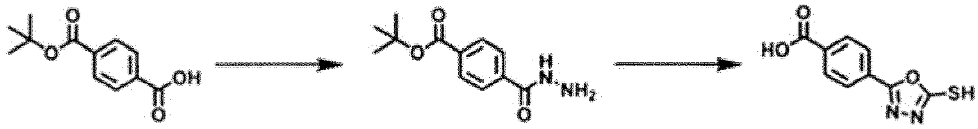

To 4-tert-butoxycarbonylbenzoic acid (307.0 mg, 1.38 mmol) in dichloromethane solutions (10 mL) were added hydrazine monohydrate (0.0558 mL, 1.79 mmmol), EDCI (411.1 mg, 2.14 mmol), DMAP (18.1 mg, 0.14 mmol) and triethylamine (0.462 mL, 3.34 mmol). After stirring at room temperature for 16 hours, the mixture was concentrated under reduced pressure, and the crude product was purified by column chromatography to obtain a mixture of tert-butyl 4-(hydrazinecarbonyl)benzoate.

Carbon disulfide (0.0471 mL, 0.78 mmol) and potassium hydroxide (103.7 mg, 1.80 mmol) were added to an ethanol solution (10 mL) of the obtained mixture, and the mixture was stirred at 70°C for 16 hours, and then an aqueous solution of 6 M hydrochloric acid was added to adjust pH in the system to 3.0. After the adjustment, the solution was subjected to separation extraction with ethyl acetate, and the obtained crude product was purified by column chromatography by concentrating the organic phase under reduced pressure. The fraction containing the product was collected and concentrated under reduced pressure to give 4-(5-sulfanyl-1,3,4-oxadiazol-2-yl)benzoic acid (72.9 mg, 0.33 mmol, 2 step yield 24%).

MS(ESI) m/z: 223[M+H]

(1-2) Synthesis of 4-(5-methylsulfanyl-1,3,4-oxadiazol-2-yl) benzoic acid

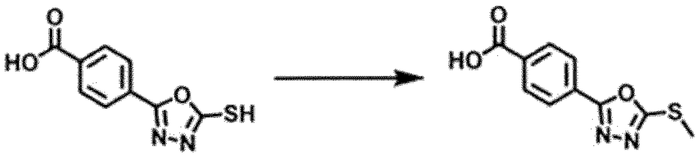

4-(5-sulfanyl-1,3,4-oxadiazol-2-yl)benzoic acid (72.9 mg, 0.33 mmol) in THF solution (3.3 mL) was cooled to

0°C, iodomethane (0.616 mL, 0.99 mmol) and triethylamine (0.228 mL, 1.60 mmol) were added, and the mixture was stirred at room temperature for 3 hours. The crude product obtained by concentrating the reaction solution under reduced pressure was purified by column chromatography. The fraction containing the product was collected and concentrated under reduced pressure to give 4-(5-methylsulfanyl-1,3,4-oxadiazol-2-yl)benzoic acid (66.2 mg, 0.28 mmol, yield 88%) .

MS(ESI) m/z: 237[M+H]

(1-3) Synthesis of N-[2-[2-[2-[2-[(4-azidobenzoyl) amino]ethoxy]ethoxy] ethoxy]ethyl]-4-(5-methylsulfanyl-1,3,4-oxadiazol-2-yl)benzamide

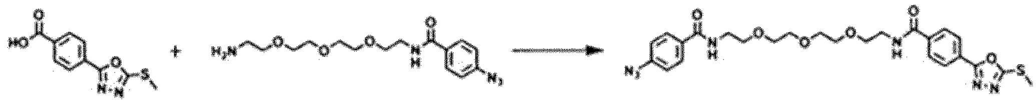

To 4-(5-methylsulfanyl-1,3,4-oxadiazol-2-yl)benzoic acid (66.2 mg, 0.28 mmol) in dichloromethane solution (2.8 mL) were added N-[2-[2-[2-(2-aminoethoxy)ethoxy]ethoxy]ethyl]-4-azido-benzamide (0.190 mg, 0.42 mmol), EDCI (88.5 mg, 0.46 mmol), DMAP (7.2 mg, 0.059 mmol) and triethylamine (0.116 mL, 0.84 mmol). After stirring at room temperature for 5 hours, the crude product obtained by concentrating the reaction solution under reduced pressure was purified by column chromatography. The fraction containing the product was collected and concentrated under reduced pressure to give 2-[2-[2-[2-[(4-azidobenzoyl)amino]ethoxy]ethoxy]ethoxy]ethyl)]-4-(5-methylsulfanyl-1,3,4-oxadiazol-2-yl)benzamide (104 mg, 0.19 mmol, yield 67 %) .

MS(ESI) m/z: 556[M+H]

(1-4) Synthesis of Azide Linker (5)

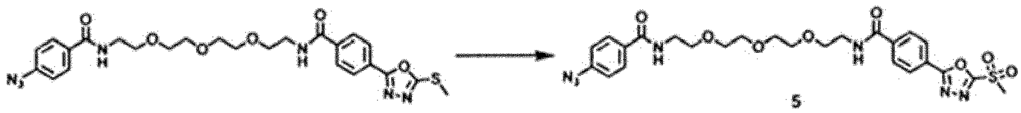

5

N-[2-[2-[2-[2-[(4-azidobenzoyl)amino]ethoxy]ethoxy]ethoxy]ethyl]-4-(5-methylsulfanyl-1,3,4-oxadiazol-2-yl)benzamide (104 mg, 0.19 mmol) in dichloromethane solution (1.9 mL) was cooled to 0°C and mCPBA (195.4 mg, 0.79 mmol) was added. After stirring at room temperature for 20 hours, a saturated aqueous sodium bicarbonate solution was added, and the mixture was subjected to separation extraction with ethyl acetate. The crude product obtained by concentrating the organic layer under reduced pressure was purified by column chromatography. The fraction containing the product was collected and concentrated under reduced pressure to give the azide linker (5) (84.8 mg, 0.14 mmol, yield 76%).

MS(ESI) m/z: 588[M+H]

(2) Introduction of Azide Group by Azide Linker (5)

The azide linker (5) was reacted with the thiol group-introduced antibody in the same manner as described in Example 2 (2).

To the product was added tris(2-carboxyethyl)phosphine hydrochloride (0.5 mM) and the sollution was stirred at room temperature for 10 minutes and the product was measured by ESI-TOFMS according to the previous report (WO2019/240287A1). The source thiol group-introduced antibody had heavy chain peaks at 50683 and 50845 and a light chain peak at 23439, and the products had light chain peaks at 51189 and 51350 in which an azide group(s) was introduced into a heavy chain(s), and a light chain peak at 23439 which is the same as the source.

Example 6: Introduction of azide groups by azide linker (6)

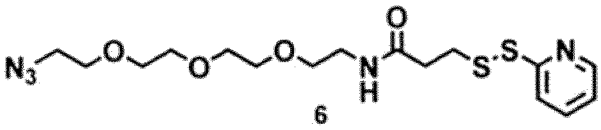

6

(1) Synthesis of Azide Linker (6)

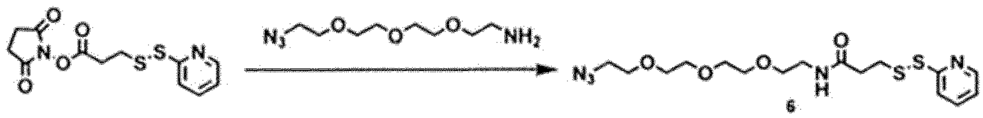

6

N-Succinimidyl 3-(2-Pyridyldithio)propionate (55.2 mg, 0.177 mmol) was dissolved in $CH_2Cl_2$ (2.0 mL) and 11-azido-3,6,9-trioxaundecan-1-amine (35 μL, 0.177 mmol) was added and the mixture was stirred at room temperature for 18 h. After the reaction was confirmed by LC/MS, the reactant was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. The fraction containing the product was collected, and the acetonitrile was removed by concentration under reduced pressure, followed by lyophilization to obtain an azide linker (6) (72.2 mg, 0.173 mmol).

$^1H$ NMR (400 MHz, Chloroform-d) δ=8.55 (s, 1H), 7.78 (s, 1H), 7.22 (s, 1H), 6.73 (s, 1H), 3.76-3.63 (m, 10H), 3.60 (dd, J=5.6, 4.5, 2H), 3.49 (q, J=5.2, 2H), 3.41 (t, J=5.0, 2H), 3.13 (tt, J=7.0, 2.4, 2H), 2.66 (td, J=6.9, 2.2, 2H). MS(ESI) m/z: z=1 416$[M+H]^+$

(2) Introduction of Azide Group by Azide Linker (6)

The azide linker (6) reagent was reacted with the thiol group-introduced antibody in the same manner as described in Example 2 (2). The reaction solution was replaced with 20 mM ammonium acetate buffer, and the mass was measured by ESI-TOFMS according to the method described in the prevous report (Anal. Chem. 2019, 91, 20, 12724-12732), and 149009 peaks in which two azide groups were introduced into the antibody were confirmed.

Example 7: Introduction of azide groups by azide linker (7)

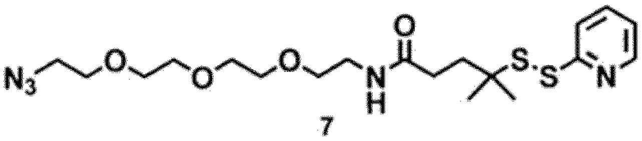

7

(1) Synthesis of Azide Linker (7)

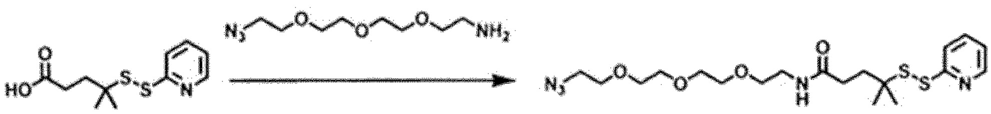

4-Methyl-4-(pyridin-2-yldisulfanyl)pentanoic acid (100.0 mg, 0.39 mmol) was dissolved in THF (3.0 mL) and 11-azido-3,6,9-trioxaundecan-1-amine (77 μL, 0.39 mmol), DCC (97.0 mg, 0.47 mmol), NHS (54.0 g, 0.47 mmol) were added and the mixture was stirred at room temperature for 18 h. After the reaction was confirmed by LC/MS, the reactant was dissolved in a 0.05% aqueous trifluoroacetic acid solution and subjected to reverse-phase high-performance liquid chromatography using octadodecyl group-chemically bonded silica gel as a filler, and the reactant was eluted with a

mixed solution of water and acetonitrile containing 0.05% trifluoroacetic acid, and the respective fractions were confirmed by LC-MS. The fraction containing the product was collected, and the acetonitrile was removed by concentration under reduced pressure, followed by lyophilization to obtain an azide linker (7) (39.9 mg, 0.087 mmol).

$^1$H NMR (400 MHz, Chloroform-d) $\delta$=8.52(d, J=5.0, 1H), 7.81 (d, J=8.2, 1H), 7.72 (t, J=7.9, 1H), 7.17 (t, J=6.2, 1H), 6.15 (s, 1H), 3.76-3.62 (m, 10H), 3.57 (dd, J=5.6, 4.6, 2H), 3.44 (dt, J=15.2, 5.1, 4H), 2.42-2.27 (m, 2H), 2.06-1.94 (m, 2H), 1.33(s,6H).
MS(ESI) m/z: z=1 458[M+H]$^+$

(2) Introduction of Azide Group by Azide Linker (7)

The azide linker (7) reagent can be reacted with the thiol group-introduced antibody in the same manner as described in Example 2 (2).

Example 8: Introduction of DBCO group by Alkyne linker (8)

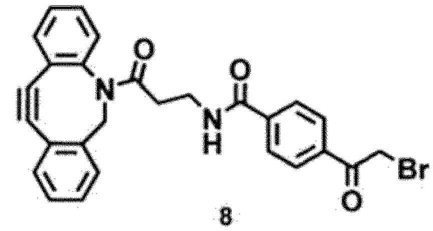

8

(1) Synthesis of Alkyne Linker (8)

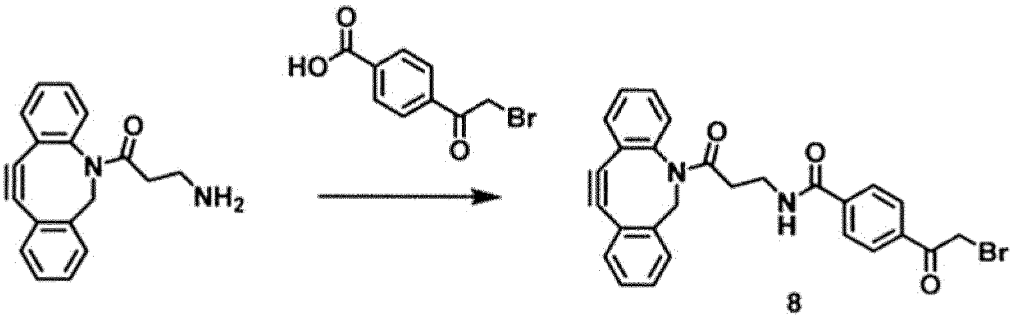

8

Diisopropylcarbodiimide (10.9 mg, 0.087 mmol) and bromoacetylbenzoic acid (21 mg, 0.087 mmol) were added to dichloromethane solutions (1 mL) of 3-Amino-1-(11,12-didehydrodibenz[b,f]azocin-5(6H)-yl)-1-propanone (20 mg, 0.072 mmol) at room temperature. After stirring at room temperature for 16 hours, concentration was performed under reduced pressure, and the obtained crude product was purified by column chromatography. The fraction containing the product was collected and concentrated under reduced pressure to give the alkyne linker (8) (12.3 mg, 0.25 mmol, yield 34%).

MS(ESI) m/z: 501[M+H]

(2) Introduction of DBCO group by Alkyne linker (8)

The alkyne linker (7) was reacted with the thiol group-introduced antibody in the same manner as described in Example 2 (2). To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody showed heavy chain peaks at 50683 and 50845 and a light chain peak at 23439, and the products showed light chain peaks at 51109 and 51270 in which an azide group(s) is introduced into a heavy chain(s), and a light chain peak at 23439 which was the same as the source.

Example 9: Introduction of DBCO group by Alkyne linker 9

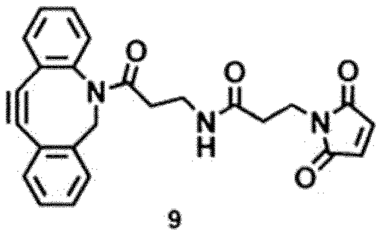

9

In the same manner as described in Example 2(2), 10 equivalents of the above alkyne linker (9, manufactured by Tokyo Chemical Industry Co., Ltd.) were reacted. To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody had heavy chain peaks at 50683 and 50845 and a light chain peak at 23439, and the products had light chain peaks at 51123 and 51291 in which an azide group(s) was introduced into a heavy chain(s) and a light chain peak at 23439 which was the same as the source.

Example 10: Introduction of DBCO group by Alkyne linker (10)

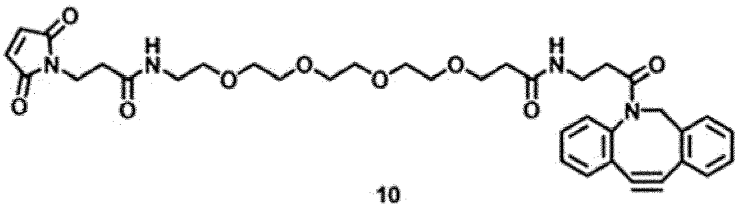

10

In the same manner as described in Example 2(2), 10 equivalents of the above alkyne linker (10) were reacted. To the product was added tris(2-carboxyethyl)phosphine hydrochloride (0.5 mM) and the mixture was stirred at room temperature for 10 minutes and the product was measured by ESI-TOFMS according to the previous report (WO2019/240287A1). The source thiol group-introduced antibody had heavy chain peaks at 50683 and 50845 and a light chain peak at 23439, and the products had light chain peaks at 51371 and 51532 in which an azide group(s) was introduced into a heavy chain(s) and a light chain peak at 23439 which is the same as the source.

Example 11: Introduction of DBCO group by Alkyne linker (11)

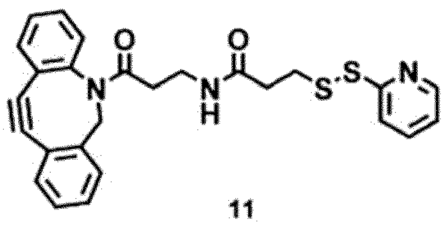

11

(1) Synthesis of Alkyne Linker (11)

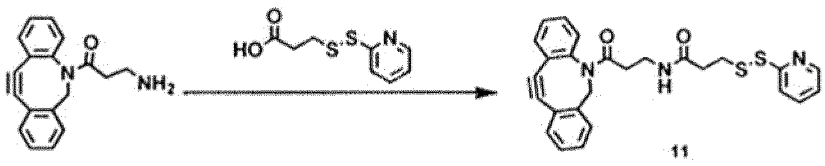


To 3-Amino-1-(11,12-didehydrodibenz[b,f]azocin-5(6H)-yl)-1-propanone (14 mg, 0.051 mmol) in dichloromethane solution (1 mL) was added WSC (15 mg, 0.079 mmol), HOBT (15 mg, 0.111 mmol), triethylamine (12 μL, 0.162 mmol), DMAP (0.5 mg, 0.004 mmol) and 3-pyridyldithiopropionate (11 mg, 0.050 mmol) at room temperature. After stirring at room temperature for 3 hours, concentration was performed under reduced pressure, and the obtained crude product was purified by column chromatography. The fraction containing the product was collected and concentrated under reduced pressure to give the alkyne linker (10) (4.2 mg, 0.0096 mmol, yield 19%).

MS(ESI) m/z: 501[M+H]

(2) Introduction of DBCO group by Alkyne linker (11)

In the same manner as described in Example 2 (2), the above alkyne linker (11) was reacted with the thiol group-introduced antibody. The reaction solution was replaced with 20 mM ammonium acetate buffer, and the mass was measured by ESI-TOFMS according to the method described in the previous report (Anal. Chem. 2019, 91, 20, 12724-12732). As a result, a peak at 149151 in which two azide groups were introduced into the antibody were confirmed.

Example 12: Determination of aggregation rate of bioorthogonal functional group-introduced antibody by SEC HPLC

Each of aggregation rate of the bioorthogonal functional group-ntroduced antibodies synthesized in Comparative Examples 1 and 2 and Examples 1 to 6 and 8 to 11 were measured according to the previous report (ACS Omega 2020, 5, 7193-7200) under the following conditions.

Measuring system: 1260 HPLC system (manufactured by Agilent)
Column: AdvanceBio SEC 300 Å 2.7 um, 4.6 mm × 150 mm manufactured by Agilent
Flow rate: 0.25 mL/min
Eluent: 100 mM sodium dihydrogen phosphate/sodium hydrogen phosphate, 250 mM sodium chloride in water (pH 6.8), 10% v/v Isopropanol
Detector: UV(280 nm)

Table 1. Measurement of aggregation rate by HPLC

| Modified antibody | Aggregation rate (%) |
|---|---|
| Comparative Example 1 | 5.1 |
| Comparative Example 2 | 12.4 |
| Example 1 | 1.9 |
| Example 2 | 1.4 |
| Example 3 | 2.8 |
| Example 4 | 1.5 |
| Example 5 | 1.6 |
| Example 6 | 3.3 |
| Example 8 | 1.5 |
| Example 9 | 2.7 |
| Example 10 | 2.7 |
| Example 11 | 2.7 |

As a result, the aggregation rate of the bioorthogonal functional group-introduced antibodies obtained using the compounds of Examples was less than 3.5%, and was lower than the aggregation rate of the bioorthogonal functional group-introduced antibodies obtained using the compounds of Comparative Examples (Table 1).

Example 13: Determination of the percentage of unreacted heavy chains by reverse phase HPLC under reduction conditions

(1) Preparation of measurement samples

To the product was added an aqueous solution of DL-dithiothreirol (an aqueous solution in which an aqueous solution of 1 M DL-dithiothreirol was added to the aqueous solution of 8 M guanidine hydrochloride) and the solution was heated at 80°C for 10 minutes.

(2) Analyses by reversed phase HPLC

The bioorthogonal functional group-introduced antibodies synthesized in Comparative Examples 1 and 2 and Examples 1 to 5 and 8, 9, and 10 were subjected to reverse phase HPLC analysis according to the previous report (Anal. Chem., 2019, 91, 20, 12724-12732). The measurement was performed using the following conditions.
Measuring system: 1260 HPLC system (manufactured by Agilent)
Column: AdvanceBio RP-mAb Diphenyl 3. 5 um, 2.1 × 100 mm manufactured by Agilent
Gradient: Linear gradient of eluent A/B
Flow rate: 0.4 mL/min
Eluent A: water, 0.1% v/v trifluoroacetic acid
Eluent B: acetonitrile, 0.1% v/v trifluoroacetic acid Detector:UV (280 nm)

The proportion of heavy chains in the reaction was calculated by (peak area of unreacted heavy chains)/(peak area of all heavy chains of the antibody).

Table 2. Measurement of rate of unreacted heavy chain

| Modified antibody | Rate of unreacted heavy chain (%) |
|---|---|
| Comparative Example 1 | 15.2 |
| Comparative Example 2 | 43.3 |
| Example 1 | 3.2 |
| Example 2 | 8.5 |
| Example 3 | 7.8 |
| Example 4 | 10.8 |
| Example 5 | 5.4 |
| Example 8 | 3.0 |
| Example 9 | 8.2 |
| Example 10 | 9.2 |

The results in Table 2 above revealed that the bioorthogonal functional group-introduced antibodies synthesized in Examples 1 to 5 and 8, 9, and 10 exceeds a conversion rate of more than 85% from the thiol-introduced antibody of the raw material.
The compound having a disulfide bond as in Examples 6 and 11 is converted into a thiol group-introduced antibody as a raw material by cleavage of the disulfide bond by DTT in the pretreatment step of the measurement by the reverse phase HPLC, and therefore, it is not possible to determine the unreacted heavy chain rate (%) by this method. Thus, the determination of the unreacted heavy chain rate (%) for the compounds of Examples 6 and 11 was performed in the following Examples.

Example 14: Determination of Rate of Unreacted Thiol-introduced Antibodies by ESI-TOFMS

(1) Cleavage of sugar chain in bioorthogonal functional group-introduced antibodies

The bioorthogonal functional group-introduced antibodies synthesized in Examples 6 and 11 were subjected to cleavage of sugar chains and post-treatment using PNGase F (New England BioLabs, catalogue number P0704) according to the procedures of the manufacturer's protocol and the previously report (WO2019240288A1). HIC-HPLC analysis was performed.

(2) ESI-TOFMS analysis of cleaved form of sugar chain and measurement of rate of unreacted antibody by DAR calculator

The masses of the cleaved form of sugar chain obtained in (1) were measured by ESI-TOFMS accorduing to the previous report (Anal. Chem., 2019, 91, 20, 12724-12732). For MS data obtained, the rate of unreacted antibodies calculated from DAR peak and % Area by DAR calculator (Agilent Software) is shown on Table 3.
The rate of unreacted antibody was calculated from the following formula.

$$\text{Rate of unreacted antibody} = (A+B/2) / (A+B+C)$$

A: Peak area of the mass number corresponding to the thiol-introduced antibody (compound 28) of the raw material
B: Peak area corresponding to the mass number in which one bioorthogonal functional group was introduced into the thiol-introduced antibody (compound 28) as a raw material C: Peak area corresponding to the mass number in which two bioorthogonal functional groups were introduced into the thiol-introduced antibody (compound 28) as a raw material

Table 3. Measurement of rate of unreacted heavy chain

| Modified antibody | Rate of unreacted heavy chain (%) |
|---|---|
| Example 6 | 4.3 |
| Example 11 | 4.4 |

From the results in Table 3 above, it was revealed that in the synthesis of the antibody derivative using the compounds of Examples 6 and 11, the antibody derivative into which the bioorthogonal functional group was introduced had a conversion rate of more than 90% from the thiol-introduced antibody as a raw material.
Example 15: Synthesis of ADC and ADC mimic by Click Reaction with Bioorthogonal Functional Group-Introduced Antibody

(1) Synthesis of ADC by click reaction

According to previous reports (WO2019/240287A1 and WO2019/240288A1), 7 equivalents of DBCO-VC-PAB-MMAE (ABZENA) in DMF solution (5 mM) were added to azide-introduced trastuzumab obtained in Examples 1 to 6, and the mixture was stirred at room temperature for 20 hours, and then the product was purified using NAP-5 Columns (GE Healthcare) to obtain a ADC.

(2) Synthesis of ADC mimic by click reaction

According to previous reports (WO2019/240287A1 and WO2019/240288A1), ADC mimics were obtained by adding Carboxyrhodamine 110-PEG4-DBCO (Broadpharm) to azide-introduced trastuzumab obtained in Examples 1 to 6.
Similarly, ADC mimics were obtained by adding Carboxyrhodamine 110-PEG3-azide (Broadpharm) to DBCO introduced trastuzumab obtained in Examples 8 to 11.

(3) Analysis of ADC and ADC mimic

ESI-TOFMS analysis of ADC synthesized in Example 13(1) and ADC mimic synthesized in Example 13(2) was performed according to the previous report (WO2019/240287A1), and it was confirmed that DAR was 2.

EXAMPLE 16: Evaluating ADC mimic by Stability Test with Rat Plasma

The stability in blood of various ADC mimics synthesized in Example 15(2) was evaluated. Specifically, the stability of ADC mimic in blood was assessed by analyzing the amounts of fluorescent molecules that were shed from ADC mimic when ADC mimic was incubated in rat blood, as described below.

(1) Synthesis of Control ADC mimic

The azide-antibody described in the previous report (WO2019/240287A1) as shown below was reacted with Carboxyrhodamine110-PEG4-DBCO (Broadpharm) according to Example 15(2) to give an ADC mimic.

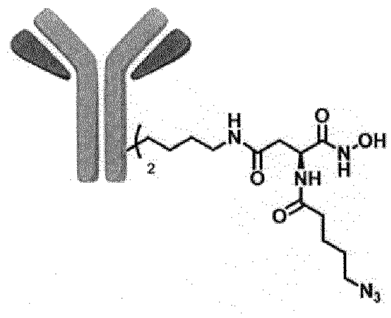

(2) Test of stability in plasma

Sterile filtration was performed on 700 μL of plasma from Charles River after ADC mimic was added to 0.1 mg/mL. The solution was dispensed into six Eppen tubes in 50 μL portions. Three of the six samples were stored in an incubator set at 37°C for 4 days. The remaining three were also stored in -80°C freezer for 4 days. A precipitate was obtained by adding 100 uL of acetonitrile to the samples, vortexing them, and centrifuging them. The resulting supernatant was collected and analyzed by HPLC.

(3) Analysis of Amount of Eliminated Fluorescent Molecules Using HPLC analysis

The determination was made using a liquid chromatography/fluorescence detection method to determine the molecular weight of fluorescence molecule that had eliminated from ADC mimic. Three samples stored in the freezer (-80°C) in Example 16 (1) (96 hours) was Day=0, the three samples stored at 37°C in Example 14 (1) (96 hours) was Day=4, the difference in the fluorescent strength of Day=4 and Day=0 was analyzed.

The results were evaluated as follows. By comparing the fluorescent intensities of the example compounds using ADC mimic synthesized in Example 16(1) as a control, the relative reduction ratio was calculated using the following Equation (A).

Increase in fluorescence intensity of fluorescent molecules eliminated from ADC mimic = [(Day=4 fluorescence intensity)-(Day=0 fluorescence intensity)].

Increase in fluorescence intensity of fluorescent molecules eliminated from the control ADC mimic = [(Day=4 fluorescence intensity)-(Day=0 fluorescence intensity)].

Ratio of increase amount = increase amount of fluorescence intensity of Example ADC mimic/increase amount of fluorescence intensity of fluorescence intensity of control ADC mimic

Table 4. Ratio of relative lowering rate of ADC mimic compared with control

| Modified antibody | Ratio of increased amount |
|---|---|
| Example 1 | <10% |
| Example 2 | <10% |
| Example 3 | <10% |
| Example 4 | <10% |
| Example 5 | <10% |

(continued)

| Modified antibody | Ratio of increased amount |
|---|---|
| Example 6 | <10% |
| Example 8 | <10% |
| Example 9 | <10% |
| Example 10 | <10% |
| Example 11 | <10% |

As a result, ADC mimics synthesized in Examples 15(1) and (2) were all 10 times more stable than the control synthesized in Example 16(1).

Example 17: Synthesis of an antibody-protein complex by a click reaction with a bioorthogonal functional group-introduced antibody

(1) Introduction of linkers into proteins

To lysozyme (pH 7.4, PBS buffer solution), a solution of 6 equivalents of N-Hydroxysuccinimidyl-4-azidobenzoate in DMF was added, and the mixture was stirred at room temperature for 3 hours, and then the product was purified using NAP-5 Columns (GE Healthcare) to obtain azide-introduced lysozyme. Mass was measured by ESI-TOFMS according to the method described in the previous report (Anal. Chem., 2019, 91, 20, 12724-12732), and a peak at 14450 in which the azide group was introduced into lysozyme was confirmed.

In addition, an azide group was introduced into bovine serum albumin by the same method. Mass was determined by ESI-TOFMS and a peak 66579 in which the azide group was introduced into bovine serum albumin was confirmed.

(2) Synthesis of antibody-protein complexes by click reaction

The azide-introduced lysozyme synthesized in Example 17(1) was added to the alkyne-introduced trastuzumab obtained in Example 8 according to the previous reports (WO2019/240287A1 and WO2019/240288A1) to obtain a trastuzumab-lysozyme complex. The masses were measured by ESI-TOFMS according to the previous report (Anal. Chem., 2019, 91, 20, 12724-12732), and a peak at 178610 in which two lysozymes were introduced into trastuzumab was confirmed.

In addition, bovine serum albumin synthesized in Example 17(1) was added to the alkyne-introduced trastuzumab obtained in Example 8 in the same manner to obtain a trastuzumab-lysozyme complex. Mass was determined by ESI-TOFMS according to the method described in the previous report (Anal. Chem., 2019, 91, 20, 12724-12732), and a peak at 283466 in which two bovine serum albumins were introduced into trastuzumab was confirmed.

The azide-introduced lysozyme synthesized in Example 17(1) was added to the alkyne-introduced trastuzumab obtained in Example 9 in the same manner to obtain a trastuzumab-lysozyme complex. The masses were measured by ESI-TOFMS according to the previous report (Anal. Chem., 2019, 91, 20, 12724-12732), and a peak at 178669 in which two lysozymes were introduced into trastuzumab was confirmed.

The azide-introduced lysozyme synthesized in Example 17(1) was added to the alkyne-introduced trastuzumab obtained in Example 10 in the same manner to obtain a trastuzumab-lysozyme complex. The masses were measured by ESI-TOFMS according to the previous report (Anal. Chem., 2019, 91, 20, 12724-12732), and a peak at 179154 in which two lysozymes were introduced into trastuzumab was confirmed.

The azide-introduced lysozyme synthesized in Example 17(1) was added to the alkyne-introduced trastuzumab obtained in Example 11 in the same manner to obtain a trastuzumab-lysozyme complex. The masses were measured by ESI-TOFMS according to the previous report (Anal. Chem., 2019, 91, 20, 12724-12732), and a peak at 178647 in which two lysozymes were introduced into trastuzumab was confirmed.

Example 18: Introduction of Bioorthogonal Functional Group to Position Lys288/290

(18-1) Synthesis of compound having affinity substance to soluble protein, a cleavable portion and reactive group (linked product of peptide thioester linker-thiophenol activator), and modification of the anti-HER2 antibody trastuzumab using compound and analysis of antibody

(18-1-1) Synthesis of IgG1 Fc Binding Peptide

Ac-FNMQCQRRFYEALHDPNLNEEQRNARIRSIKDDC-$NH_2$ (SEQ ID NO: 4) which is an affinity substance to a soluble protein was synthesized by a Fmoc solid-state synthetic method. A Liberty Blue manufactured by CEM was used as a peptide synthesizer. All reagents from Watanabe Chemical Industries were used. Resin were double-coupled with Fmoc-NH-SAL-PEG Resin, HL, arginine (R), cysteine (C), and histidine (H). The excision from Resin was carried out under a condition stirring for 3 hours in trifluoroacetic acid:water:triisopropylsilane:ethanedithiol=94:2.5:1.0:2.5. After excision, Resin was removed by filtration and trifluoroacetic acid was removed. Diethyl ether was added to the resulting crystals, followed by ether precipitation, and the resulting white crystals were collected by filtration. This was dissolved in 0.1% trifluoroacetic acid aqueous solution and subjected to reverse phase high performance liquid chromatography using octadodecyl group chemically bonded silica gel as a filler, and eluted with a mixed solution of 0.1% trifluoroacetic acid in water and acetonitrile, and the fractions were confirmed by LC-MS. Fractions containing the product were collected, and only acetonitrile was removed by concentration under reduced pressure, followed by lyophilization.

(18-1-2) Forming an intramolecular disulfide bond at Cysteins at positions 5 and 34 of Ac-FNMQCQRRFYEALHDPNLNEEQRNARIRSIKDDC-NH2 (SEQ ID NO: 4)

The peptide synthesized in (18-1-1) was dissolved in DMSO and 0.1 M Tris-HCl pH 8.0 was added. To this solution was added the glutathione oxidized form and the mixture was stirred at room temperature for 20 hours. The reaction was stopped by adding an aqueous 2 M trifluoroacetic acid solution to the reaction solution, which was dissolved in a 0.05% aqueous trifluoroacetic acid solution, and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the target substance (20.0 mg, 4.70 μmol).

MS(ESI) m/z: z=4 1063.65$[M+4H]^{4+}$, z=5 851.15$[M+5H]^{5+}$

(18-1-3) Synthesis of the thioester linker

(18-1-3-1)

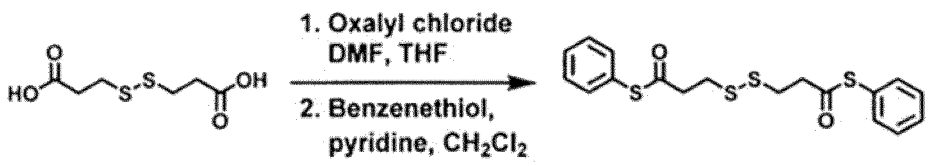


3,3'-Dithiodipropionic acid (1 g, 5.0 mmol) was solved in THF (10 mL) and DMF (100 μL) at 0°C and oxalyl chloride (1.5 mL, 15.0 mmol) was added to mix at 0 °C for 15 minutes and at room temperature for 1 hour. Then, Benzenethiol (1.53 mL, 15.0 mmol) at 0°C, Pyridine (4 mL, 50 mmol) and $CH_2Cl_2$ (10 mL) were added, and the mixture was stirred at room temperature for 3 hours. After confirming the reaction with TLC (hexane/ethyl acetate=5/1), the crystals were removed, followed by extraction with ethyl acetate and 1 M hydrochloric acid aqueous solution, and the organic layers was concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (1.2 g, 3.04 mmol) .

(18-1-3-2)

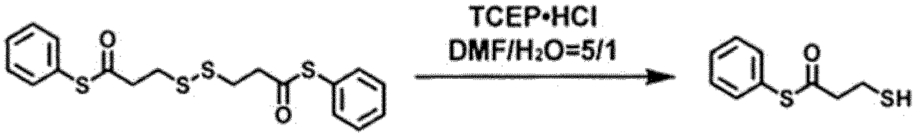


The compound (1.2 g, 3.04 mmol) synthesized in (18-1-3-1) was dissolved in a mixed solvent of DMF/$H_2O$=5/1, TCEP•HCl (1.74 g, 6.08 mmol) was added, and the mixture was stirred at room temperature for 1 hour. After confirming the reaction with TLC (hexane/ethyl acetate=5/1), the reaction was extracted with ethyl acetate and water, and then the organic layer was concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (1.18 g, 6.5 mmol).

(18-1-3-3)

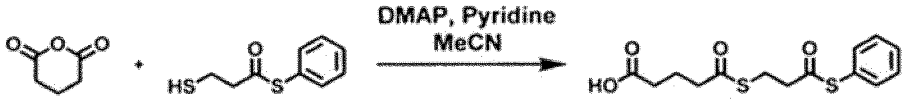


The compound (200 mg, 1.10 mmol) synthesized in (18-1-3-2) was dissolved in acetonitrile (2.0 mL), Glutaric Anhydride (125.5 mg, 1.10 mmol), DMAP (6.72 mg, 0.06 mmol), Pyridine (0.22 mL) was added, and the mixture was stirred at room temperature for 4 hours. After confirming the reaction with TLC (dichloromethane/methanol=10/1), the reaction was extracted with ethylacetate and 0.5 M HCl, and the organic layers was concentrated. It was eluted with a mixed solution of dichloromethane and methanol, and the fractions were confirmed by TLC (dichloromethane/methanol=10/1). Fractions containing the product were collected and concentrated under reduced pressure to remove the organic solvents, followed by vacuum-drying to obtain the above compound (135 mg, 0.45 mmol) .

(18-1-3-4)

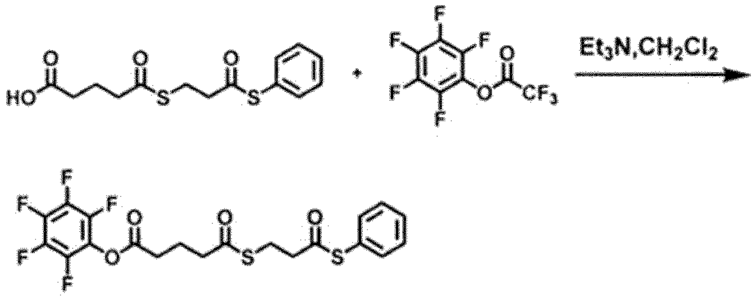


The compound (134 mg, 0.45 mmol) synthesized with (18-1-3-3) was dissolved by adding $CH_2Cl_2$ (2.25 mL) and triethylamine (157 μL, 1.13 mmol). Pentafluorophenyl trifluoroacetic acid (154 μL, 0.90 mmol) was added at 0°C and the mixture was stirred for 1 h. After confirming the reaction with TLC (hexane/ethyl acetate=3/1), the reaction solutions were concentrated. It was eluted with a mixed solution of hexane and ethyl acetate and the fractions were confirmed by TLC (hexane/ethyl acetate=3/1). Fractions containing the product were collected and concentrated under reduced pressure to remove the organic solvents, followed by vacuum-drying to obtain the above compound (96 mg, 0.20 mmol).

$^1$H NMR (400 MHz, Chloroform-d) δ=7.44 (s, 5H), 3.23 (t, J=6.9, 2H), 3.01 (t, J=6.9, 2H), 2.77 (dt, J=14.5, 7.3,

4H), 2.16 (t, J=7.3, 2H).

(18-1-4) Linkage between the peptide and the linker

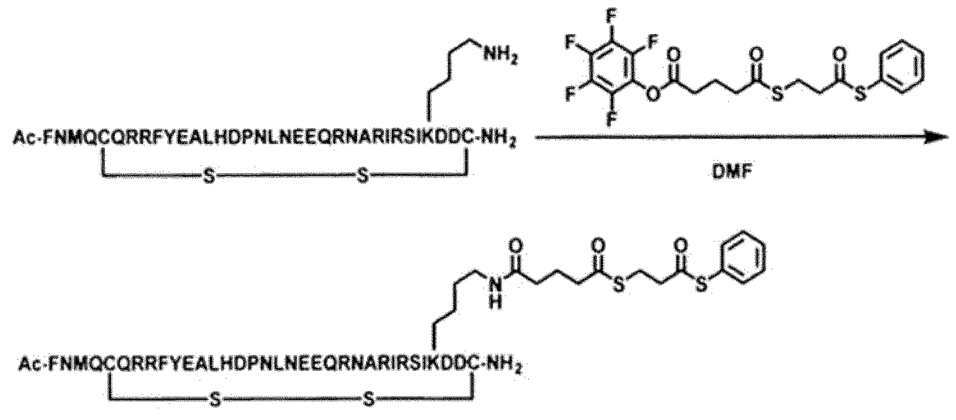


Both of the two aforementioned acid sequences are the amino acid sequence of SEQ ID NO: 4.

Ac-FNMQCQRRFYEALHDPNLNEEQRNARIRSIKDDC-$NH_2$ (SEQ ID NO: 4) (30.0 mg, 7.06 μmol, where two cysteines at positions 5 and 34 each forms a disulfide bond in the molecule) synthesized in (18-1-2) was dissolved in N,N-dimethylformamide (1.00 mL) and a thioester linker (96.0 mg, 201 μmol) synthesized in (1-3) was added thereto, followed by stirring at room temperature for 24 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the linked product of peptide thioester-thiophenol activator (15.8 mg, 3.48μ mol).

MS(ESI) m/z: z=4 1136.80$[M+4H]^{4+}$

(18-1-5) Specific Modification of the Anti HER2 IgG Antibody Trastuzumab and Analysis by ESI-TOFMS

The linked product of peptide linker synthesized in (18-1-4) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of HEPES buffer (pH 8.2), and 3.38 μL of 10 mM peptide reagent (10 equivalents to the antibody) was added and the mixture was stirred at room temperature for 1 hour. The reactant was replaced with 20 mM ammonium acetate buffer and the mass was measured by ESI-TOFMS. As a result, trastuzumab as a raw material peaked at 148223. The product was identified as 152660 with one binding peptide introduced, 157093 with two binding peptides introduced, and 161528 peaks with three introduced.

(18-1-6) Confirmation of Heavy Chain Selectivity by ESI-TOFMS Analysis of Specific Modified Trastuzumab under Reducing Conditions

To the antibody-peptide complex produced in (18-1-5), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab had a heavy chain peak at 50596 and a light chain peak at 23439, and the product was 55033 in which one linker was introduced into the heavy chain and the light chain had a peak at 23439 which was the same as the raw material.

(18-1-7) Confirmation of Peptide/Antibody Binding by DAR calculator of Specific Modifications of Trastuzumab

For MS data analyzed in (18-1-5), the peptide/antibody bonding ratio was confirmed by DAR calculator (Agilent Software). The results are shown in Table 5. The average peptide/antibody bonding ratio calculated from DAR peak and % Area in Table-5 was 2.0. Therefore, the production of an antibody intermediate (average peptide/antibody bonding ratio 2.0) represented by the following structural formula was confirmed.

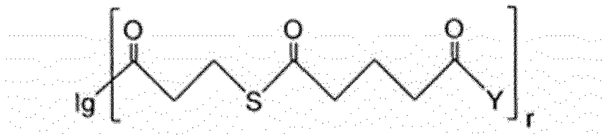

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 4,
The average bond ratio r between Ig and the two carbonyl groups (C=O) adjacent to Ig is 2.0.]

Table 5

| DAR Peak | Observed mass(Da) | Peak Area | %Area |
|---|---|---|---|
| 1 | 152660 | 6. 23E+003 | 4.56 |
| 2 | 157093 | 1. 22E+005 | 89. 26 |
| 3 | 161528 | 8. 44E+003 | 6. 18 |

(18-1-8) Preparation of Thiol Group-Introduced Antibody Derivatives by Cleavage of Thioester Group

To the antibody-intermediates obtained in (18-1-7), hydroxylamine solutions were added according to WO2019240287A1 and allowed to stand at room temperature for 1 hour. After 2 hours, the compound was replaced with 20 mM PBS buffer, 10 mM EDTA (pH 7.4) to obtain a thiol-group-introduced antibody derivative. The mass was measured by ESI-TOFMS. As a result, the peak was confirmed at 148409 at which the cleavage proceeded.

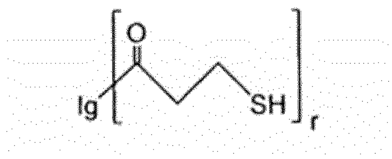

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;
The average bond ratio r between Ig and the two carbonyl groups (C=O) adjacent to Ig is 2.0.]

(18-1-9) Peptide mapping by trypsinization

The thiol-introduced trastuzumab obtained in (1-8) was subjected to peptide mapping in the following steps.

(18-1-9-1) Trypsinization of the thiol-introduced trastuzumab

To 1.5 mL low-adsorption microtest tube, 10 μL of the sample solution, 50 mM ammonium hydrogencarbonate buffer solution, and 10 μL of a dithiothreitol aqueous solution of 20 mM dissolved in 40% trifluoroethanol were added, and after warming at 65°C for 1 hour, 10 μL of 50 mM iodoacetamide aqueous solution was added, and the mixture was allowed to react for 30 minutes at room temperature under light shielding. After the reaction, 40 μL of 50 mM ammonium bicarbonate buffer was added and the mixture was stirred, and 10 μL of 20 ng/μL of trypsin aqueous solution was added and enzymatically digested at 37°C for 16 hours. After digestion, 2 μL of 20% aqueous trifluoroacetic acid solution was added, the reaction was stopped, and LC-MS/MS was measured.

(18-1-9-2) LC-MS/MS determination of trastuzumab

(Analyzer)

Nano HPLC:EASY-nLC 1000 (Thermo Fisher Scientific)
Mass Spectrometer: Tribrid Mass Spectrometer Orbitrap Fusion (Thermo Fisher Scientific)

(HPLC Analytical Conditions)

Trap Column: Acclaim PepMap ® 100, 75 μm × 2 cm

(Thermo Fisher Scientific)

Analytical columns: ESI-column (NTCC-360/75-3-125, 75 μm × 12.5 cm, 3 μm (Nikkyo Technos Co., Ltd.)

Mobile phase A: 0.1% formic acid in water
Mobile phase B: 0.1% formic acid, acetonitrile solution
Loading solution: 0.1% aqueous trifluoroacetic acid solution
Flow rate: 300nL/min
Sample injection volume: 1 μL
Gradient conditions (B%): 2% (0.0-0.5 min), 2% → 30%

(0.5-23.5 min), 30% → 75% (23.5-25.5 min), 75% (25.5-35.0 min)

(Mass Spectrometer Analysis Conditions)

Ionization method: ESI, Positive
Scan Type: Data Dependent Aquisition
Activation Type: Collision Induced Dissociation(CID)
Data were acquired using the attached software Xcalibur 3.0 (Thermo Fisher Scientific) and Thermo Orbitrap Fusion Tune Application 2.0 (Thermo Fisher Scientific).

(18-1-9-3) Analysis of trastuzumab modification sites

Modification site analysis for LC-MS/MS assay was performed using BioPharma Finder 3.0 (Thermo Fisher Scientific).

Analysis in BioPharma Finder was performed with S/N Threshold set to 1 and MS Noise Level set to 0.01% of the peak-top strength. The digestive enzyme was set to Trypsin and Specificity to High. For Static Modification, Carbamidomethyl (+57.021 Da) was set as a modification of cysteine residue by iodoacetamide. For Dynamic Modifications, oxidations of methionine residues (+15.995 Da) and modifications to lysine residues (thiol-introduced product which is carbamidomethylated with iodoacetamide (+ 145.019 Da)) were established. In addition, filters were set so that Confidence Score was 80 or more, Mass Accuracy at the time of identification was 5ppm or less, and only those for which MS/MS was observable. Regarding the residue number of the lysine residue, the heavy chain VH domain and the light chain are indicated by the number in the sequence (i.e., the first amino acid at the N-terminal end; the same shall apply hereinafter), and the heavy chain CH1, CH2, CH3 domain is indicated by EU numbering.

In addition, (1) and (2) shown in FIG. 6 were used as the data of the amino acid sequence to be searched for the modification site.

(18-1-9-4) Analysis of trastuzumab LC-MS/MS modification sites

Analysis using LC-MS/MS revealed that MS spectra (found: m/z 577.03606, theoretical value: 577.03557, tetravalent) of the peptide fragment FNWYVDGVEVHNAKTKPR (SEQ ID NO: 3) which is a peptide consisting of 18

amino acids including a modification site of trastuzumab to lysine residue by tryptic digestion of trastuzumab (thiol-introduced trastuzumab (+145.019 Da) which is carbamidomethylated by iodoacetamide) was observed (FIG. 7), and m/z 682.13 (theoretical value: 682.01) corresponding to a trivalent y16 showing modification of the lysine residue at position 288 or 290 of the heavy chain according to EU numbering was confirmed from CID spectrum (FIG. 8). Analysis with BioPharma Finder also showed that the modification to the lysine residue at position 288 or 290 occurred highly selectively (FIG. 9).

From these results, it was found that in the trastuzumab thiol transfectants obtained in (18-1-8) above, conjugation proceeded regioselectively on the heavy chain of the antibody at Lys288 and Lys290 according to EU numbering.

(18-2-1) Synthesis of the thioester linker

(18-2-1-1)

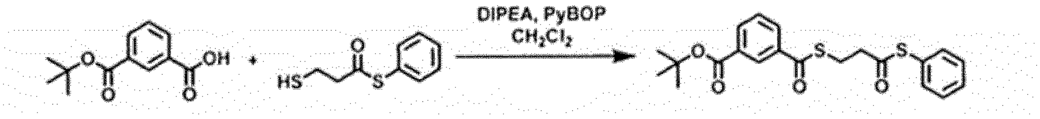


The compound (295 mg, 1.62 mmol) synthesized with (18-1-3-2) was dissolved in $CH_2Cl_2$ (13.5 mL), and 3-(tert-Butoxycarbonyl)benzoic acid (300 mg, 1.35 mmol), DIPEA (700μL, 2.03 mmol), and PyBOP (843 mg, 1.62 mmol were stirred at normal temperature for 1 h. After confirming the reaction with TLC (hexane/ethyl acetate=5/1), the reaction solutions were concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (259 mg, 0.64 mmol).

(18-2-1-2)

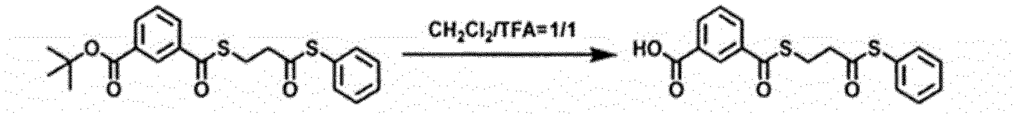


The compound (259 mg, 0.64 mmol) synthesized in (18-2-1-1) was dissolved in a mixed solution of $CH_2Cl_2$ and TFA=1/1, and the mixture was stirred at room temperature for 1 hour. After confirming that the reaction solution had fallen to the starting point with TLC (hexane/ethyl acetate=5/1), the reaction solution was concentrated and then dried under reduced pressure to obtain the above compound (227 mg, 0.66 mmol).

(18-2-1-3)

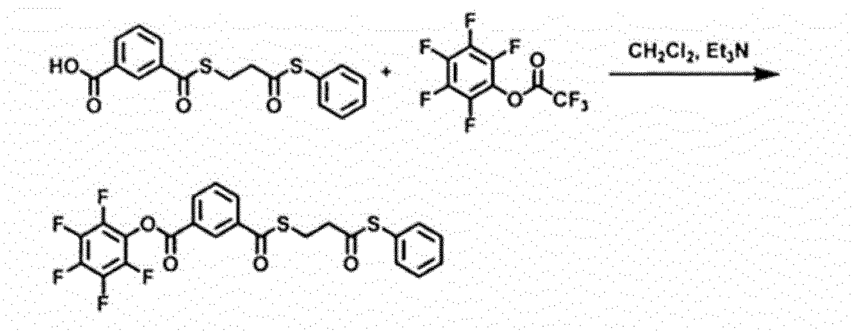

To the compound (227 mg, 0.66 mmol) synthesized in (18-2-1-2), $CH_2Cl_2$ (3.3 mL) and triethylamine (230 μL, 1.65 mmol) were added and dissolved. Pentafluorophenyl trifluoroacetic acid (225 μL, 1.32 mmol) was added at 0°C and the mixture was stirred for 1 h. After confirming the reaction with TLC (hexane/ethyl acetate=3/1), the reaction solutions were concentrated. It was eluted with a mixed solution of hexane and ethyl acetate and the fractions were confirmed by TLC (hexane/ethyl acetate=3/1). Fractions containing the product were collected and concentrated under reduced pressure to remove the organic solvents, followed by vacuum-drying to obtain the above compound (175.3 mg, 0.34 mmol).

$^1H$ NMR (400 MHz, Chloroform-d) δ=8.79 (t, J=1.8, 1H), 8.43 (dt, J=7.8, 1.5, 1H), 8.30 (dt, J=7.9, 1.5, 1H), 7.70 (t, J=7.8, 1H), 7.45 (s, 4H), 3.45 (t, J=6.9, 2H), 3.14 (t, J=6.9, 2H).

(18-2-2) Linkage between the peptide and the linker

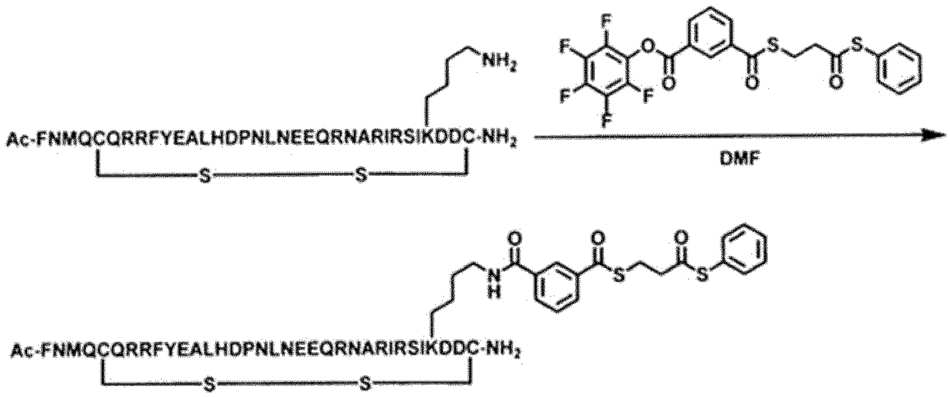


Both of the two aforementioned acid sequences are the amino acid sequence of SEQ ID NO: 4.

Ac-FNMQCQRRFYEALHDPNLNEEQRNARIRSIKDDC-$NH_2$ (SEQ ID NO: 4) (30.0 mg,7.06 μmol, 30.0 mg,7. 06 μmol, where two cysteines at positions 5 and 34 each forms a disulfide bond in the molecule) synthesized with (18-1-2) was dissolved in N,N-dimethylformamide (1.00 mL), a linker (72.0 mg, 141 μmol) was added, and the mixture was stirred at room temperature for 24 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the linked product of peptide thioester-thiophenol activator (10.0 mg, 2.19 pmol).

MS(ESI) m/z: z=4 1145.6$[M+4H]^{4+}$

(18-2-3) Specific Modification of the Anti-HER2 Antibody Trastuzumab and Analysis by ESI-TOFMS

The linked product of peptide linker synthesized in (18-2-2) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The raw material trastuzumab peaked at 148223 as measured by ESI-TOFMS. A peak of 152691 in which one binding peptide was introduced, 157163 in which two binding peptides were introduced, and 161634 in which three binding peptides were introduced were confirmed.

(18-2-4) Confirmation of Heavy Chain Selectivity by ESI-TOFMS Analysis of Specific Modified Trastuzumab under Reducing Conditions

To the antibody-peptide complex produced in (18-2-3), 2μL of 100 mM tris(2-carboxyethyl)phosphine hydrochloride (equivalent to the antibody) was added, and the mixture was stirred at room temperature for 15 minutes. The masses were measured by ESI-TOFMS. As a result, raw material trastuzumab was observed to have a heavy chain peak at 50596 and a light chain peak at 23439. The reactants had a peak at 55067 where one linker was introduced into the heavy chain and at 23439 where the light chain was the same as the starting material.

(18-2-5) Confirmation of Peptide/Antibody Binding by DAR calculator of Specific Modifications of Trastuzumab

For MS data analyzed in (18-2-3), the peptide/antibody bonding ratio was confirmed by DAR calculator (Agilent Software). The results are shown in Table 6. The average peptide/antibody bonding ratio calculated from DAR peak and % Area in Table-6 was 2.0. Therefore, the production of an antibody intermediate (average peptide/antibody bonding ratio 2.0) represented by the following structural formula was confirmed.

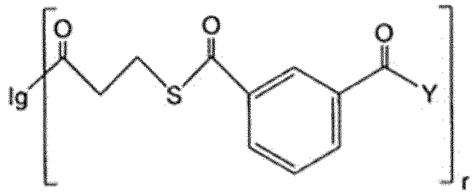

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 4,
The average bond ratio r between Ig and the two carbonyl groups (C=O) adjacent to Ig is 2.0.]

Table 6

| DAR Peak | Observed mass(Da) | Peak Area | %Area |
|---|---|---|---|
| 1 | 152691 | 5.74E+003 | 4.25 |
| 2 | 157163 | 1.26E+005 | 93.33 |
| 3 | 161634 | 3.27E+003 | 2.42 |

(18-2-6) Preparation of Thiol Group-Introduced Antibody Derivatives by Cleavage of Thioester Group

The thiol group-introduced antibody derivative was obtained by subjecting the antibody intermediate obtained in (18-2-5) to the cleavage reaction of the thioester group described in (18-1-8) to obtain the thiol group-introduced antibody derivative described in (18-1-8).

(18-2-7) Peptide mapping by trypsinization

The thiol-introduced trastuzumab obtained in (18-2-6) was subjected to peptide mapping in the following step.

(18-2-7-1) Trypsinization of the thiol-introduced trastuzumab

Trypsinization of the thiol-introduced trastuzumab obtained in (18-2-6) was performed in the same manner as in (18-1-9-1).

(18-2-7-2) LC-MS/MS determination of trastuzumab

LC-MS/MS was measured in the same manner as in (18-1-9-2) .

(18-2-7-3) Analysis of trastuzumab modification sites

Analysis was performed in the same manner as in (18-1-9-3) .

(18-2-7-4) Analysis of trastuzumab LC-MS/MS modification sites

Analysis using LC-MS/MS revealed that MS spectrum (found: m/z 577.03571, theoretical value: 577.03557, 4

valence) of the peptide fragment FNWYVDGVEVHNAKTKPR (SEQ ID NO: 3) which is a peptide consisting of 18 amino acids including a modification site of trastuzumab to lysine residue by tryptic digestion of trastuzumab (thiol-introduced trastuzumab (+145.019 Da) which is carbamidomethylated by iodoacetamide) was observed (FIG. 10), and m/z 682.41 (theoretical value: 682.01) corresponding to a trivalent y16 showing modification of the lysine residue at position 288 or 290 of the heavy chain according to EU numbering was confirmed from CID spectrum (FIG. 11). Analysis with BioPharma Finder also showed that the modification to the lysine residue at position 288 or 290 occurred highly selectively (FIG. 12).
From these results, it was found that in the trastuzumab thiol transfectant obtained in (18-2-6) above, conjugation proceeds regioselectively on the heavy chain of the antibody to Lys288 and Lys290 according to EU numbering.

(18-3-1) Synthesis of the thioester linker

(18-3-1-1)

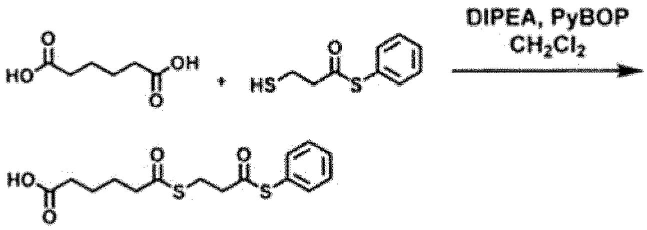


The compound (220 mg, 1.21 mmol) synthesized with (18-1-3-2) was dissolved in $CH_2Cl_2$ (12.0 mL) and the mixture was stirred for 1 h at normal temperature with Adipic Acid (530 mg, 3.63 mmol), DIPEA (314 μL, 1.82 mmol), and PyBOP (755 mg, 1.45 mmol). After confirming the reaction with TLC (dichloromethane/methanol=10/1), the reaction solutions were concentrated. It was eluted with a mixed solution of dichloromethane and methanol, and the fractions were confirmed by TLC (dichloromethane/methanol=10/1). Fractions containing the product were collected and concentrated under reduced pressure to remove the organic solvents, followed by vacuum-drying to obtain the above compound (205 mg, 0.63 mmol).

(18-3-1-2)

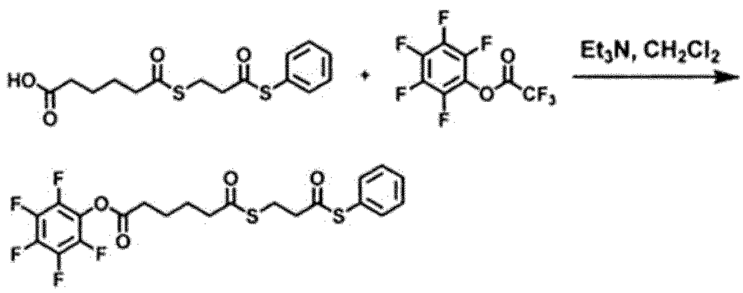


$CH_2Cl_2$ (3.15 mL) and triethylamine (220 μL, 1.58 mmol) were added to the compound (205 mg, 0.63 mmol) synthesized in (18-3-1-1) and dissolved. Pentafluorophenyl trifluoroacetic acid (215μL, 1.26mmol) was added at 0°C and the mixture was stirred for 1 h. After confirming the reaction with TLC (hexane/ethyl acetate=3/1), the reaction solutions were concentrated. It was eluted with a mixed solution of hexane and ethyl acetate and the fractions were confirmed by TLC (hexane/ethyl acetate=3/1). Fractions containing the product were collected and concentrated under reduced pressure to remove the organic solvents, followed by vacuum-drying to obtain the above compound (200 mg, 0.41 mmol).
$^1H$ NMR (400 MHz, Chloroform-d) δ=7.44 (s, 5H), 3.20 (t, J=7.0, 2H), 3.00 (t, J=6.9, 2H), 2.66 (dt, J=28.3, 7.4, 5H), 1.79 (ddt, J=20.4, 15.2, 7.5, 5H), 1.57-1.38 (m, 3H).

(18-3-2) Linkage between the peptide and the linker

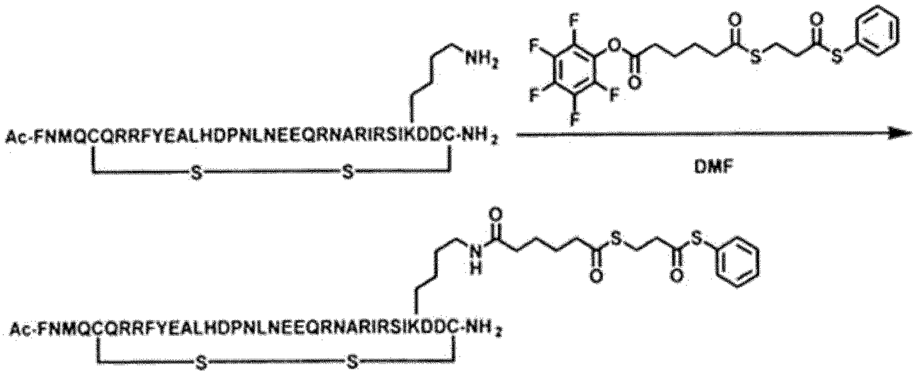


Both of the two aforementioned acid sequences are the amino acid sequence of SEQ ID NO: 4.

Ac-FNMQCQRRFYEALHDPNLNEEQRNARIRSIKDDC-$NH_2$ (SEQ ID NO: 4) (30.0 mg, 7.06 μmol, where two cysteines at positions 5 and 34 each forms a disulfide bond in the molecule) synthesized with (18-1-2) was dissolved in N,N-dimethylformamide (1.00 mL), a linker (69.0 mg, 141 μmol) was added, and the mixture was stirred at room temperature for 24 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the linked product of peptide thioester-thiophenol activator (7.5 mg, 1.65 pmol).

MS(ESI) m/z: z=4 1140.50$[M+4H]^{4+}$

(18-3-3) Specific Modification of the Anti-HER2 Antibody Trastuzumab and Analysis by ESI-TOFMS

The linked product of peptide linker synthesized in (18-3-2) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The raw material trastuzumab peaked at 148223 as measured by ESI-TOFMS. A peak of 152676 in which one binding peptide was introduced, 157126 in which two binding peptides were introduced, and 161572 in which three binding peptides were introduced were confirmed.

(18-3-4) Confirmation of Heavy Chain Selectivity by ESI-TOFMS Analysis of Specific Modified Trastuzumab under Reducing Conditions

To the antibody-peptide complex produced in (18-3-3), 2 μL of 100 mM tris(2-carboxyethyl)phosphine hydrochloride (equivalent to antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55048 where one linker was introduced into the heavy chain and at 23439 where the light chain was the same as the starting material.

(18-3-5) Confirmation of Peptide/Antibody Binding by DAR calculator of Specific Modifications of Trastuzumab

For MS data analyzed in (18-3-3), the peptide/antibody bonding ratio was confirmed by DAR calculator (Agilent Software). The results are shown in Table 7. The average peptide/antibody bonding ratio calculated from DAR peak and % Area in Table-7 was 1.9. Therefore, the production of an antibody intermediate (average peptide/antibody bonding ratio 1.9) represented by the following structural formula was confirmed.

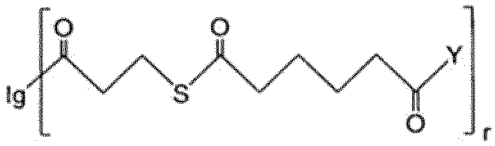

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 4,
The average bond ratio r between Ig and the two carbonyl groups (C=O) adjacent to Ig is 1.9.]

Table 7

| DAR Peak | Observed mass (Da) | Peak Area | %Area |
|---|---|---|---|
| 1 | 152676 | 1.71E+004 | 13.70 |
| 2 | 157126 | 1.01E+005 | 81.15 |
| 3 | 161572 | 6.43E+003 | 5.15 |

(18-3-6) Preparation of Thiol Group-Introduced Antibody Derivatives by Cleavage of Thioester Group

The thiol group-introduced antibody derivative was obtained by subjecting the antibody intermediate obtained in (18-3-5) to the cleavage reaction of the thioester group described in (18-1-8) to obtain the thiol group-introduced antibody derivative described in (18-1-8).

(18-4-1) Synthesis of the thioester linker

(18-4-1-1)

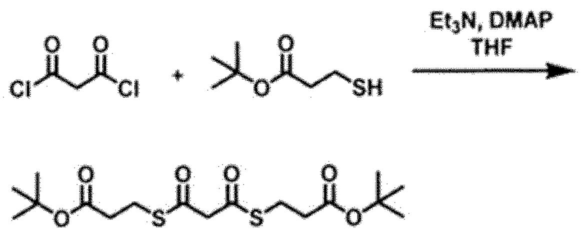


tBu-3-Sulfanylpropanoate (500 mg, 3.08 mmol) was dissolved in tetrahydrofuran (7 mL), triethylamine (0.64 mL, 4.62 mmol) was added, and then malonyl chloride (0.15 mg, 1.54 mmol) was added at 0°C and the mixture was stirred for 3 hours. After confirming the reaction with TLC (hexane/ethyl acetate=5/1), the reaction solutions were concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (104 mg, 0.26 mmol) .

(18-4-1-2)

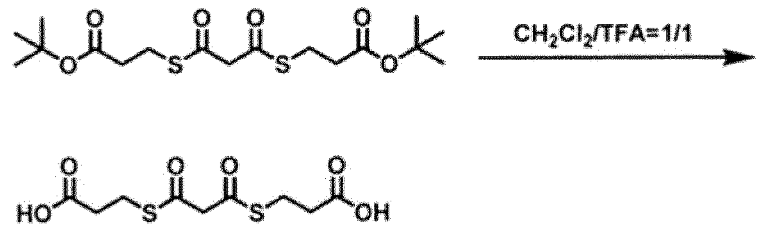


The compound (104 mg, 0.26 mmol) synthesized in (18-4-1-1) was dissolved in a mixed solution of $CH_2Cl_2$ and TFA=1/1, and the mixture was stirred at room temperature for 1 hour. After confirming that the reaction solution had fallen to the starting point with TLC (hexane/ethyl acetate=5/1), the reaction solution was concentrated and then dried under reduced pressure to obtain the above compound (104 mg, 0.37 mmol).

(18-4-1-3)

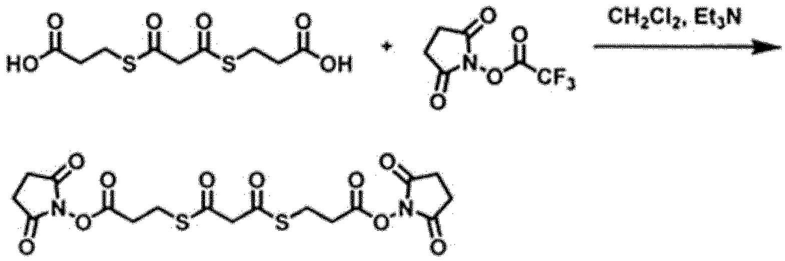


To the compound (104 mg, 0.37 mmol) synthesized in (18-4-1-2), $CH_2Cl_2$ (1.85 mL) and triethylamine (130 μL, 0.93 mmol) were added and dissolved. N-SuccinimidylTrifluoroacetate (156 mg, 0.74 mmol) was added at 0°C and the mixture was stirred for 1 h. After confirming the reaction with TLC (hexane/ethyl acetate=1/1), the reaction solutions were concentrated. It was eluted with a mixed solution of hexane and ethyl acetate, and the fractions were confirmed by TLC (hexane/ethyl acetate=1/1). Fractions containing the product were collected and concentrated under reduced pressure to remove the organic solvents, followed by vacuum-drying to obtain the above compound (66.8 mg, 0.14 mmol) .

$^1$H NMR (400 MHz, Chloroform-d) δ=3.84 (s, 2H), 3.28 (t, J=6.9, 2H), 3.19 (t, J=6.8, 2H), 3.00 (t, J=6.8, 2H), 2.74 (t, J=6.8, 2H).

(18-4-2) Linkage between the peptide and the linker

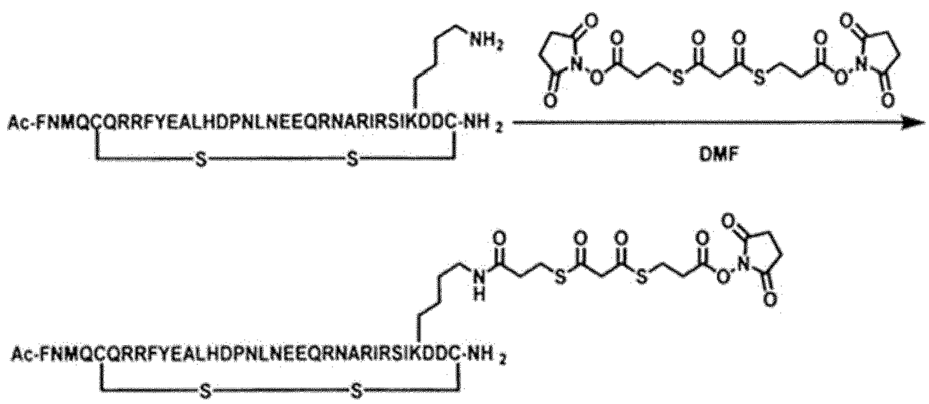


Both of the two aforementioned acid sequences are the amino acid sequence of SEQ ID NO: 4.

Ac-FNMQCQRRFYEALHDPNLNEEQRNARIRSIKDDC-$NH_2$ (SEQ ID NO: 4) (7.00 μmol 29.7 mg, where two cysteines at positions 5 and 34 each forms a disulfide bond in the molecule) synthesized in (18-1-2) were dissolved in

N,N-dimethylformamide (1.00 mL), added with a linker (66.8 mg, 0.14 mmol), and agitated at room temperature for 4 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the linked product of peptide thioester-NHS activator (13 mg, 2.82 pmol).

MS(ESI) m/z: z=4 1153.10$[M+4H]^{4+}$

(18-4-3) Specific Modification of the Anti-HER2 Antibody Trastuzumab and Analysis by ESI-TOFMS

The linked product of peptide linker synthesized in (18-4-2) was dissolved in dimethyl sulfoxide so as to be 10 mM. 500 μg of the anti-HER2 antibody trastuzumab (Chugai Pharmaceutical Co., Ltd.) was dissolved in 200μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The raw material trastuzumab peaked at 148223 as measured by ESI-TOFMS. A peak of 152722 in which one binding peptide was introduced, 157215 in which two binding peptides were introduced, and 161708 in which three binding peptides were introduced were confirmed.

(18-4-4) Confirmation of Heavy Chain Selectivity by ESI-TOFMS Analysis of Specific Modified Trastuzumab under Reducing Conditions

To the antibody-peptide complex produced in (18-4-3), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50594 and a light chain peak at 23439. The reactants had a peak at 55091 where one linker was introduced into the heavy chain and at 23439 where the light chain was the same as the starting material.

(18-4-5) Confirmation of Peptide/Antibody Binding by DAR calculator of Specific Modifications of Trastuzumab

For MS data analyzed in (18-4-3), the peptide/antibody bonding ratio was confirmed by DAR calculator (Agilent Software). The results are shown in Table 8. The average peptide/antibody bonding ratio calculated from DAR peak and % Area in Table-8 was 1.8. Therefore, the production of an antibody intermediate (average peptide/antibody bonding ratio 1.8) represented by the following structural formula was confirmed.

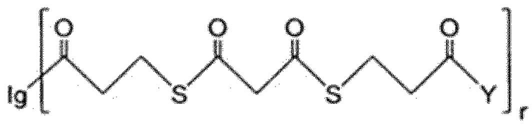

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 4,
The mean bond ratio r between Ig and the two carbonyl groups (C=O) adjacent to Ig is 1.8.]

Table 8

| DAR Peak | Observed mass (Da) | Peak Area | %Area |
|---|---|---|---|
| 1 | 152722 | 4.47E+005 | 24.92 |
| 2 | 157215 | 1.21E+006 | 67.20 |
| 3 | 161708 | 1.22E+005 | 6.78 |

(18-4-6) Preparation of Thiol Group-Introduced Antibody Derivatives by Cleavage of Thioester Group

The thiol group-introduced antibody derivative was obtained by subjecting the antibody intermediate obtained in (18-4-5) to the cleavage reaction of the thioester group described in (18-1-8) to obtain the thiol group-introduced antibody derivative described in (18-1-8).

(18-5-1) Linkage between the peptide and the linker

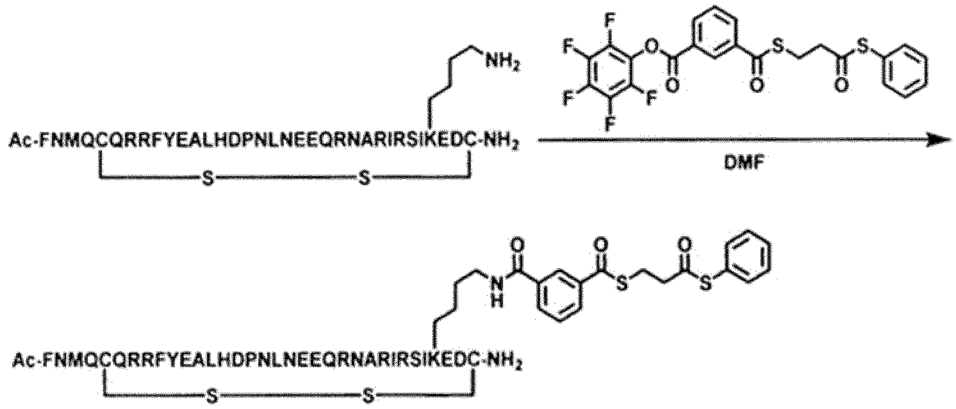


Both of the two aforementioned acid sequences are the amino acid sequence of SEQ ID NO: 5.

The linker was attached to Ac-FNMQCQRRFYEALHDPNLNEEQRNARIRSIKEDC-$NH_2$ (SEQ ID NO: 5) synthesized by the method described in (18-1-1) (30.0 mg, 7.06 μmol, where two cysteines at positions 5 and 34 each forms a disulfide bond in the molecule) in the same manner as in Example 2 (2-2) to obtain the linked product of peptide linker-thiophenol activator (10.0 mg, 2.19 μmol).

MS(ESI)m/z:z=4 1145.6$[M+4H]^{4+}$

(18-5-2) Specific Modification of the Anti-HER2 Antibody Trastuzumab and Analysis by ESI-TOFMS

The linked product of peptide linker synthesized in (18-5-1) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The raw material trastuzumab peaked at 148223 as measured by ESI-TOFMS. A peak of 152707 in which one binding peptide was introduced, 157188 in which two binding peptides were introduced, and 161676 in which three binding peptides were introduced were confirmed.

(18-5-3) Confirmation of Heavy Chain Selectivity by ESI-TOFMS Analysis of Specific Modified Trastuzumab under Reducing Conditions

To the antibody-peptide complex produced in (18-5-2), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55077 where one linker was introduced into the heavy chain and at 23439 where the light chain was the same as the starting material.

(18-5-4) Confirmation of Peptide/Antibody Binding by DAR calculator of Specific Modifications of Trastuzumab

For MS data analyzed in (5-2), the peptide/antibody bonding ratio was confirmed by DAR calculator (Agilent Software). The results are shown in Table 9. The average peptide/antibody bonding ratio calculated from DAR peak and % Area in FIG. 9 was 2.0. Therefore, the production of an antibody intermediate (average peptide/antibody bonding ratio 2.0) represented by the following structural formula was confirmed.

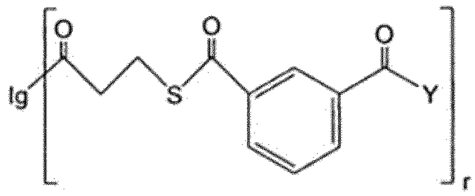

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 5,
The average bond ratio r between Ig and the two carbonyl groups (C=O) adjacent to Ig is 2.0.]

Table 9

| DAR Peak | Observed mass (Da) | Peak Area | %Area |
|---|---|---|---|
| 1 | 152707 | 6.90E+003 | 2.22 |
| 2 | 157188 | 2.96E+005 | 95.07 |
| 3 | 161676 | 8.47E+003 | 2.72 |

(18-5-5) Preparation of Thiol Group-Introduced Antibody Derivatives by Cleavage of Thioester Group

The thiol group-introduced antibody derivative was obtained by subjecting the antibody intermediate obtained in (18-5-4) to the cleavage reaction of the thioester group described in (18-1-8) to obtain the thiol group-introduced antibody derivative described in (18-1-8).

(18-6-1) Linkage between the peptide and the linker

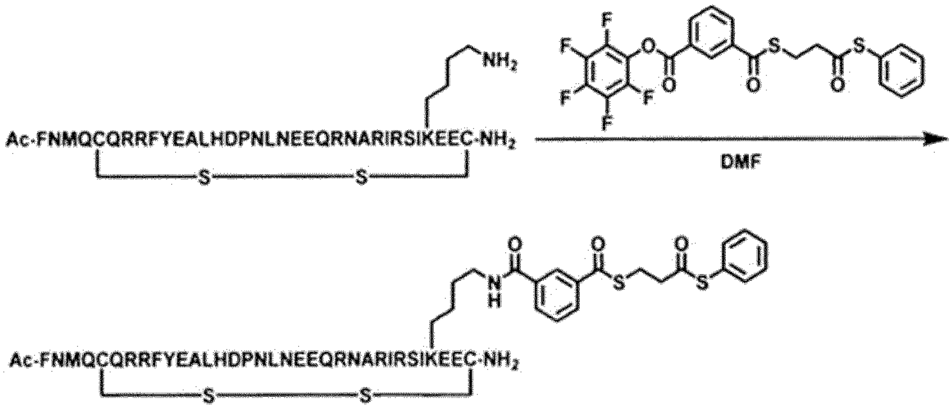


Both of the two aforementioned acid sequences are the amino acid sequence of SEQ ID NO: 6.
A linker was attached to Ac-FNMQCQRRFYEALHDPNLNEEQRNARIRSIKEEC-$NH_2$ (SEQ ID NO: 6) synthesized by the method described in (18-1-1) (SEQ ID NO: 6) (30.0 mg,7. 06 μmol, where two cysteines at positions 5 and 34 each forms a disulfide bond in the molecule) in the same manner as in (18-2-2) to obtain the linked product of peptide thioester linker-thiophenol activator (22.2 mg, 4.82 pmol).
MS(ESI) m/z: z=4 1152.4$[M+4H]^{4+}$

(18-6-2) Specific Modification of the Anti-HER2 Antibody Trastuzumab and Analysis by ESI-TOFMS

The linked product of peptide linker synthesized in (18-6-1) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The raw material trastuzumab peaked at 148223 as measured by ESI-TOFMS. A peak of 152720 in which one binding peptide was introduced, 157216 in which two binding peptides were introduced, and 161716 in which three binding peptides were introduced were confirmed.

(18-6-3) Confirmation of Heavy Chain Selectivity by ESI-TOFMS Analysis of Specific Modified Trastuzumab under Reducing Conditions

To the antibody-peptide complex produced in (18-6-2), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55091 where one linker was introduced into the heavy chain and at 23439 where the light chain was the same as the starting material.

(18-6-4) Confirmation of Peptide/Antibody Binding by DAR calculator of Specific Modifications of Trastuzumab

For MS data analyzed in (18-6-2), the peptide/antibody bonding ratio was confirmed by DAR calculator (Agilent Software). The results are shown in Table 10. The average peptide/antibody bonding ratio calculated from DAR peak and % Area in Table-10 was 2.0. Therefore, the production of an antibody intermediate (average peptide/antibody bonding ratio 2.0) represented by the following structural formula was confirmed.

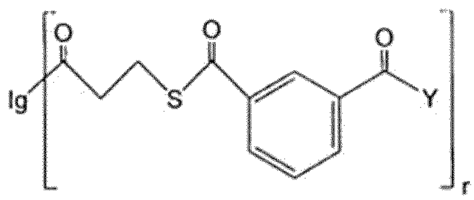

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 6,
The average bond ratio r between Ig and the two carbonyl groups (C=O) adjacent to Ig is 2.0.]

Table 10

| DAR Peak | Observed mass(Da) | Peak Area | %Area |
|---|---|---|---|
| 1 | 152720 | 5.84E+003 | 2.31 |
| 2 | 157216 | 2.41E+005 | 95.07 |
| 3 | 161716 | 6.65E+003 | 2.63 |

(18-6-5) Preparation of Thiol Group-Introduced Antibody Derivatives by Cleavage of Thioester Group

The thiol group-introduced antibody derivative was obtained by subjecting the antibody intermediate obtained in (18-6-4) to the cleavage reaction of the thioester group described in (18-1-8) to obtain the thiol group-introduced antibody derivative described in (18-1-8).

(18-7-1) Linkage between the peptide and the linker

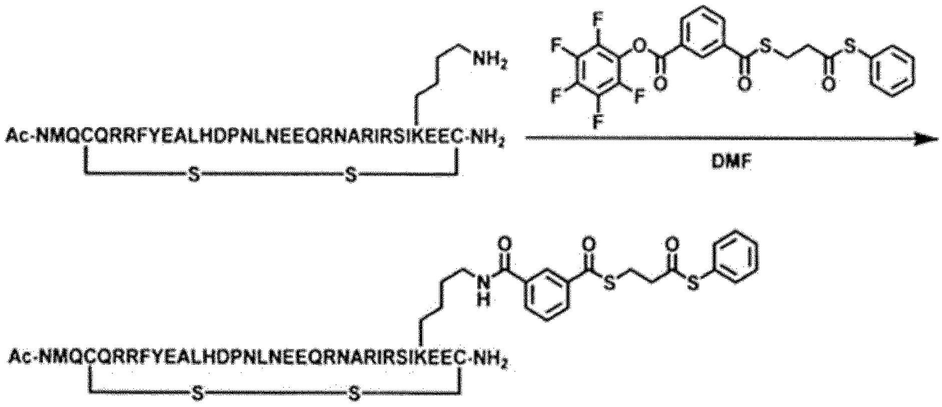


Both of the above two amino acid sequences are the amino acid sequence of SEQ ID NO: 7.

A linker was attached to Ac-NMQCQRRFYEALHDPNLNEEQRNARIRSIKEEC-$NH_2$ (SEQ ID NO: 7) synthesized by the method described in (18-1-1) (SEQ ID NO: 06μmol of 30.0mg,7., where two cysteines at positions 5 and 34 each forms a disulfide bond in the molecule) in the same manner as (18-2-2) to obtain the linked product of peptide thioester linker-thiophenol activator (12.0 mg, 2.69 μmol).

MS(ESI)m/z:z=4 1115.8$[M+4H]^{4+}$

(18-7-2) Specific Modification of the Anti-HER2 Antibody Trastuzumab and Analysis by ESI-TOFMS

The linked product of peptide linker synthesized in (18-7-1) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The raw material trastuzumab peaked at 148223 as measured by ESI-TOFMS. 152573 peaks in which one binding peptide was introduced and 156927 peaks in which two binding peptides were introduced were confirmed.

(18-7-3) Confirmation of Heavy Chain Selectivity by ESI-TOFMS Analysis of Specific Modified Trastuzumab under Reducing Conditions

To the antibody-peptide complex produced in (18-7-2), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 54942 where one linker was introduced into the heavy chain and at 23439 where the light chain was the same as the starting material.

(18-7-4) Confirmation of Peptide/Antibody Binding by DAR calculator of Specific Modifications of Trastuzumab

For MS data analyzed in (18-7-2), the peptide/antibody bonding ratio was confirmed by DAR calculator (Agilent Software). The results are shown in Table 11. The average peptide/antibody bonding ratio calculated from DAR peak and % Area in Table-11 was 2.0. Therefore, the production of an antibody intermediate (average peptide/antibody bonding ratio 2.0) represented by the following structural formula was confirmed.

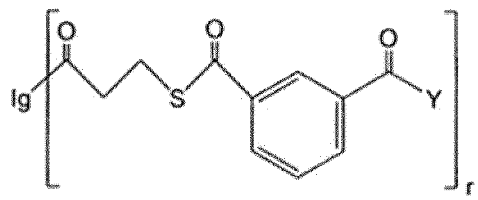

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 7,
The average bond ratio r between Ig and the two carbonyl groups (C=O) adjacent to Ig is 2.0.]

Table 11

| DAR Peak | Observed mass (Da) | Peak Area | %Area |
|---|---|---|---|
| 1 | 152573 | 9.84E+004 | 9.08 |
| 2 | 156927 | 1.11E+006 | 90.92 |

(18-7-5) Preparation of Thiol Group-Introduced Antibody Derivatives by Cleavage of Thioester Group

The thiol group-introduced antibody derivative was obtained by subjecting the antibody intermediate obtained in (18-7-4) to the cleavage reaction of the thioester group described in (18-1-8) to obtain the thiol group-introduced antibody derivative described in (18-1-8).

(18-7-6) Peptide mapping by trypsinization

The thiol-introduced trastuzumab obtained in (18-7-5) was subjected to peptide mapping in the following step.

(18-7-6-1) Trypsinization of the thiol-introduced trastuzumab

Trypsinization of the thiol-introduced trastuzumab obtained in (18-7-5) was performed in the same manner as in (18-1-9-1).

(18-7-6-2) LC-MS/MS determination of trastuzumab

LC-MS/MS was measured in the same manner as in (18-1-9-2) .

(18-7-6-3) Analysis of trastuzumab modification sites

Analysis was performed in the same manner as in (18-1-9-3) .

(18-7-6-4) Analysis of trastuzumab LC-MS/MS modification sites

Analysis using LC-MS/MS revealed that MS spectrum (found: m/z 769.04506, theoretical value: 769.04482, trivalent) of the peptide fragment FNWYVDGVEVHNAKTKPR (SEQ ID NO: 3) which is a peptide consisting of 18 amino acids including a modification site of trastuzumab to lysine residue by tryptic digestion of trastuzumab (thiol-introduced trastuzumab (+145.019 Da) which is carbamidomethylated by iodoacetamide) was observed (FIG. 13), and m/z 1022.71 (theoretical value: 1022.51) corresponding to a divalent y16 showing modification of the lysine residue at position 288 or 290 of the heavy chain according to EU numbering was confirmed from CID spectrum (FIG. 14). Analysis with BioPharma Finder also showed that the modification to the lysine residue at position 288 or 290 occurred highly selectively (FIG. 15).

It was found that the trastuzumab thiol transfectants obtained in the above (18-7-5) were conjugated to Lys288 and Lys290 of the heavy chain of the antibody according to EU numbering.

(18-8-1) Linkage between the peptide and the linker

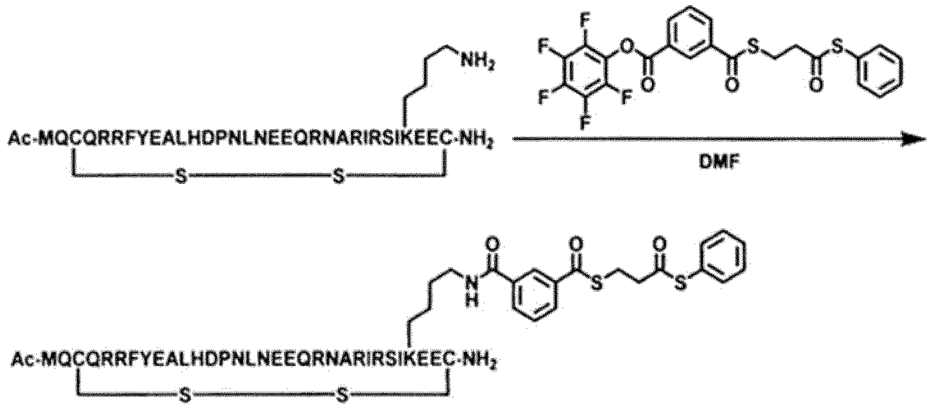


Both of the above two amino acid sequences are the amino acid sequence of SEQ ID NO: 8.

A linker was attached to Ac-MQCQRRFYEALHDPNLNEEQRNARIRSIKEEC-$NH_2$ (SEQ ID NO: 8) synthesized by the method described in (18-1-1) (SEQ ID NO: 8) (30.0 mg, 7.06 μmol, where two cysteines at positions 5 and 34 each forms a disulfide bond in the molecule) in the same manner as (18-2-2) to obtain the linked product of peptide thioester linker-thiophenol activator (16.8 mg, 3.87 pmol).

MS(ESI) m/z: z=4 1087.3$[M+4H]^{4+}$

(18-8-2) Specific Modification of the Anti-HER2 Antibody Trastuzumab and Analysis by ESI-TOFMS

The linked product of peptide linker synthesized in (18-8-1) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with 20 mM ammonium acetate buffer. The masses were measured by ESI-TOFMS. As a result, the raw material trastuzumab had a peak at 148223. A peak 152457 in which one binding peptide was introduced, a peak 156692 in which two binding peptides were introduced, and a peak 160929 in which three binding peptides were introduced were confirmed.

(18-8-3) Confirmation of Heavy Chain Selectivity by ESI-TOFMS Analysis of Specific Modified Trastuzumab under Reducing Conditions

To the antibody-peptide complex produced in (18-8-2), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 54829 where one linker was introduced into the heavy chain and a peak at 23439 where the light chain was the same as the starting material.

(18-8-4) Confirmation of Peptide/Antibody Binding by DAR calculator of Specific Modifications of Trastuzumab

For MS data analyzed in (18-8-2), the peptide/antibody bonding ratio was confirmed by DAR calculator (Agilent Software). The results are shown in Table 12. The average peptide/antibody bonding ratio calculated from DAR peak and % Area in Table-12 was 2.0. Therefore, the production of an antibody intermediate (average peptide/antibody bonding ratio 2.0) represented by the following structural formula was confirmed.

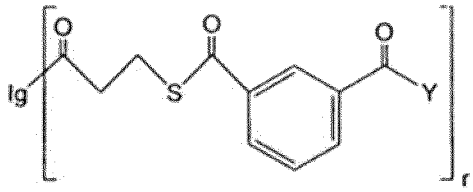

[where Ig represents an immunoglobulin unit (IgG) comprising two heavy chains and two light chains and forms an amide bond with two carbonyl groups (C=O) adjacent to Ig via an amino group in the side chain of the lysine residue present

at position 288/290 in the two heavy chains according to Eu numbering;

Y represents an affinity peptide represented by the amino acid sequence of SEQ ID NO: 8,
The average bond ratio r between Ig and the two carbonyl groups (C=O) adjacent to Ig is 2.0.]

Table 12

| DAR Peak | Observed mass(Da) | Peak Area | %Area |
|---|---|---|---|
| 1 | 152457 | 1.74E+004 | 3.08 |
| 1 | 156692 | 5.22E+005 | 93.53 |
| 3 | 160929 | 1.89E+004 | 3.39 |

(18-8-5) Preparation of Thiol Group-Introduced Antibody Derivatives by Cleavage of Thioester Group

The thiol group-introduced antibody derivative was obtained by subjecting the antibody intermediate obtained in (18-8-4) to the cleavage reaction of the thioester group described in (18-1-8) to obtain the thiol group-introduced antibody derivative described in (18-1-8).

(18-9-1) Linkage between the peptide and the linker

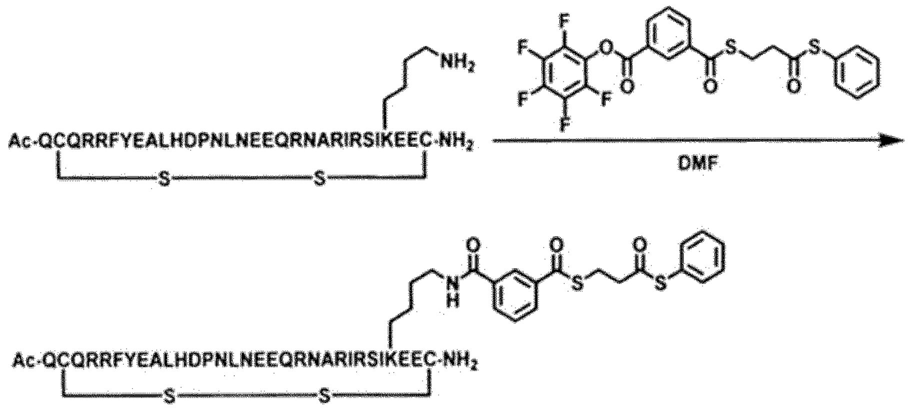


Both of the two aforementioned acid sequences are the amino acid sequence of SEQ ID NO: 9.
A linker was attached to Ac-QCQRRFYEALHDPNLNEEQRNARIRSIKEEC-$NH_2$ (SEQ ID NO: 9) synthesized by the method described in (18-9-1) (SEQ ID NO: 9) (30.0 mg,7.06 μmol, where two cysteines at positions 5 and 34 each forms a disulfide bond in the molecule) in the same manner as (18-2-2) to obtain the linked product of peptide thioester linker-thiophenol activator (14.1 mg, 3.35 pmol).
MS(ESI) m/z: z=4 1054.4$[M+4H]^{4+}$

(18-9-2) Specific Modification of the Anti-HER2 Antibody Trastuzumab and Analysis by ESI-TOFMS

The linked product of peptide linker synthesized in (18-9-1) was dissolved in dimethyl sulfoxide so as to be 10 mM. 500 μg of the anti-HER2 antibody trastuzumab (Chugai Pharmaceutical Co., Ltd.) was dissolved in 200 μL (20 μM) of 50 mM HEPES buffer (pH 8.2), and 3.38 μL of 10 mM peptide reagent (10 equivalents to the antibody) was added and the mixture was stirred at room temperature for 1 hour. The reaction solution was replaced with ammonium 20 mM acetate buffer. The masses were measured by ESI-TOFMS. As a result, the raw material trastuzumab had a peak at 148223. A peak 152236 in which one binding peptide was introduced, a peak 156430 in which two binding peptides were introduced, and a peak 160541 in which three binding peptides were introduced were confirmed.

(18-9-3) Confirmation of Heavy Chain Selectivity by ESI-TOFMS Analysis of Specific Modified Trastuzumab under Reducing Conditions

To the antibody-peptide complex produced in (18-9-2), 2 μL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 54698 where one linker was introduced into the heavy chain and a peak at 23439 where the light chain was the same as the starting material.

(18-9-4) Confirmation of Peptide/Antibody Binding by DAR calculator of Specific Modifications of Trastuzumab

For MS data analyzed in (18-9-2), the peptide/antibody bonding ratio was confirmed by DAR calculator (Agilent Software). The results are shown in Table 13. The average peptide/antibody bonding ratio calculated from DAR peak and % Area in Table-13 was 2.0. Therefore, the production of an antibody intermediate (average peptide/antibody bonding ratio 2.0) represented by the following structural formula was confirmed.

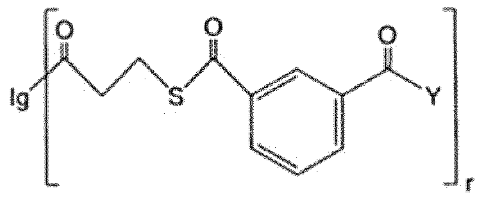

[where Ig represents an immunoglobulin unit (IgG) comprising two heavy chains and two light chains and forms an amide bond with two carbonyl groups (C=O) adjacent to Ig via an amino group in the side chain of the lysine residue present at position 288/290 in the two heavy chains according to Eu numbering;

Y represents an affinity peptide represented by the amino acid sequence of SEQ ID NO: 9,
The average bond ratio r between Ig and the two carbonyl groups (C=O) adjacent to Ig is 2.0.]

Table 13

| DAR Peak | Observed mass (Da) | Peak Area | %Area |
|---|---|---|---|
| 1 | 152236 | 1.24E+004 | 7.09 |
| 2 | 156430 | 1.57E+005 | 89.82 |
| 3 | 160541 | 5.41E+003 | 3.09 |

(18-9-5) Preparation of Thiol Group-Introduced Antibody Derivatives by Cleavage of Thioester Group

The thiol group-introduced antibody derivative was obtained by subjecting the antibody intermediate obtained in (18-9-4) to the cleavage reaction of the thioester group described in (18-1-8) to obtain the thiol group-introduced antibody derivative described in (18-1-8).

(18-10-1) Introduction of Bioorthogonal Functional Groups to Thiols Introduced at Ly288/Lys290 Positions of Antibodies

The azide linker (1) of Example 1 was used to modify Ly288/Lys290 thiol-introduced antibody synthesized in Example (18-1-8). To the product was added tris(2-carboxyethyl)phosphine hydrochloride (0.5 mM) and the mixture was stirred at room temperature for 10 minutes and the masses were mesured by ESI-TOFMS according to the previous report (WO2019/240287A1). The source thiol group-introduced antibody showed heavy chain peaks at 50683 and 50845 and a light chain peak at 23439, and the products showed heavy chain peaks at 50816 and 50978 in which an azide group(s) is introduced to a heavy chain(s), and a light chain peak at 23439 which were the same as the source.

In a similar manner, Ly288/Lys290 thiol-introduced antibody synthesized in Example (18-1-8) was modified using the azide linker (2) of Example 2. To the product was added tris(2-carboxyethyl)phosphine hydrochloride (0.5 mM) and the mixture was stirred at room temperature for 10 minutes and the mass were measured by ESI-TOFMS according

to the previous report (WO2019/240287A1). The source thiol group-introduced antibody had heavy chain peaks at 50683 and 50845, and a light chain peak at 23439, and the products had heavy chain peaks at 50848 and 51011 in which an azide group(s) is introduced to a heavy chain(s), and a light chain peak at 23439 which were the same as the source.

In a similar manner, Ly288/Lys290 thiol-introduced antibody synthesized in Example (18-1-8) was modified using the azide linker (6) synthesized in Example 6. The reaction solution was replaced with ammonium 20 mM acetate buffer, and the mass was measured by ESI-TOFMS according to the method described in a previous report (Anal. Chem. 2019, 91, 20, 12724-12732), and a peak 149023 in which two azide groups were introduced into the antibody were confirmed.

In a similar manner, Ly288/Lys290 thiol-introduced antibody synthesized in Example (18-1-8) was modified using the alkyne linker (8) of Example 8. To the product was added tris(2-carboxyethyl)phosphine hydrochloride (0.5 mM) and the mixture was stirred at room temperature for 10 minutes and measured by ESI-TOFMS according to the previous report (WO2019/240287A1). The source thiol group-introduced antibody showed heavy chain peaks at 50683 and 50845 and a light chain peak at 23439, and the products showed heavy chain peaks at 51137 and 51304 in which an alkyne group(s) was introduced into a heavy chain(s), and a light chain peak at 23439 which was the same as the source.

In a similar manner, Ly288/Lys290 thiol-introduced antibody synthesized in Example (18-1-8) was modified using the alkyne linker (11) synthesized in Example 11. The reaction solution was replaced with ammonium 20 mM acetate buffer, and the mass was measured by ESI-TOFMS according to the method described in a previous report (Anal. Chem. 2019, 91, 20, 12724-12732), and a peak 149185 in which two alkyne groups were introduced into the antibody were confirmed.

Example 19: Synthesis of DAR=4 type ADC using a branch type reagent.

(19-1) Synthesis of branched azide reagents

(19-1-1) Synthesis of tert-butyl N-[5-(tert-butoxycarbonylamino)-1-(hydrazinecarbonyl)pentyl]carbamate

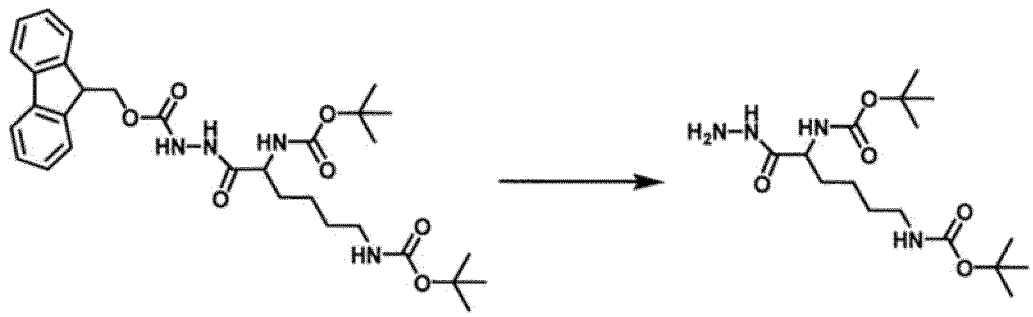

To 9H-Fluoren-9-ylmethyl N-[2,6-bis(tert-butoxycarbonylamino) hexanoylamino]carbamate (191 mg, 0.328 mmol) was added a 3 mL of 5% piperidine-containing acetonitrile and the mixture was stirred at room temperature for 3 hours. After confirming the disappearance of the raw material by TLC, the crude product obtained by concentration under reduced pressure was purified by column chromatography. The fraction containing the product was collected and concentrated under reduced pressure to give tert-Butyl N-[5-(tert-butoxycarbonylamino)-1-(hydrazinecarbonyl)pentyl]carbamate) (136mg).

MS(ESI) m/z: 361[M+H]

$^1$H NMR (400 MHz, Chloroform-d) δ=7.97 (s, 1H), 5.33 (s, 1H), 4.74 (s, 1H), 4.12-4.04 (m, 1H), 3.95 (s, 2H), 3.11 (q, J=6.6, 2H), 1.84-1.77 (m, 1H), 1.72-1.65 (m, 1H), 1.54-1.37 (m, 4H), 1.45 (s, 18H).

(19-1-2) Synthesis of tert-butyl N-[5-(tert-butoxycarbonylamino)-1-(5-sulfanyl-1,3,4-oxadiazol-2-yl)pentyl]carbamete

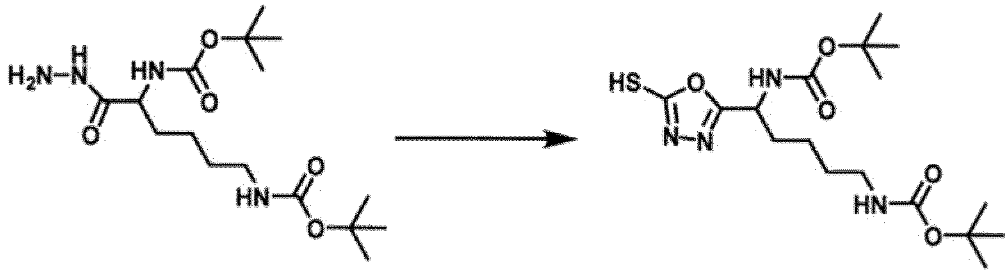

Carbon disulfide (0.034 mL, 0.567 mmol) and potassium hydroxide (66.4 mg, 1.18 mmol) were added to a solution of tert-Butyl N-[5-(tert-butoxycarbonylamino)-1-(hydrazinecarbonyl)pentyl]carbamate (136.2 mg, 0.328 mmol) in ethanol (8 mL), and the mixture was stirred at 70°C for 20 hours. The reaction was concentrated under reduced pressure with 1N hydrochloric acid, and extracted with ethyl acetate. The organic layer was then concentrated under reduced pressure and the resulting crude product was purified by column chromatography. The fraction containing the product was collected and concentrated under reduced pressure to give tert-butyl N-[5-(tert-butoxycarbonylamino)-1-(5-sulfanyl-1,3,4-oxadiazol-2-yl)pentyl]carbamete (94 mg, 0.234 mmol, 2 step yield 71%).

MS(ESI) m/z: 401[M-H]

$^1H$ NMR (400 MHz, Chloroform-d) δ = 5.14 (s, 1H), 4.74 (s, 1H), 4.55 (s, 1H), 3.06 (d, J=6.7, 2H), 1.84-1.76 (m, 2H), 1.46-1.41 (m, 2H), 1.38 (s, 18H), 1.22-1.20 (m, 2H), 0.81 (t, J=6.8, 1H).

(19-1-3) Synthesis of tert-butyl N-[5-(tert-butoxycarbonylamino)-1-(5-methylsulfanyl-1,3,4-oxadiazol-2-yl)pentyl]carbamate

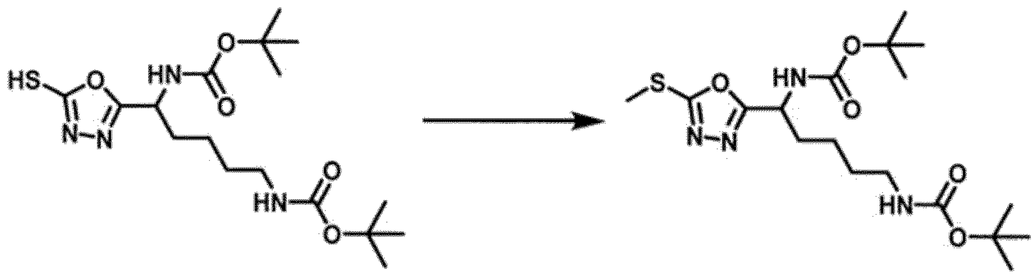

THF solutions of tert-butyl N-[5-(tert-butoxycarbonylamino)-1-(5-sulfanyl-1,3,4-oxadiazol-2-yl)pentyl]carbamete (94.1 mg, 0.234 mmol) (3 mL) were cooled to 0°C and methyl iodide (0.044 mL, 0.702 mmol) and triethylamine (0.097 mL, 0.702 mmol) were added. The mixture was returned to room temperature and the mixture was stirred, and the disappearance of the raw material was confirmed by TLC. The reaction mixture was concentrated under reduced pressure, and the obtained crude product was purified by column chromatography. The fraction containing the product was collected and concentrated under reduced pressure to give tert-butyl N-[5-(tert-butoxycarbonylamino)-1-(5-methylsulfanyl-1,3,4-oxadiazol-2-yl)pentyl]carbamate (81 mg, 0.194 mmol, yield 83%).

MS(ESI) m/z: 417[M+H]

$^1H$ NMR (400 MHz, Chloroform-d) δ=5.08 (s, 1H), 4.89 (s, 1H), 4.53 (s, 1H), 3.05 (q, J=6.6, 2H), 2.64 (s, 3H), 1.89-1.74 (m, 2H), 1.48-1.41 (m, 4H), 1.37 (d, J=2.5, 18H).

(19-1-4) Synthesis of 1-(5-methylsulfanyl-1,3,4-oxadiazol-2-yl)pentane-1,5-diamine

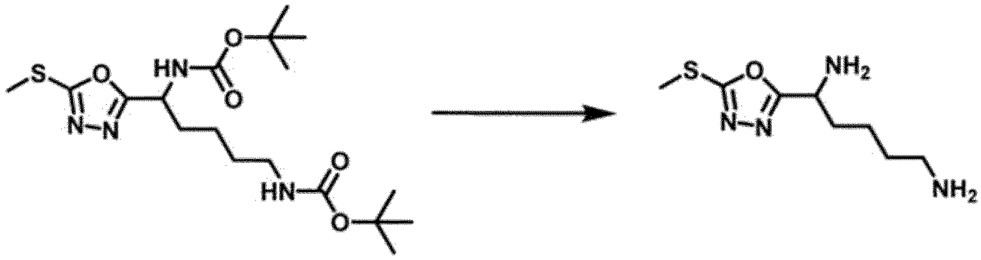

Trifluoroacetic acid (5 mL) was added to tert-butyl N-[5-(tert-butoxycarbonylamino)-1-(5-methylsulfanyl-1,3,4-oxadiazol-2-yl)pentyl]carbamate (80.9 mg, 0.194 mmol) in dichloromethane (5 mL), and the mixture was stirred at room temperature for 2 hours, and the mixture was concentrated under reduced pressure. To the obtained residue was added 1N hydrochloric acid and ethyl acetate, and the target was extracted into an aqueous layer and lyophilized to obtain 1-(5-methylsulfanyl-1,3,4-oxadiazol-2-yl)pentane-1,5-diamine) (57 mg).

MS(ESI) m/z: 217[M+H]

$^1H$ NMR (400 MHz, Methanol-d) δ=4.81 (dd, J=8.3, 5.8, 1H), 2.97 (t, J=7.7, 2H), 2.79 (s, 3H), 2.22-2.09 (m, 2H), 1.82-1.73 (m, 2H), 1.61-1.50 (m, 2H).

(19-1-5) Synthesis of 2-[2-[2-(2-azidoethoxy)ethoxy]ethoxy]-N-[5-[2-[2-[2-(2-azidoethoxy)ethoxy]ethoxy]acetyl]amino]-5-(5-methylsulfanyl-1,3,4-oxadiazol-2-yl)pentyl]acetamide)

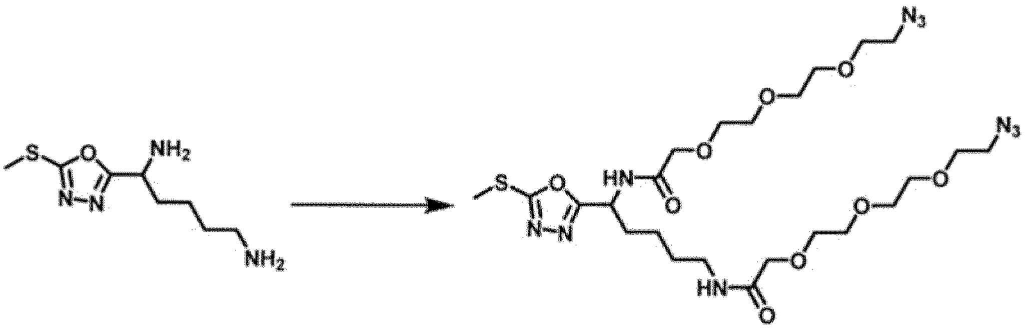

To 1-(5-methylsulfanyl-1,3,4-oxadiazol-2-yl)pentane-1,5-diamine (57.3 mg, 0.200 mmol) in DMF (3 mL) was added 1-Ethyl-3-(3-dimethylaminopropyl)carbodiimide, hydrochloride (134.2mg,0.700mmol), triethylamine (0.139 mL, 1.00 mmol), N,N-dimethylaminopyridine (4.9 mg, 0.040 mmol), 11-azido-3,6,9-trioxaundecanoic acid (102.0 mg, 0.44 mmol. After stirring at room temperature for 3 hours, the raw materials still remained, so 1-ethyl-3-(3-dimethylaminopropyl)carbodiimide, hydrochloride (40.0 mg, 0.209 mmol), triethylamine (0.070 mL, 0.505 mmol) and 11-azido-3,6,9-trioxaundecanoic acid (37.0 mg, 0.160 mmol) were added, and the mixture was stirred for 1 hour. The reaction was diluted with acetonitrile and purified by preparative HPLC to recover the fraction containing the product. By freeze-drying the water solution after excluding acetonitrile by concentrating under reduced pressure, 1-4)2-[2-[2-(2-azidoethoxy)ethoxy]ethoxy]-N-[5-[2-[2-[2-(2-azidoethoxy)ethoxy]ethoxy]acetyl]amino]-5-(5-methylsulfanyl-1,3,4-oxadiazol-2-yl)pentyl]acetamide) was obtained (48 mg,0.0734 mmol, 2 process yield 38%).

MS(ESI) m/z: 647[M+H]

$^1H$ NMR (400 MHz, Chloroform-d) δ=7.46 (d, J=9.0, 1H), 7.01 (t, J=6.1, 1H), 5.35 (td, J=8.5, 6.2, 1H), 4.07 (d, J=3.2, 2H), 4.00 (s, 2H), 3.79-3.58 (m, 20H), 3.40 (dt, J=5.7, 4.3, 4H), 3.30 (q, J=6.9, 2H), 2.72 (s, 3H), 2.15-2.01 (m, 1H), 2.10-1.84 (m, 1H), 1.68-1.53 (m, 2H), 1.53-1.35 (m, 2H).

(19-1-6) Synthesis of 2-[2-[2-(2-azidoethoxy)ethoxy]ethoxy]-N-[5-[2-[2-[2-(2-azidoethoxy)ethoxy]ethoxy]acetyl]amino]-5-(5-methylsulfonyl-1,3,4-oxadiazol-2-yl)pentyl]acetamide)

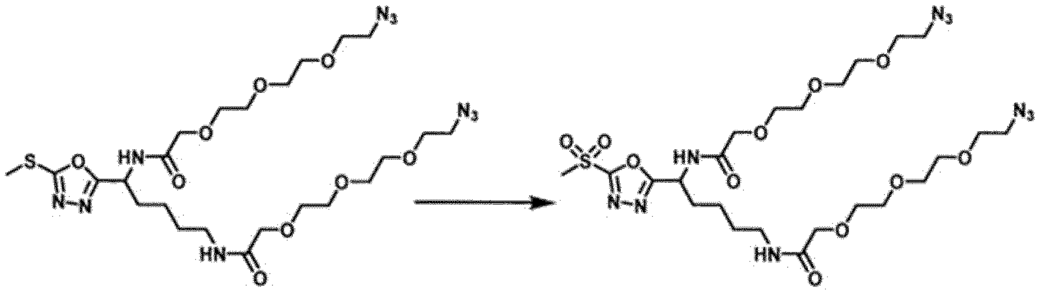

To 2-[2-[2-(2-azidoethoxy)ethoxy]ethoxy]-N-[5-[2-[2-[2-(2-azidoethoxy)ethoxy]ethoxy]acetyl]amino]-5-(5-methylsulfanyl-1,3,4-oxadiazol-2-yl)pentyl]acetamide (47.5 mg, 0.0734 mmol)) in dichloromethane solution (2 mL) was added 3-chloroprobenzoic acid (including about 30% water) (112.4 mg, 0.456 mmol). After stirring at room temperature for 5 hours, the reaction was concentrated under reduced pressure, and the resulting residue was purified by preparative HPLC. The fraction containing the product was collected, concentrated under reduced pressure to remove acetonitrile, and then the aqueous solution was lyophilized to obtain the desired product (92 mg). Repurification was carried out by column chromatography because residual impurities were still observed. The fraction containing the product was collected and concentrated under reduced pressure to give 2-[2-[2-azidoethoxy)ethoxy]ethoxy]-2-[[5-[2-[2-[2-(2-azidoethoxy)ethoxy]ethoxy]acetyl]amino)]-5-(5-methylsulfonyl-1,3,4-oxadiazol-2-yl)pentyl]acetamide (31 mg, 0.0457 mmol, yield 62%).

MS(ESI) m/z: 679[M+H]

$^1$H NMR (400 MHz, Chloroform-d) δ=7.67 (d, J=8.6, 1H), 7.04 (t, J=5.6, 1H), 5.46 (td, J=8.5, 6.2, 1H), 4.10 (d, J=5.4, 2H), 4.00 (s, 2H), 3.76-3.64 (m, 20H), 3.49 (s, 3H), 3.44-3.37 (m, 4H), 3.32 (q, J=6.8, 2H), 2.23-2.08 (m, 1H), 2.08-1.94 (m, 1H), 1.56-1.40 (m, 2H), 1.38-1.21 (m, 2H).

(19-2) Conjugation of a branch-type azide reagent with a thiol group-introduced antibody

The azide linker synthesized in Example 19-1-6 was reacted with the thiol group-introduced antibody in the same manner as described in Example 2(2).

Mass was measured by ESI-TOFMS according to the method described in the previous report (Anal. Chem., 2019, 91, 20, 12724-12732), and a peak 149620 in which four azide groups were introduced into the antibody were confirmed.

(19-3) Synthesis of ADC by click reaction

To the branched azide-introduced trastuzumab obtained in Example 19-2 according to previous reports (WO2019/240287A1 and WO2019/240288A1) and Example 15-1, a solution of 20 equivalents of DBCO-VC-PAB-MMAE (ABZENA) in DMF (5 mM) was added, and the mixture was stirred at room temperature for 20 hours, and then the product was purified using NAP-5 Columns (GE Healthcare) to obtain ADC.

Mass was measured by ESI-TOFMS according to the method described in the previous report (Anal. Chem., 2019, 91, 20, 12724-12732), and a peak 155284 in which four DBCO-VC-PAB-MMAE were introduced into the antibody was confirmed.

(19-4) Synthesis of branched protected thiol reagents (19-4-1) Synthesis of tert-Butyl(1,3-dihydroxypropan-2-yl)carbamate

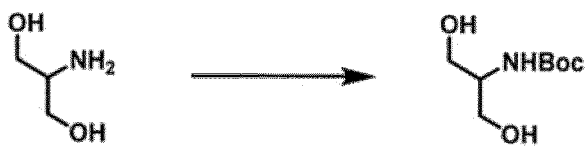

To a methanol solution (1.8 mL) of 2-Amino-1,3-propanediol (186.2 mg,2.00 mmol) was added t-butyl alcohol (1.8 mL) and a t-butyl alcohol solution (1.4 mL) of Di-tert-butyl dicarbonate (597.3 mg, 2.60 mmol) was added dropwise. After stirring at room temperature for 17 hours, the reaction was concentrated under reduced pressure. Hexane (4 mL)

was added to the obtained crude product to precipitate white solid. The filtrate was washed with hexane (3 mL) and dried under reduced pressure to give a tert-Butyl (1,3-dihydroxypropan-2-yl)carbamate (352.9 mg, 1.845 mmol, yield 92 %) .

MS(ESI) m/z: 213[M+Na]

$^1$H NMR (400 MHz, Chloroform-d) δ=5.22 (s, 1H), 3.84 (qd, J=11.0, 4.3, 4H), 3.71 (s, 1H), 1.48 (s, 9H)

(19-4-2) Synthesis of tert-Butyl N-[2-allyoxy-1-(allyloxymethyl) ethyl] carbamete

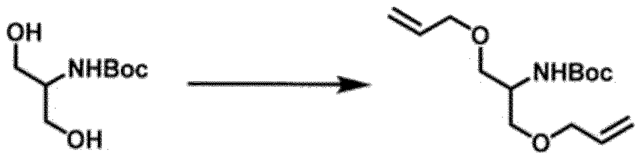

Allyl bromide (0.547 mL, 6.34 mmol) was added to DMF solutions (4 mL) of tert-Butyl (1,3-dihydroxypropan-2-yl)carbamate (324.9 mg,1.70 mmol) and potassium hydroxide (359.0 mg, 6.40 mmol) was added in portions. After stirring at room temperature for 4 hours, the reaction solution was concentrated under reduced pressure and extracted with dichloromethane and water. The crude product obtained by washing the organic layer with saturated brine and concentrating under reduced pressure was purified by column chromatography. The fraction containing the product was collected and concentrated under reduced pressure to give a tert-Butyl N-[2-allyoxy-1-(allyloxymethyl)ethyl]carbamete (326.6 mg, 1.20 mmol, yield 71%).

MS(ESI) m/z: 294[M+Na]

$^1$H NMR (400 MHz, Chloroform-d) δ=5.95-5.86 (m, 2H), 5.29(dq, J=17.2, 1.7, 2H), 5.20 (dq, J=10.4, 1.4, 2H), 4.94 (s, 1H) 4.01 (dt, J=5.5, 1.5, 4H), 3.91 (s, 1H), 3.58 (dd, J=9.4, 4.3, 2H), 3.51 (dd, J=9.4, 6.0, 2H), 1.47 (s, 9H).

(19-4-3) Synthesis of S-[3-[3-acetylsulfanylpropoxy]-2-(tert-butoxycarbonylamino)propoxy]propyl]ethanethioate)

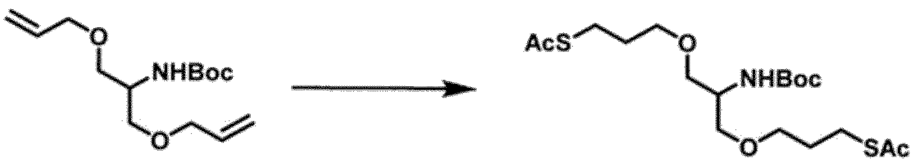

To a solution of tert-Butyl N-[2-allyoxy-1-(allyloxymethyl)ethyl] carbamete (323.8 mg, 1.19 mmol) in toluene (7.4 mL) was added 2,2'-azobisisobutyronitrile (1.993 g, 25.66 mmol). To the suspension was added thioacetic acid (0.890 mL, 11.88 mmol) in five portions and the mixture was stirred at 65°C for 21 hours. The crude product obtained by returning the reaction to room temperature and concentrating under reduced pressure was purified by column chromatography. The fraction containing the product was collected and concentrated under reduced pressure to give S-[3-[3-acetylsulfanylpropoxy]-2-(tert-butoxycarbonylamino)propoxy] propyl]ethanethioate (362.0 mg, 0.854 mmol, yield 72%).

MS(ESI) m/z: 446[M+Na]

$^1$H NMR (400 MHz, Chloroform-d) δ=4.95 (s, 1H), 3.87 (s, 1H), 3.55-3.44 (m, 8H), 3.03-2.89 (m, 4H), 2.35 (s, 6H), 1.93-1.76 (m, 4H), 1.47 (s, 9H).

(19-4-4) Synthesis of S-[3-[3-(3-acetylsulfanylpropoxy)-2-amino-propoxy]propyl]ethanethioate)

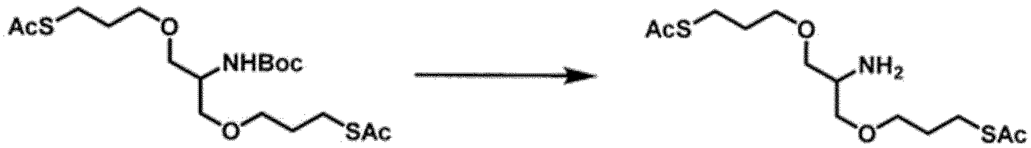

To S-[3-[3-acetylsulfanylpropoxy]-2-(tert-butoxycarbonylamino) propoxy]propyl]ethanethioate (360.1 mg, 0.850 mmol) in dichloromethane (2 mL) was added trifluoroacetic acid (2 mL) and the mixture was stirred at room temperature for 1 hour. 0.1 N hydrochloric acid (10 mL) was added to the residue obtained by concentrating the reactant under reduced pressure, and the aqueous solution obtained by concentrating under reduced pressure was lyophilized to obtain S-[3-[3-(3-acetylsulfanylpropoxy)-2-amino-propoxy]propyl]ethanethioate hydrochloride salt (384.4 mg).
MS(ESI) m/z: 324[M+H]
$^1$H NMR (400 MHz, Chloroform-d) δ=4.51 (s, 2H), 3.82-3.64 (m, 5H), 3.64-3.45 (m, 4H), 3.10-2.92 (m, 4H), 2.37 (s, 6H), 1.95-1.81 (m, 4H).

(19-4-5) Synthesis of S-[3-[3-(3-acetylsulfanylpropoxy)-2-[3-(2,5-dioxoppyrrol-1-yl)propanoylamino]propoxy]propyl]ethanethioate

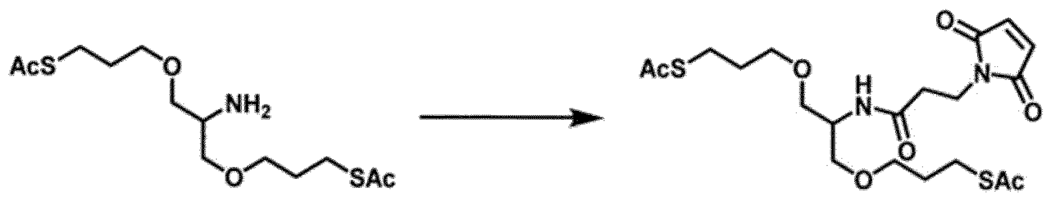

To 3-maleimidopropionic acid (17.6 mg, 0.10 mmol) in THF (1.5 mL) was added o-Benzotriazol-1-yl-tetramethyluronium hexafluorophosphate (56.9 mg, 0.15 mmol, triethylamine (0.021 mL, 0.15 mmol) and a solution of S-[3-[3-(3-acetylsulfanylpropoxy)-2-amino-propoxy]propyl]ethanethioate (53.2 mg, 0.11 mmol) in THF (0.5 mL). After stirring at room temperature for 2 hours, the reaction was concentrated under reduced pressure, and the obtained crude product was purified by column chromatography. The fraction containing the product was collected and concentrated under reduced pressure to give S-[3-[3-(3-acetylsulfanylpropoxy)-2-[3-(2,5-dioxoppyrrol-1-yl)propanoylamino]propoxy]propyl]ethanethioate) (18.7 mg, 0.039 mmol, yield 41%).
MS(ESI) m/z: 475[M+H]
$^1$H NMR (400 MHz, Chloroform-d) δ=6.72 (s, 2H), 6.43 (s, 1H), 4.18 (s, 1H), 3.88 (t, J=7.0, 2H), 3.59-3.38 (m, 8H), 3.07-2.88 (m, 4H), 2.61 (t, J=7.1, 2H) 2.36 (s, 6H), 1.88-1.84 (m, 4H).

(19-5) Conjugation of a branch-type protected thiol reagent with a thiol group-introduced antibody

The branched linker synthesized in Example 19-4-5 was reacted with the thiol group-introduced antibody in the same manner as described in Example 2(2).
Mass was measured by ESI-TOFMS according to the method described in the previous report (Anal. Chem., 2019, 91, 20, 12724-12732), and a peak 149392 in which four protected thiol groups were introduced into the antibody were confirmed.

(19-6) Preparation of branched thiol groups-introduced antibody derivatives by cleavage of thioester groups

To the antibody-intermediates obtained in (19-5), hydroxylamine solutions were added according to WO2019/240287A1 and allowed to stand at room temperature for 1 hour. After 2 hours, the compound was replaced with 20 mM PBS buffer, 10 mM EDTA (pH 7.4) to obtain a thiol-group-introduced antibody derivative. Mass was determined by ESI-TOFMS. As a result, a peak at 149231 where deprotection proceeded was confirmed.

(19-7) Synthesis of ADC mimic

(19-7-1) Synthesis of Maleimide-VC-Pyrene

Maleimide-VC-Pyrene was synthesized as follows. Commercially available MC-VC-PAB-PNP (CAS No: 159857-81-5) and known Sarcosine-pyrene (WO2018/218004A1) were synthesized in one step.

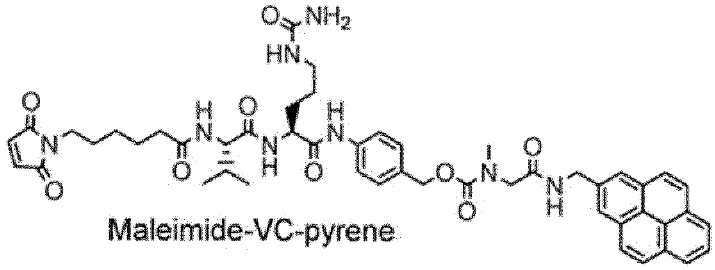

Maleimide-VC-pyrene

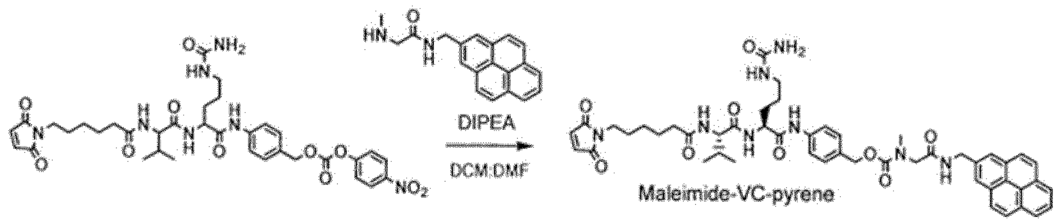


Commercially available MC-VC-PAB-PNP (CAS No: 159857-81-5)(15.5 mg, 0.021 mmol) was dissolved in dichloromethane (1 mL) and N,N-diisopropylethylamine (0.025 mL, 0.142 mmol), a known Sarcosine-pyrene (WO2018/218004A1) (7.6 mg, 0.025 mmol) in dimethylformamide (0.5 mL) was added and the mixture was stirred for 17 h. After purification by reverse phase preparative chromatography, the fraction containing the product was collected, and the acetonitrile was removed by concentration under reduced pressure, and lyophilization was performed to obtain Maleimide-VC-Pyrene (7.3 mg, 0.008 mmol) .

$^1$H NMR (400 MHz, DMSO-d6) δ9.98 (s, 1H), 8.34 (d, J=9.2Hz, 2H), 8.32-8.23 (m, 4H), 8.16 (s, 2H), 8.10-8.00 (m, 4H), 7.80 (d, J=8.8Hz, 1H), 7.59 (d, J=8.4Hz, 2H), 7.31 (d, J=8.0Hz, 2H), 6.99 (s, 2H), 5.96 (m, 1H), 5.40 (s, 2H), 5.01 (s, 2H), 4.95 (d, J=6.0Hz, 2H), 4.38 (m, 1H), 4.19 (m, 1H), 3.03-2.92 (m, 3H), 2.67 (m, 1H), 2.33 (m, 1H), 2.20-2.07 (m, 2H), 1.97 (m, 1H), 1.67 (m, 1H), 1.59 (m, 1H), 1.51-1.45 (m, 6H), 1.26-1.15 (m, 3H), 0.83 (dd, J=12.8, 6.8Hz, 6H)

MS(ESI) m/z: 901.45[M+H]$^+$

(19-7-2) Synthesis of ADC mimic

To a solution (20 μM) of the thiol-group-introduced antibody obtained in Example 19-6 in buffer(pH 7.4, PBS buffer) was added a solution of 10 equivalents of Maleimide-VC-Pyrene synthesized in Example 19-7-1 in DMF (1.25 mM), and the mixture was allowed to stand at room temperature for 2 hours, and then purified using NAP-5 Columns (GE Healthcare) to obtain a ADC mimic.

Mass was measured by ESI-TOFMS according to the method described in the previous report (Anal. Chem., 2019, 91, 20, 12724-12732), and a peak 152843 in which four Maleimide-VC-Pyrene were introduced into the antibody was confirmed.

[Sequence Listing]

## Claims

**1.** An antibody composition comprising

(A) an antibody intermediate or a salt thereof having a bioorthogonal functional group which may be protected, which is represented by the following formula (A):

$$Ab\text{—}[S\text{—}L\text{—}R]_n \qquad (A)$$

wherein

Ab is an antibody comprising an immunoglobulin unit comprising two heavy chains and two light chains

and having a disulfide bond between the heavy chains and between the heavy and light chains,
S is a sulfur atom,
L is a divalent group,
R is a bioorthogonal functional group which may be protected, and
n is an integer from 1 to 8, and

(B) a thiol group-introduced antibody or a salt thereof, which is represented by the following formula (B):

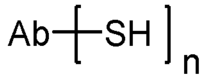

(B)

wherein

Ab is the same antibody as the antibody in formula (A),
SH is a thiol group, and
n is the same integer as n in formula (A),
wherein the sulfur atom (S) adjacent to the antibody (Ab) in formula (A) and the thiol group (SH) adjacent to the antibody (Ab) in formula (B) are directly bonded or bonded via a linker to the atom in the side chain of the same amino acid residue present at the same position in the constant region of the antibody heavy chain,
wherein the partial structure represented by L-R has a molecular weight of 700 or less,
wherein the percentage of the amount of the antibody intermediate or salt thereof relative to the total amount of the antibody intermediate or salt thereof and the thiol group-introduced antibody or salt thereof [100 (%) × (the amount of the antibody intermediate or salt thereof)/(the total amount of the antibody intermediate or salt thereof and the thiol group-introduced antibody or salt thereof) ] is 80% or more,
wherein the antibody intermediate or salt thereof and the thiol group-introduced antibody or salt thereof have an aggregation rate of 5% or less.

**2.** The antibody composition of claim 1, wherein the antibody intermediate is represented by the following formula (I'), (II'), (III'), or (IV'):

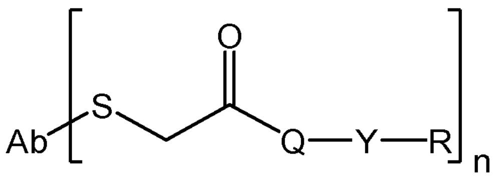

(I')

wherein

Ab is an antibody comprising an immunoglobulin unit comprising two heavy chains and two light chains and having a disulfide bond between the heavy chains and the light chains,
S is a sulfur atom,
Q is arylene or alkylene,
Y is a bond or a divalent group,
R is a bioorthogonal functional group which may be protected, and
n is an integer from 1 to 8,

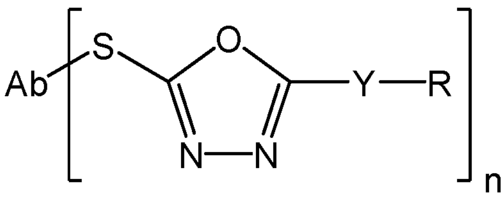

(II')

wherein

Ab is an antibody comprising an immunoglobulin unit comprising two heavy chains and two light chains and having a disulfide bond between the heavy chains and the light chains,
S is a sulfur atom,
Y is a bond or a divalent group,
R is a bioorthogonal functional group which may be protected, and
n is an integer from 1 to 8,

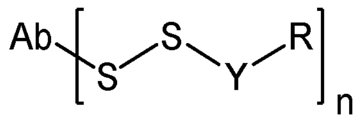
(III')

wherein
Ab is an antibody comprising an immunoglobulin unit comprising two heavy chains and two light chains and having a disulfide bond between the heavy chains and the light chains,
S is a sulfur atom,
Y is a bond or a divalent group,
R is a bioorthogonal functional group which may be protected, and
n is an integer from 1 to 8, or

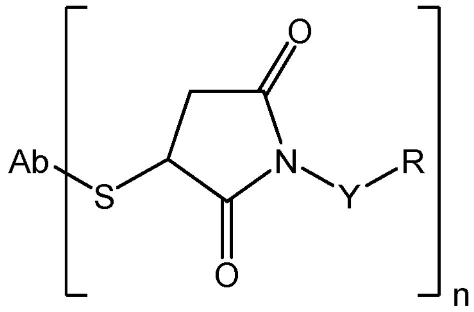
(IV')

wherein
Ab is an antibody comprising an immunoglobulin unit comprising two heavy chains and two light chains and having a disulfide bond between the heavy chains and the light chains,
S is a sulfur atom,
Y is a bond or a divalent group,
R is a bioorthogonal functional group which may be protected, and
n is an integer from 1 to 8.

**3.** The antibody composition of claim 1 or 2, wherein the amino acid residue is an amino acid residue other than a cysteine residue.

**4.** The antibody composition of any one of claims 1 to 3, wherein the sulfur atom (S) adjacent to the antibody (Ab) in formula (A) and the thiol group (SH) adjacent to the antibody (Ab) in formula (B) are regioselectively bonded to a nitrogen atom in a side chain of a lysine residue present at the same position in the constant region of the heavy chain of the antibody (Ab) via a linker which does not contain a peptide.

**5.** The antibody composition of any one of claims 1 to 4, wherein the antibody is an IgG antibody.

**6.** The antibody composition of claim 4 or 5, wherein the lysine residue is present at one or more positions selected from the group consisting of the positions 246/248, 288/290, and 317 of a heavy chain of a human IgG of EU numbering.

**7.** The antibody composition of any one of claims 1 to 6, wherein the antibody composition is selected from the group consisting of antibody intermediates represented by the following formulae (1')-(12'):

(1')

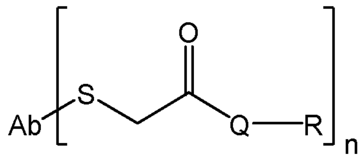

(2')

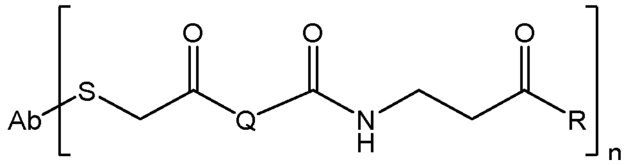

(3')

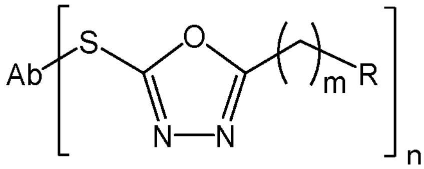

(4')

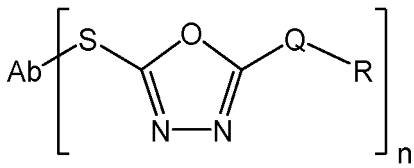

(5')

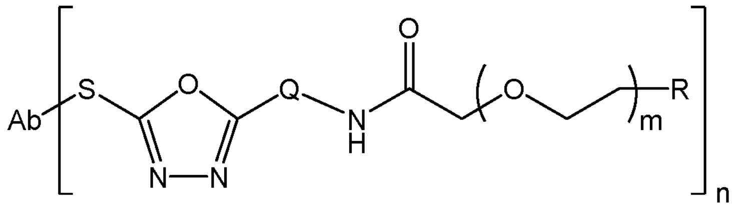

(6')

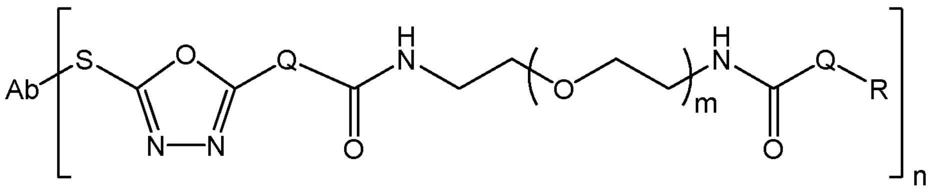

(7')

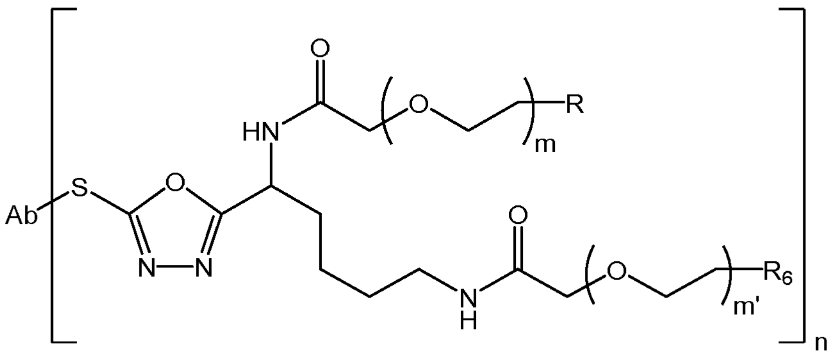

(8') 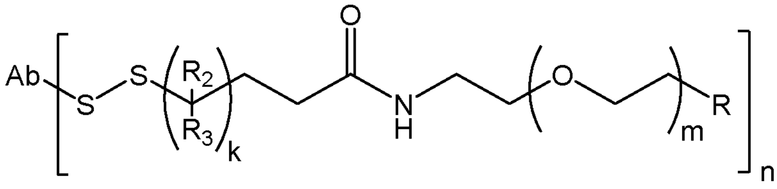

(9') 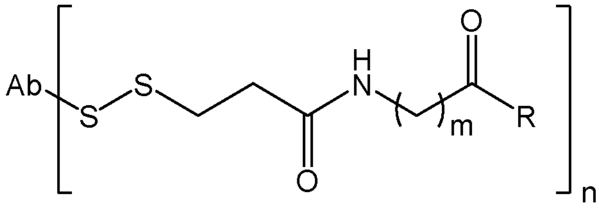

(10') 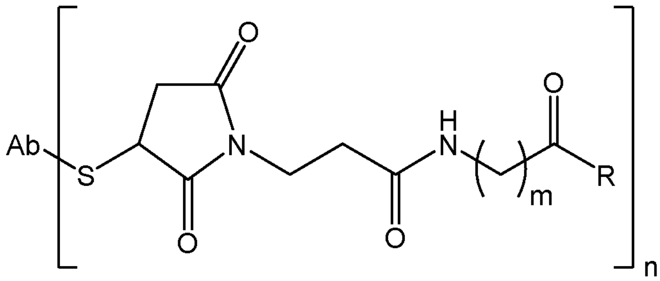

(11') 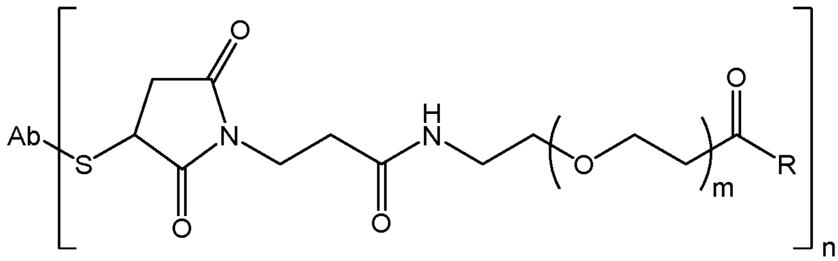

(12') 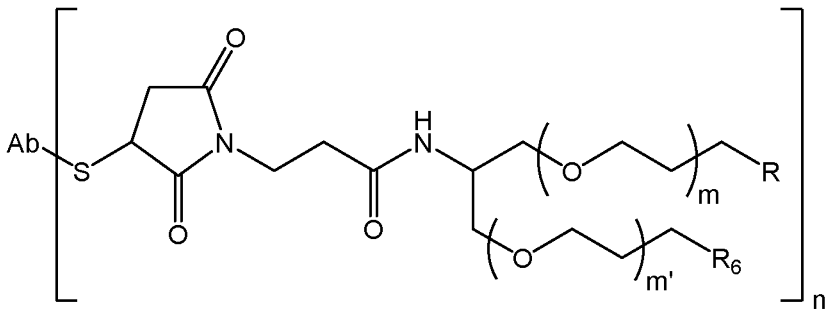

wherein

k is an integer of 0 or 1,
m is an integer from 1 to 5,
m' is an integer from 1 to 5,
$R_2$ and $R_3$ are each independently a hydrogen atom or methyl,
$R_6$ is a bioorthogonal functional group which may be protected, and
Ab, S adjacent to Ab, Q, R and n are the same as defined in claim 2.

**8.** The antibody composition of any one of claims 1 to 7, wherein the bioorthogonal functional group which may be protected is an alkyne residue or azide.

**9.** The antibody composition of claim 8, wherein the alkyne residue is a ring group having a triple bond between carbon atoms, which may be substituted.

**10.** A reagent for derivatizing a thiol group-introduced antibody or a salt thereof, which comprises a compound represented by the following formula (I), (II), (III), or (IV) :

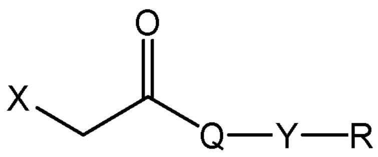

(I)

wherein

X is a halogen atom,
Q is arylene or alkylene,
Y is a bond or a divalent group, and
R is a bioorthogonal functional group which may be protected,

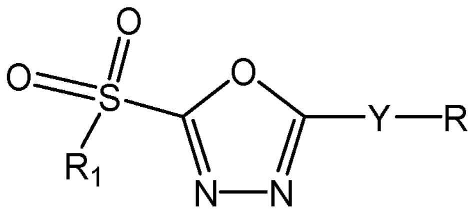

(II)

wherein
$R_1$ is alkyl,
Y is a divalent group, and
R is a bioorthogonal functional group which may be protected,

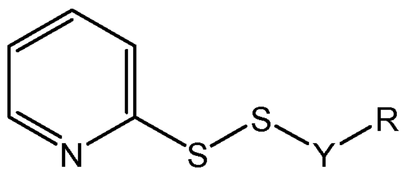

(III)

wherein,
Y is a divalent group, and
R is a bioorthogonal functional group which may be protected, or

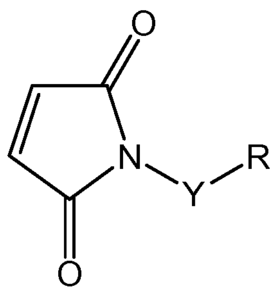

(IV)

wherein
Y is a divalent group, and
R is a bioorthogonal functional group which may be protected.

**11.** The reagent of claim 10, wherein the reagent is a reagent for derivatizing the thiol group-introduced antibody or salt thereof by reacting with a thiol group introduced via a side chain of an amino acid residue other than a cysteine residue in a thiol group-introduced antibody or a salt thereof.

**12.** The reagent of claim 10 or 11, wherein the thiol group in the thiol group-introduced antibody is regioselectively bonded to a nitrogen atom in a side chain of a lysine residue in a constant region of a heavy chain in the antibody via a linker which does not contain a peptide.

**13.** The reagent of any one of claims 10 to 12, wherein the thiol group-introduced antibody is an IgG antibody.

**14.** The reagent of claim 12 or 13, wherein the lysine residue is present in one or more positions selected from the group consisting of the positions 246/248, 288/290, and 317 of a heavy chain of a human IgG of EU numbering.

**15.** The reagent of any one of claims 10 to 14, wherein the divalent group is one group selected from the group consisting of alkylene, arylene, -C(=O)-, $-NR_2-$, $-C(=O)-NR_2-$, $-NR_2-C(=O)-$, -O-, and $-(O-R_3)_m$, or

the divalent group is a divalent group in which two or more groups selected from the group consisting of alkylene, arylene, -C(=O)-, $-NR_2-$, $-C(=O)-NR_2-$, $-NR_2-C(=O)-$, -O-, and $-(O-R_3)_m$ are linked,
$R_2$ is a hydrogen atom or alkyl,
$R_3$ is alkylene or arylene, and
m is an integer from 1 to 5.

**16.** The reagent of any one of claims 10 to 15, wherein the reagent is selected from the group consisting of compounds represented by the following formulae (1)-(12):

(1) 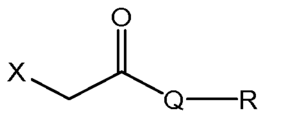

(2) 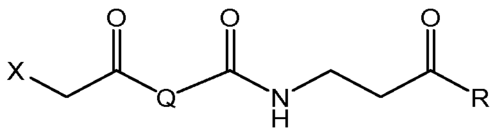

(3) 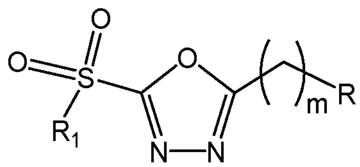

(4) 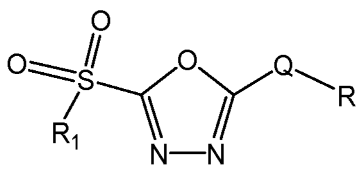

(5) 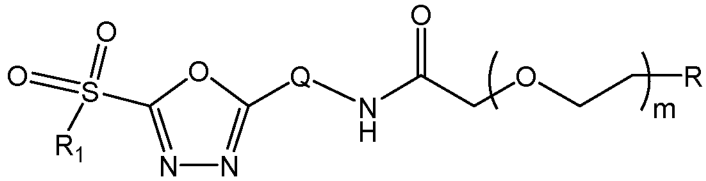

(6) 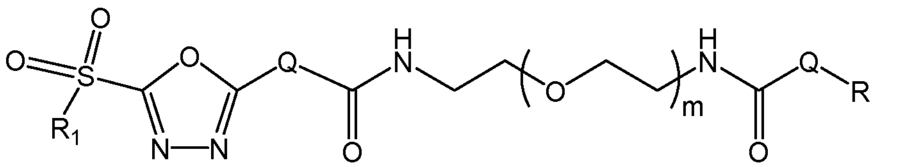

(7) 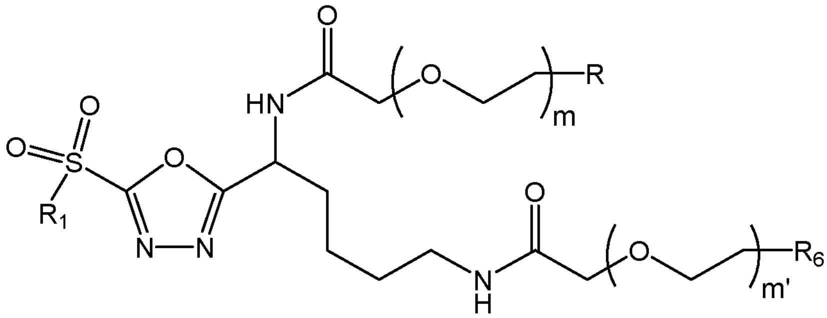

(8) 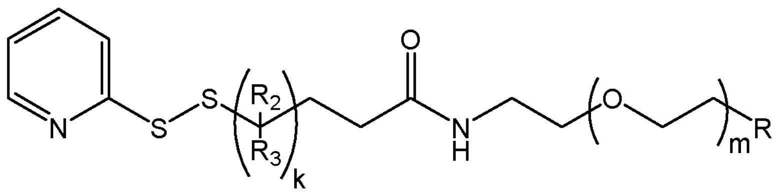

(9) 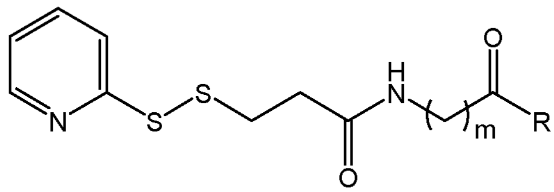

(10) 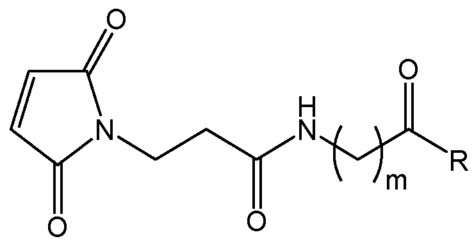

(11) 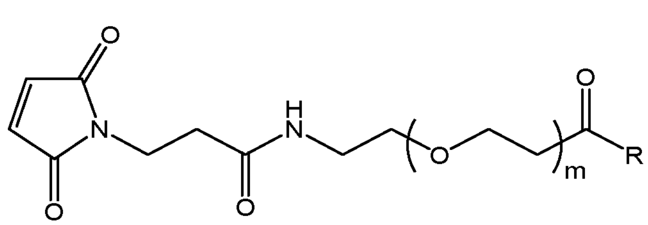

(12) 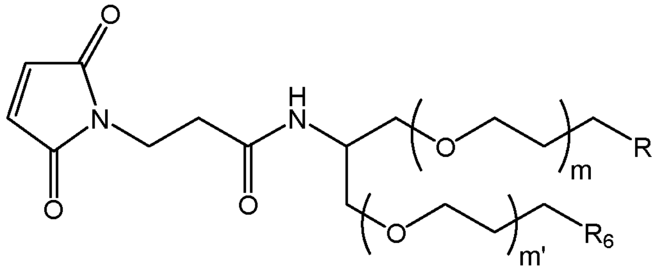

wherein

k is an integer of 0 or 1,
m is an integer from 1 to 5,
m' is an integer from 1 to 5,
$R_2$ and $R_3$ are each independently a hydrogen atom or methyl,
$R_6$ is a bioorthogonal functional group which may be protected, and
X, $R_1$, Q and R are the same as defined in claim 10.

**17.** A compound or a salt thereof, wherein the compound is represented by the following formula (I), (II), (III), or (IV) :

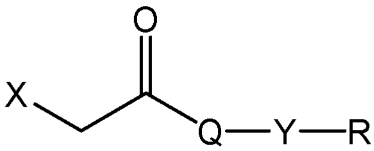 (I)

wherein

X is a halogen atom,
Q is arylene or alkylene,
Y is a divalent group, and
R is a bioorthogonal functional group which may be protected,

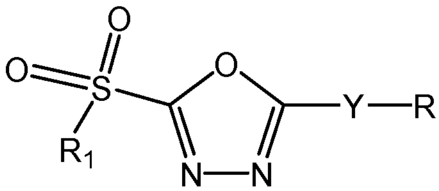 (II)

wherein
$R_1$ is alkyl,
Y is a divalent group, and
R is a bioorthogonal functional group which may be protected,

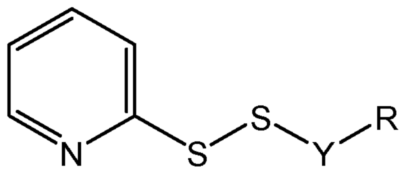 (III)

wherein
Y is a divalent group, and
R is a bioorthogonal functional group which may be protected, or

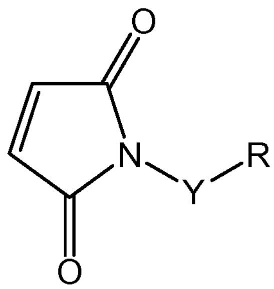

(IV)

wherein
Y is a divalent group, and
R is a bioorthogonal functional group which may be protected.

**18.** The compound or salt thereof of claim 17, wherein the compound is selected from the group consisiting of the compounds represented by the following formulae (2)-(12):

(2) 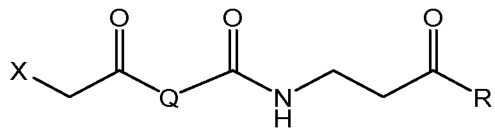

(3) 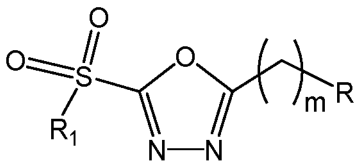

(4) 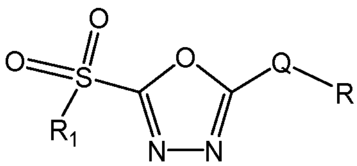

(5) 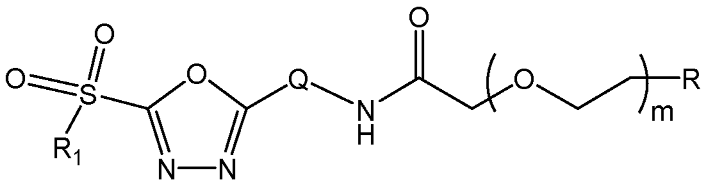

(6) 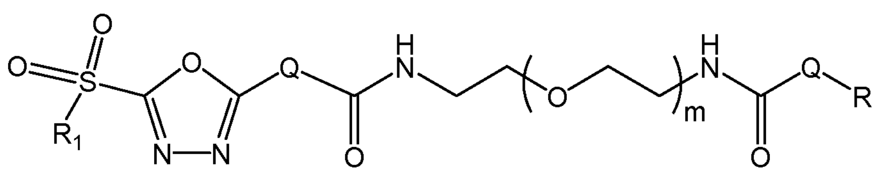

(7) 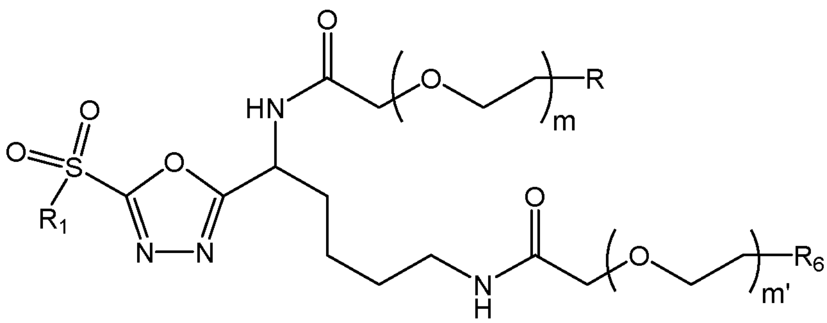

(8) 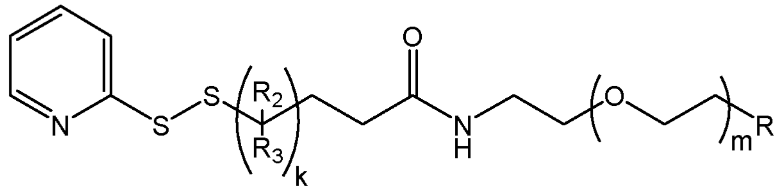

(9) 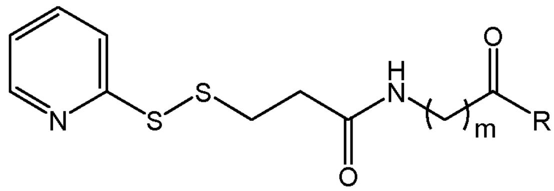

(10) 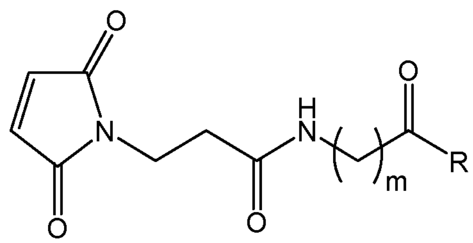

(11) 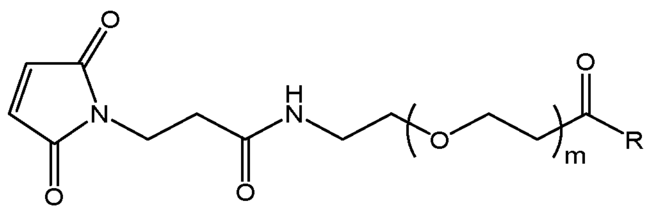

(12) 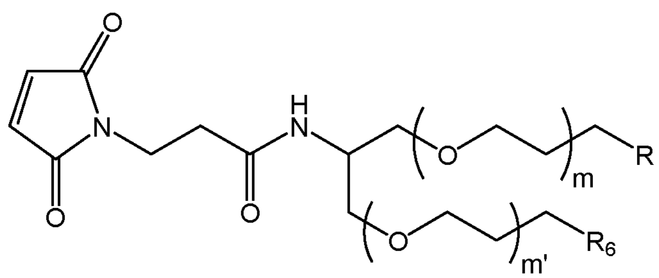

wherein

k is an integer of 0 or 1,

m is an integer from 1 to 5,
m' is an integer from 1 to 5,
$R_2$ and $R_3$ are each independently a hydrogen atom or methyl,
$R_6$ is a bioorthogonal functional group which may be protected, and
X, $R_1$, Q and R are the same as defined in claim 17.

**19.** An antibody intermediate or salt thereof having a bioorthogonal functional group which may be protected, wherein the antibody intermediate is represented by the following formula (I'), (II'), (III'), or (IV'):

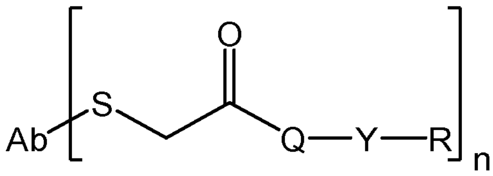

(I')

wherein

Ab is an antibody,
S is a sulfur atom,
Q is arylene or alkylene,
Y is a bond or a divalent group,
R is a bioorthogonal functional group which may be protected, and
n is an integer from 1 to 8,

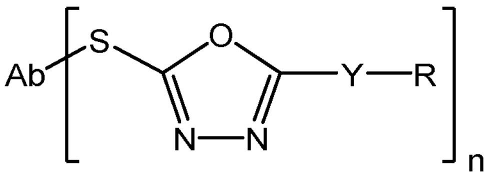

(II')

wherein
Ab is an antibody,
S is a sulfur atom,
Y is a bond or a divalent group,
R is a bioorthogonal functional group which may be protected, and
n is an integer from 1 to 8,

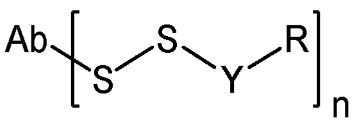

(III')

wherein
Ab is an antibody,
S is a sulfur atom,
Y is a bond or a divalent group,
R is a bioorthogonal functional group which may be protected, and
n is an integer from 1 to 8, or

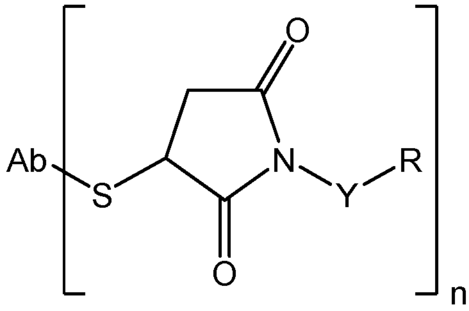

(IV')

wherein
Ab is an antibody,
S is a sulfur atom,
Y is a bond or a divalent group,
R is a bioorthogonal functional group which may be protected, and
n is an integer from 1 to 8.

**20.** The antibody intermediate or salt thereof of claim 19, wherein the sulfur atom (S) adjacent to the antibody (Ab) in formula (I'), (II'), (III'), or (IV') is directly bonded or bonded via a linker to an atom in a side chain of an amino acid residue other than a cysteine residue in a constant region of a heavy chain of the antibody (Ab).

**21.** The antibody intermediate or salt thereof of claim 20, wherein the sulfur atom (S) adjacent to the antibody (Ab) in formula (I'), (II'), (III'), or (IV') is regioselectively bonded to a nitrogen atom in a side chain of a lysine residue in the constant region of the heavy chain of the antibody (Ab) via a linker which does not contain a peptide.

**22.** The antibody intermediate or salt thereof of any one of claims 19-21, wherein the antibody intermediate is an IgG antibody.

**23.** The antibody intermediate or salt thereof of claim 21 or 22, wherein the lysine residue is present at one or more positions selected from the group consisting of the positions 246/248, 288/290, and 317 of a heavy chain in a human IgG of EU numbering.

**24.** The antibody intermediate or salt thereof of any one of claims 19-23, wherein the antibody intermediate is selected from the group consisting of antibody intermediates represented by the following formulae (1')-(12'):

(1')

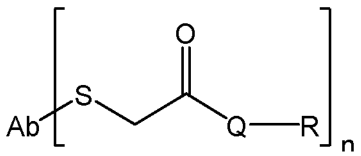

(2')

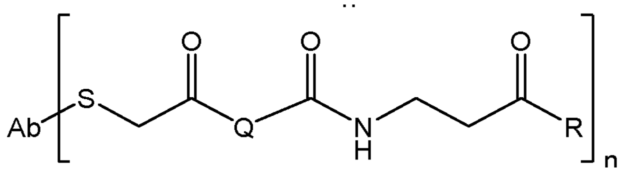

(3')

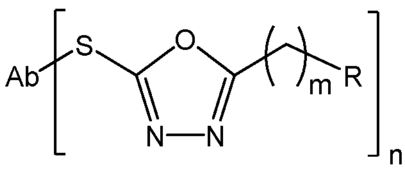

(4')

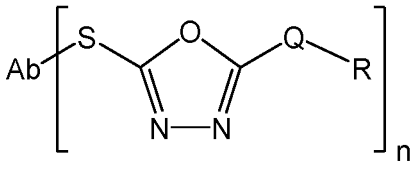

(5')

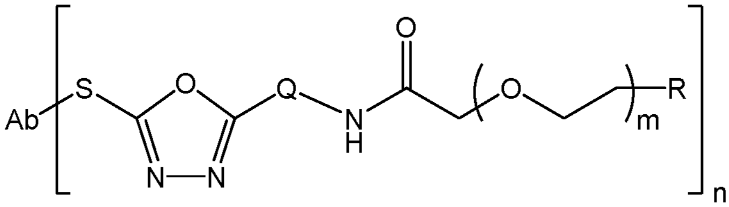

(6')

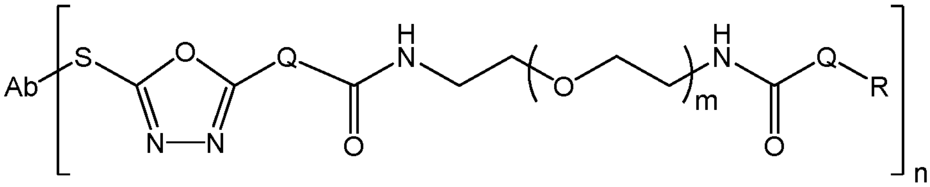

(7')

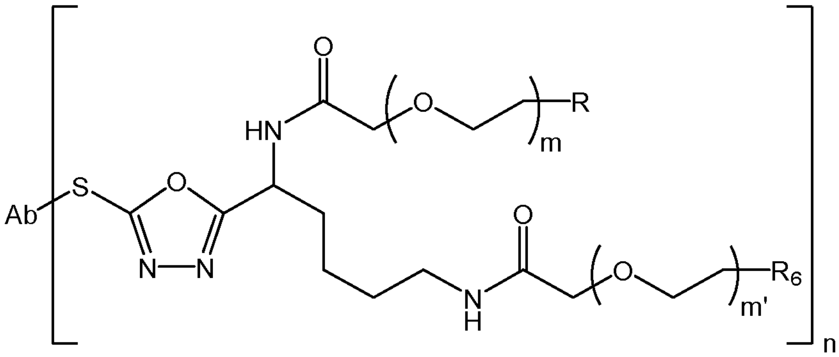

(8')

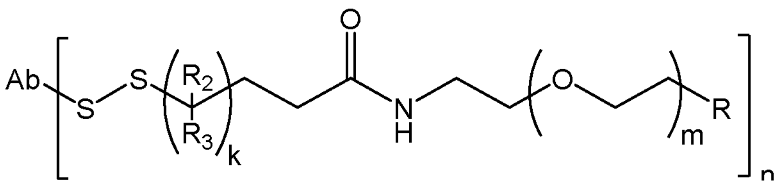

(9')

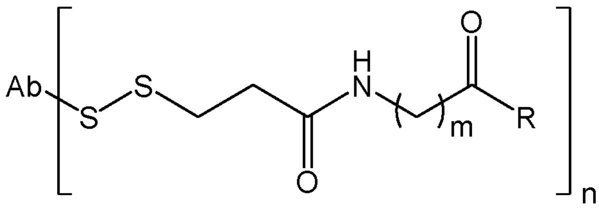

(10') 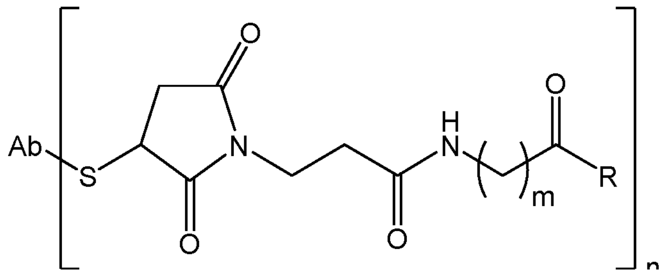

(11') 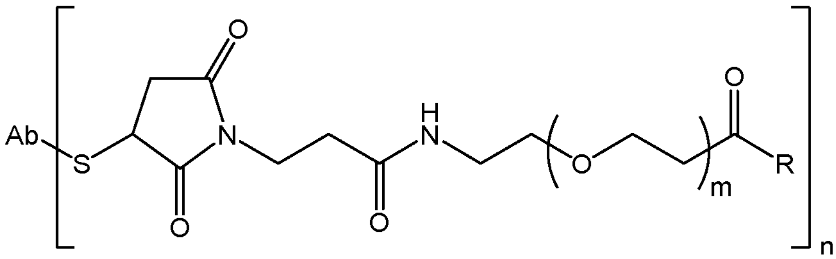

(12') 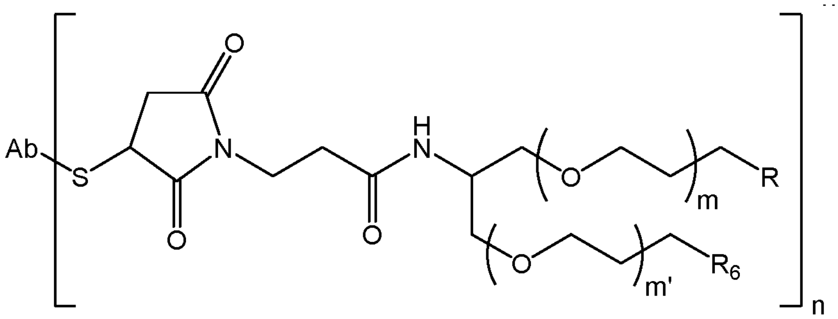

wherein

k is an integer of 0 or 1,
m is an integer from 1 to 5,
m' is an integer from 1 to 5,
$R_2$ and $R_3$ are each independently a hydrogen atom or methyl,
$R_6$ is a bioorthogonal functional group which may be protected, and
Ab, S adjacent to Ab, Q, R and n are the same as defined in claim 18.

**25.** A method for producing an antibody intermediate or a salt thereof having a bioorthogonal functional group which may be protected, comprising reacting a compound represented by the following formula (I), (II), (III), or (IV) :

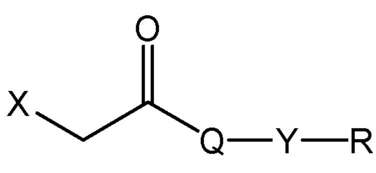 (I)

wherein

X is a halogen atom,
Q is arylene or alkylene,
Y is a bond or a divalent group, and
R is a bioorthogonal functional group which may be protected,

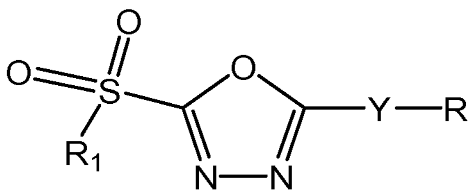

(II)

wherein
$R_1$ is alkyl,
Y is a divalent group, and
R is a bioorthogonal functional group which may be protected,

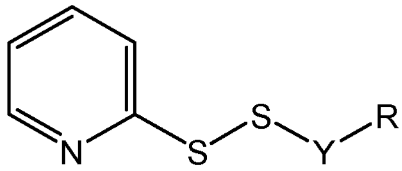

(III)

wherein
Y is a divalent group, and
R is a bioorthogonal functional group which may be protected, or

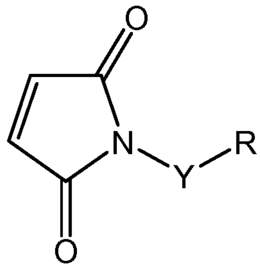

(IV)

wherein
Y is a divalent group, and
R is a bioorthogonal functional group which may be protected, or
a salt thereof with a thiol group-introduced antibody or a salt thereof to give the antibody intermediate or salt thereof having a bioorthogonal functional group which may be protected, which is represented by the following formula (I'), (II'), (III'), or (IV'):

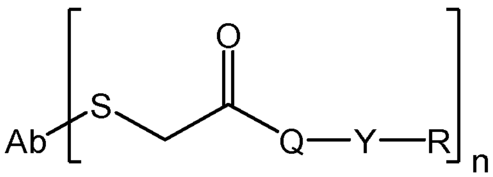

(I')

wherein
Ab is an antibody,
S is a sulfur atom,
n is an integer from 1 to 8,
Q, Y and R are the same as those of Formula (I),

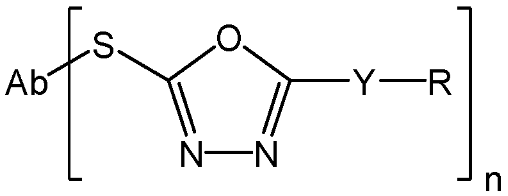

(II')

wherein
Ab is an antibody,
S is a sulfur atom,
n is an integer from 1 to 8,
Y and R are the same as those of Formula (II),

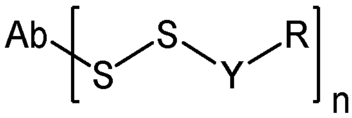

(III')

wherein,
Ab is an antibody,
S is a sulfur atom,
n is an integer from 1 to 8,
Y and R are the same as those of Formula (III), or

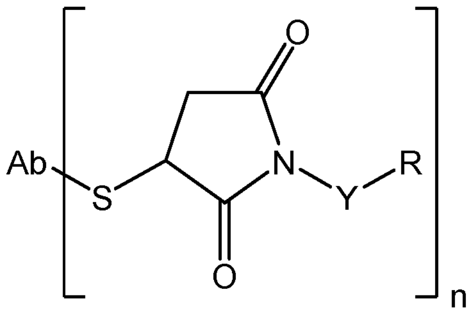

(IV')

wherein
Ab is an antibody,
S is a sulfur atom,
n is an integer from 1 to 8,
Y and R are the same as those of formula (IV).

**26.** A conjugate of an antibody and a functional substance or a salt thereof, which is represented by the following formula (I"), (II"), (III"), or (IV") :

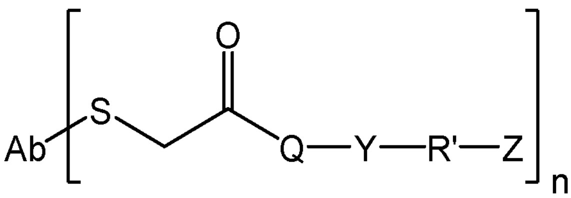

(I")

wherein

Ab is an antibody,
S is a sulfur atom,
Q is arylene or alkylene,

Y is a bond or a divalent group,
R' is a group produced by the reaction between two bioorthogonal functional groups capable of reacting with each other,
Z is a functional substance, and
n is an integer from 1 to 8,

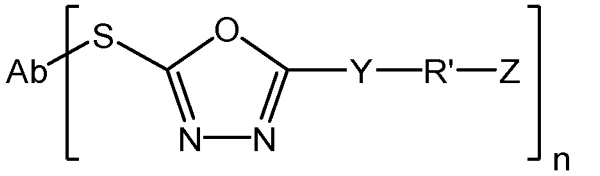

(II")

wherein
Ab is an antibody,
S is a sulfur atom,
Y is a bond or a divalent group,
R' is a group produced by the reaction between two bioorthogonal functional groups capable of reacting with each other,
Z is a functional substance, and
n is an integer from 1 to 8,

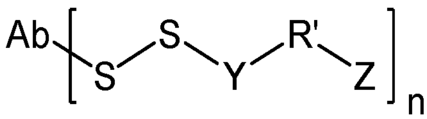

(III")

wherein
Ab is an antibody,
S is a sulfur atom,
Y is a bond or a divalent group,
R' is a group produced by the reaction between two bioorthogonal functional groups capable of reacting with each other,
Z is a functional substance, and
n is an integer from 1 to 8, or

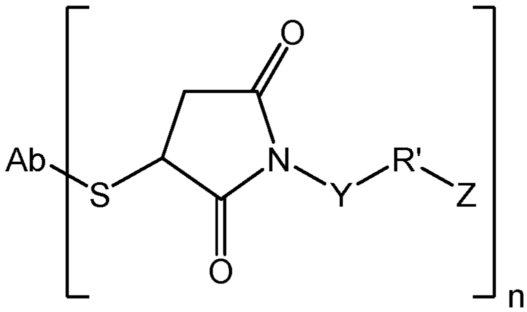

(IV")

wherein
Ab is an antibody,
S is a sulfur atom,
Y is a bond or a divalent group,
R' is a group produced by the reaction between two bioorthogonal functional groups capable of reacting with each other,
Z is a functional substance, and
n is an integer from 1 to 8.

**27.** The conjugate or salt thereof of claim 26, wherein the conjugate is selected from the group consisting of conjugates represented by the following formulae (1")-(12") :

(1'')

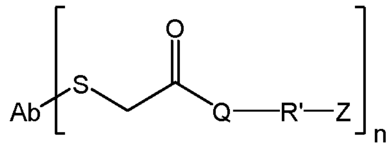

(2'')

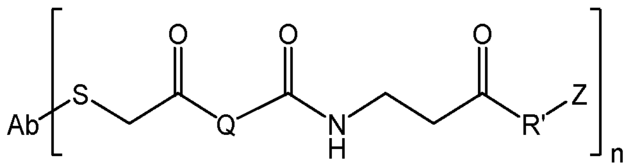

(3'')

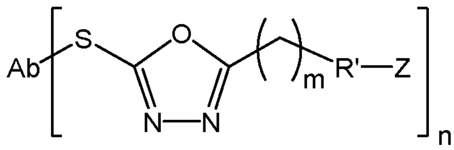

(4'')

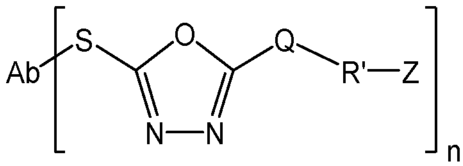

(5'')

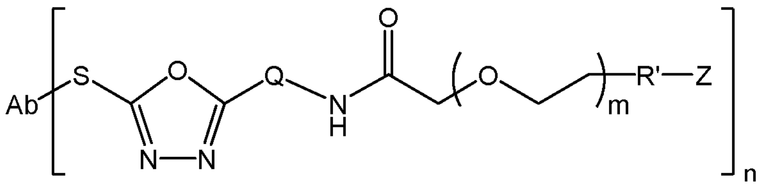

(6'')

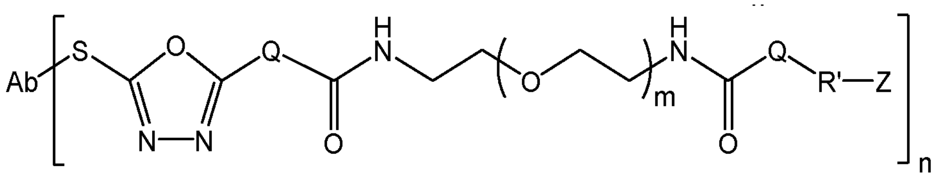

(7'')

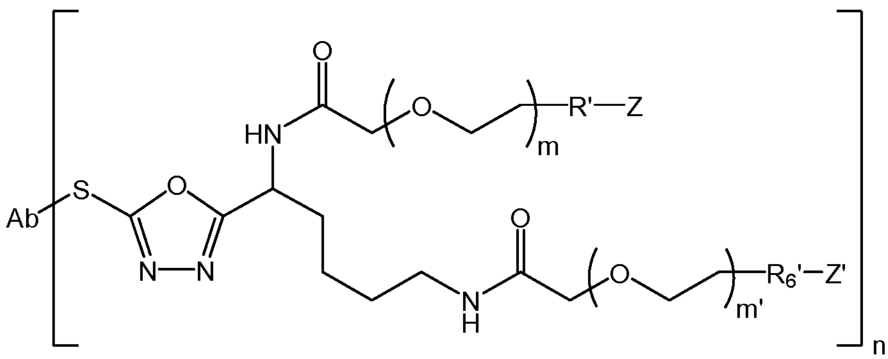

(8")

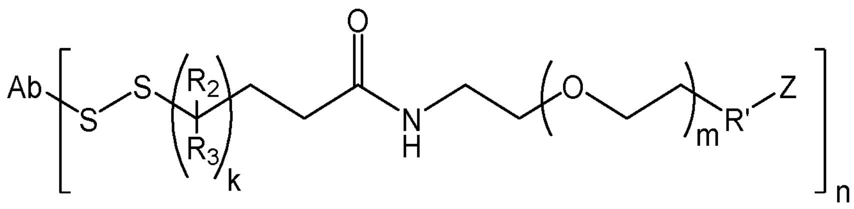

(9")

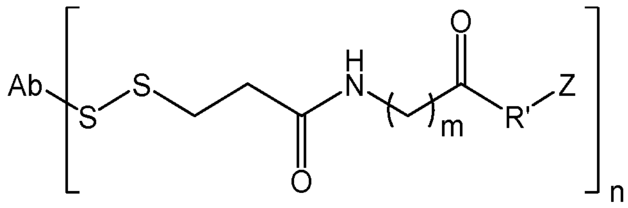

(10")

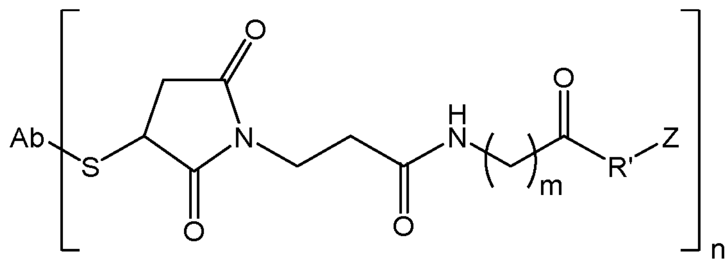

(11")

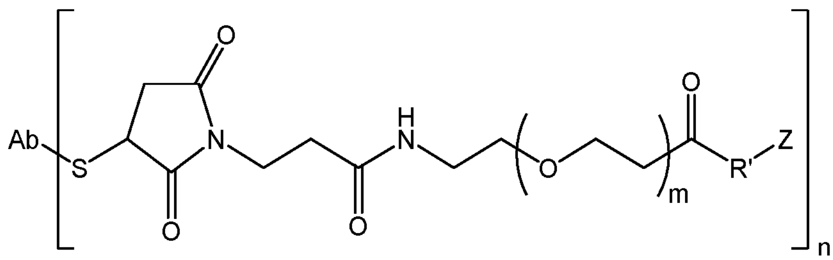

(12")

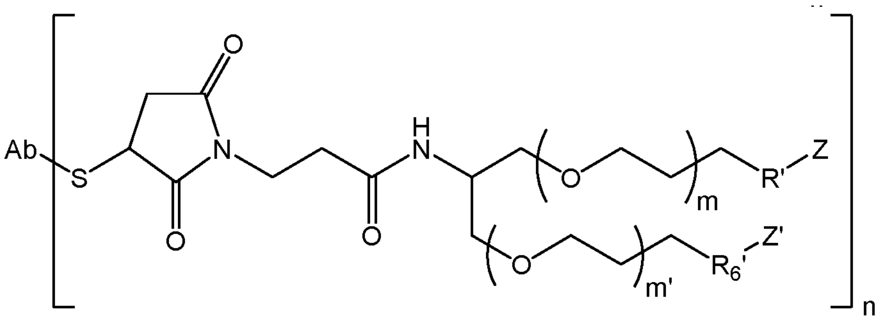

wherein

k is an integer of 0 or 1,
m is an integer from 1 to 5,
m' is an integer from 1 to 5,
$R_2$ and $R_3$ are each independently a hydrogen atom or methyl,
$R_6$' is a group produced by a reaction between two bioorthogonal functional groups capable of reacting with each other,
Z' is a functional substance, and
S, Q, R', Z and n adjacent to Ab, Ab are the same as defined in claim 26.

**28.** A method for producing a conjugate of an antibody and a functional substance or a salt thereof, comprising reacting an antibody intermediate or salt thereof having a bioorthogonal functional group which may be protected, which is represented by the following formula (I'), (II'), (III'), or (IV'):

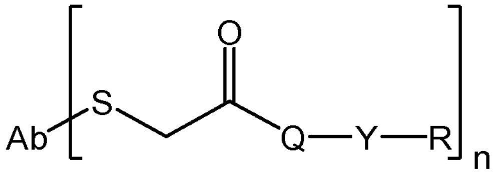

(I')

wherein

Ab is an antibody,
S is a sulfur atom,
Q is arylene or alkylene,
Y is a bond or a divalent group,
R is a bioorthogonal functional group which may be protected, and
n is an integer from 1 to 8,

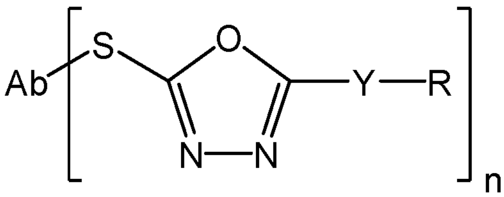

(II')

wherein
Ab is an antibody,
S is a sulfur atom,
Y is a bond or a divalent group,
R is a bioorthogonal functional group which may be protected, and
n is an integer from 1 to 8,

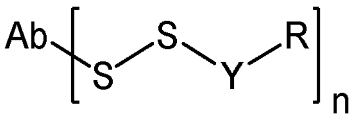

(III')

wherein
Ab is an antibody,
S is a sulfur atom,
Y is a bond or a divalent group,
R is a bioorthogonal functional group which may be protected, and
n is an integer from 1 to 8, or

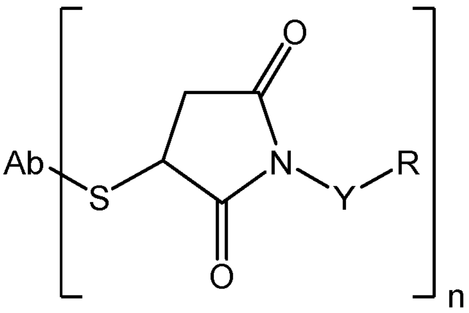

(IV')

wherein
Ab is an antibody,
S is a sulfur atom,
Y is a bond or a divalent group,
R is a bioorthogonal functional group which may be protected, and
n is an integer from 1 to 8,
with a functional substance having a bioorthogonal functional group capable of reacting with a bioorthogonal functional group carried by the antibody intermediate
to give the conjugate of the antibody and the functional substance or salt thereof,
wherein the conjugate is represented by the following formula (I"), (II"), (III"), or (IV") :

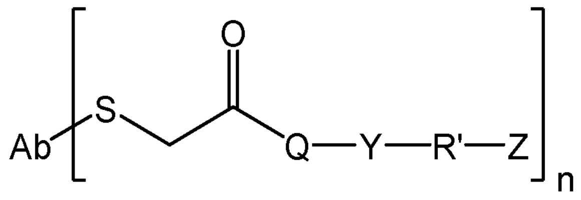
(I")

wherein
Ab, S adjacent to Ab, Q, Y, and n are the same as those of formula (I'),
R' is a group produced by the reaction between two bioorthogonal functional groups capable of reacting with each other, and
Z is a functional substance,

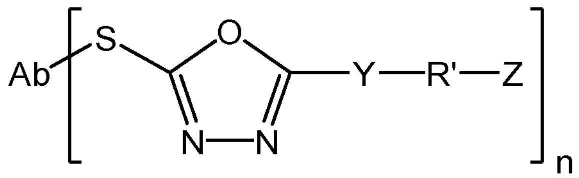
(II")

wherein
Ab, S adjacent to Ab, Y and n are the same as those of formula (II'),
R' is a group produced by the reaction between two bioorthogonal functional groups capable of reacting with each other, and
Z is a functional substance,

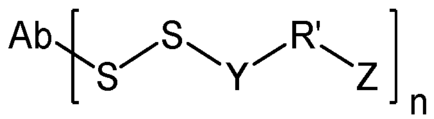
(III")

wherein
Ab, S adjacent to Ab, Y and n are the same as those of formula (III'),
R' is a group produced by the reaction between two bioorthogonal functional groups capable of reacting with each other, and
Z is a functional substance, or

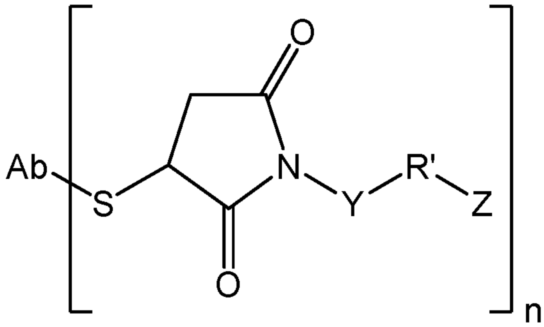

(IV")

wherein
Ab, S adjacent to Ab, Y and n are the same as those of formula (IV'),
R' is a group produced by the reaction between two bioorthogonal functional groups capable of reacting with each other, and
Z is a functional substance,
when the antibody intermediate or salt thereof has an alkyne residue, the antibody intermediate or salt thereof is reacted with a functional substance having an azide, or
when the antibody intermediate or salt thereof has an azide, the antibody intermediate or a salt thereof is reacted with a functional substance having an alkyne residue.

**29.** The method of claim 28, further comprising reacting a compound or salt thereof, wherein the compound is represented by the following formula (I), (II), (III), or (IV) :

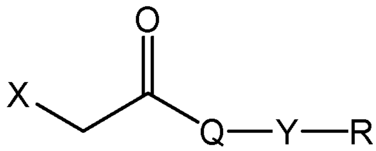

(I)

wherein

X is a halogen atom, and
Q, Y and R are the same as those of formula (I'),

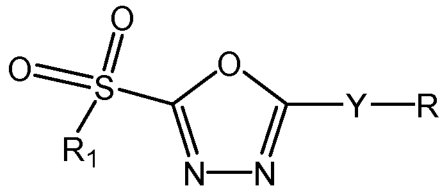

(II)

wherein
$R_1$ is alkyl, and
Y and R are the same as those of formula (II'),

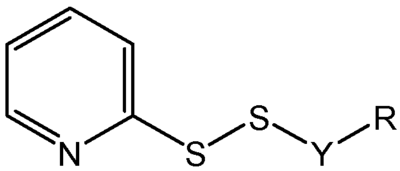

(III)

wherein
Y and R are the same as those of formula (III'), or

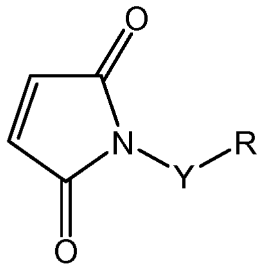

(IV)

wherein
Y and R are the same as those of formula (III').
with a thiol group-introduced antibody or salt thereof to give the antibody intermediate or salt thereof having a bioorthogonal functional group which may be protected, which is represented by the above formula (I), (II), (III), or (IV).

FIG. 1

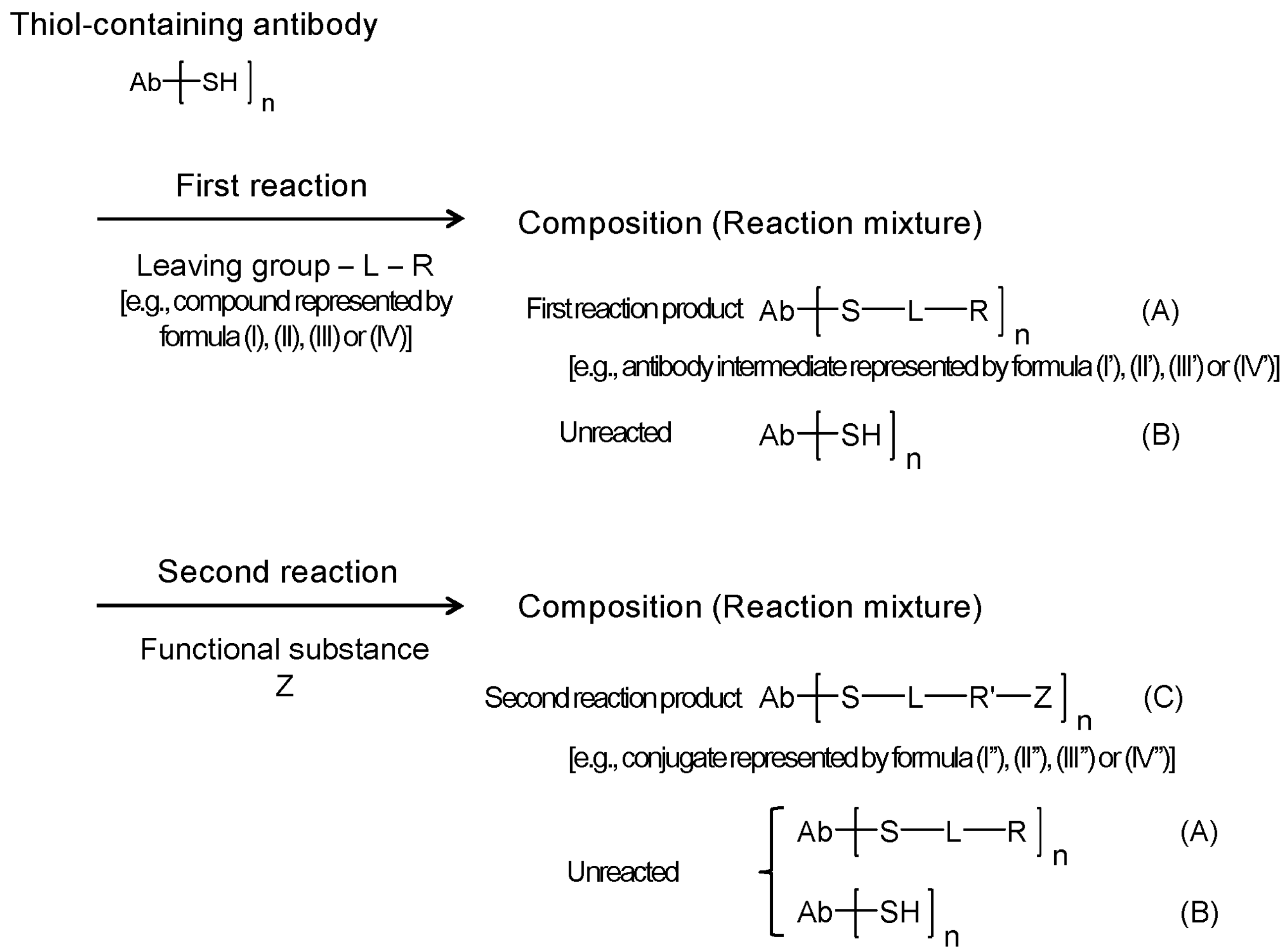
Thiol-containing antibody
First reaction
Leaving group – L – R
[e.g., compound represented by formula (I), (II), (III) or (IV)]
Composition (Reaction mixture)
First reaction product
(A)
[e.g., antibody intermediate represented by formula (I'), (II'), (III') or (IV')]
Unreacted
(B)
Second reaction
Functional substance
Z
Composition (Reaction mixture)
Second reaction product
(C)
[e.g., conjugate represented by formula (I"), (II"), (III") or (IV")]
Unreacted
(A)
(B)

FIG. 2

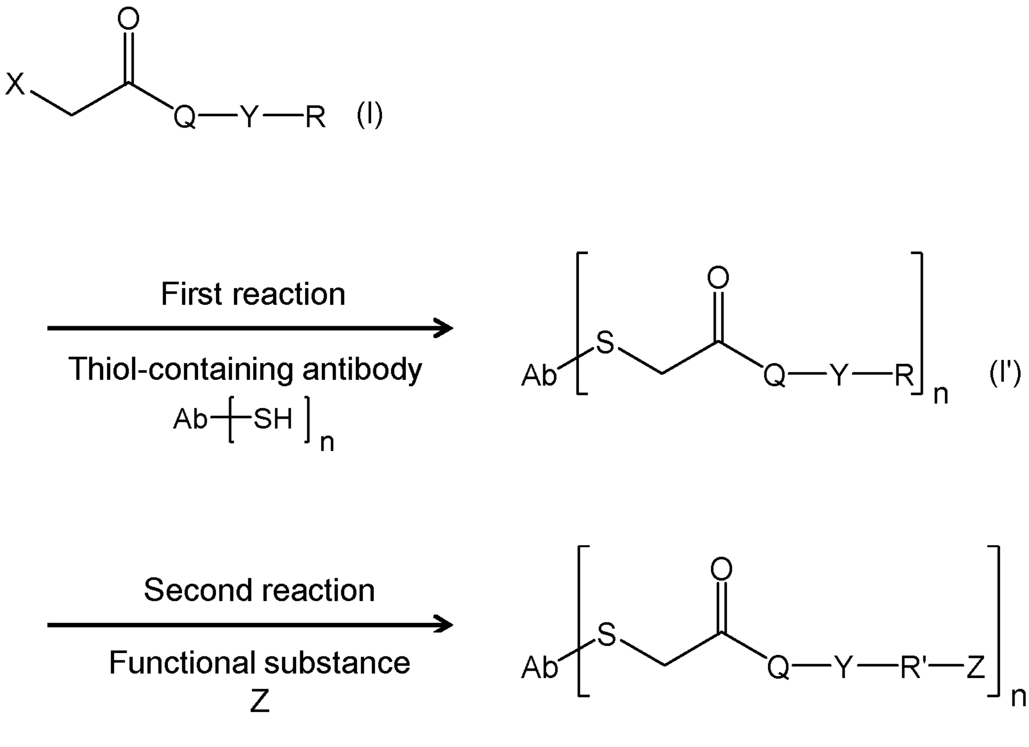
First reaction
Thiol-containing antibody
Second reaction
Functional substance
Z

(I) (I') (I'')

# FIG. 3

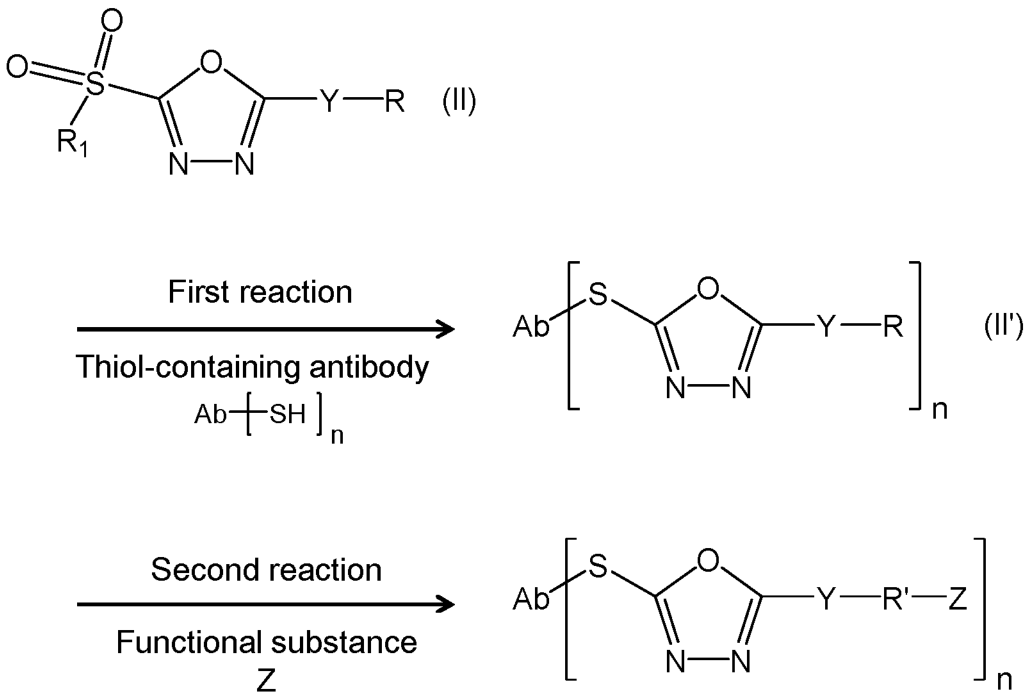
First reaction
Thiol-containing antibody
Second reaction
Functional substance
Z

(II)

(II')

(II'')

FIG. 4

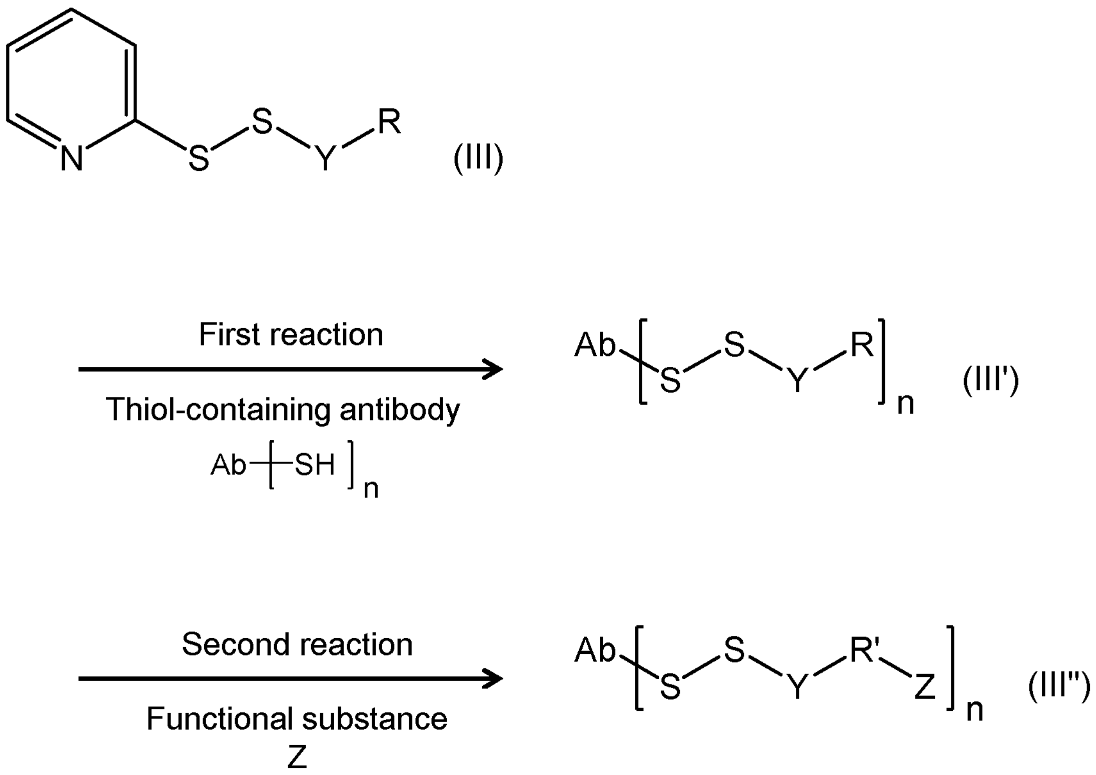


(III)

(III')

(III")

FIG. 5

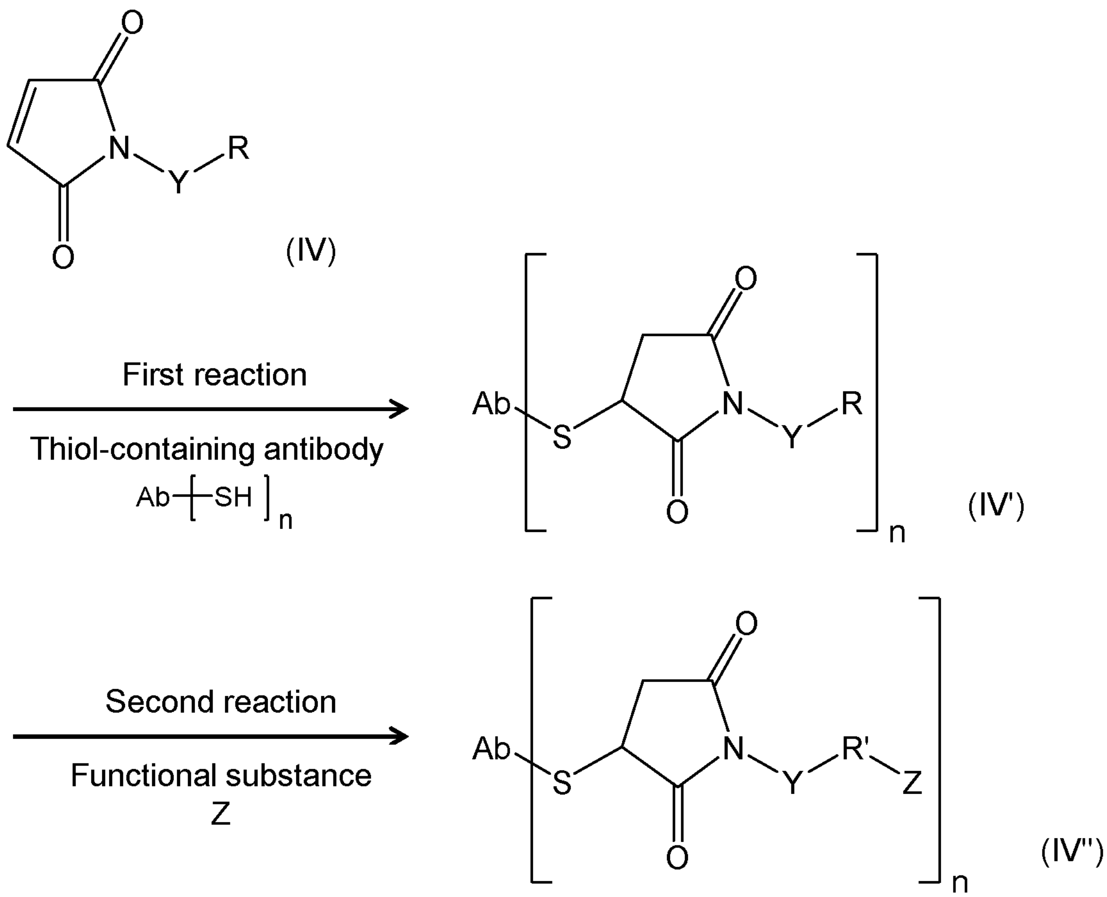
First reaction
Thiol-containing antibody
Second reaction
Functional substance
Z

(IV)

(IV')

(IV'')

# FIG. 6

(1) Amino acid sequence of heavy chain of trastuzumab
EVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRW
GGDGFYAMDYWGQGTLVTVSSASTKGPSVFPLAPSSKSTSGGTAALGCL
VKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPP
KPKDTLMISRTPEVTCVVVDVSHEDPEVKFNWYVDGVEVHNAKTKPREE
QYDSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLS
LSPG (SEQ ID NO: 1)

(2) Amino acid sequence of light chain of trastuzumab
DIQMTQSPSSLSASVGDRVTITCRASQDVNTAVAWYQQKPGKAPKLLIYS
ASFLYSGVPSRFSGSRSGTDFTLTISSLQPEDFATYYCQQHYTTPPTFGQ
GTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQ
GLSSPVTKSFNRGEC (SEQ ID NO: 2)

FIG. 7

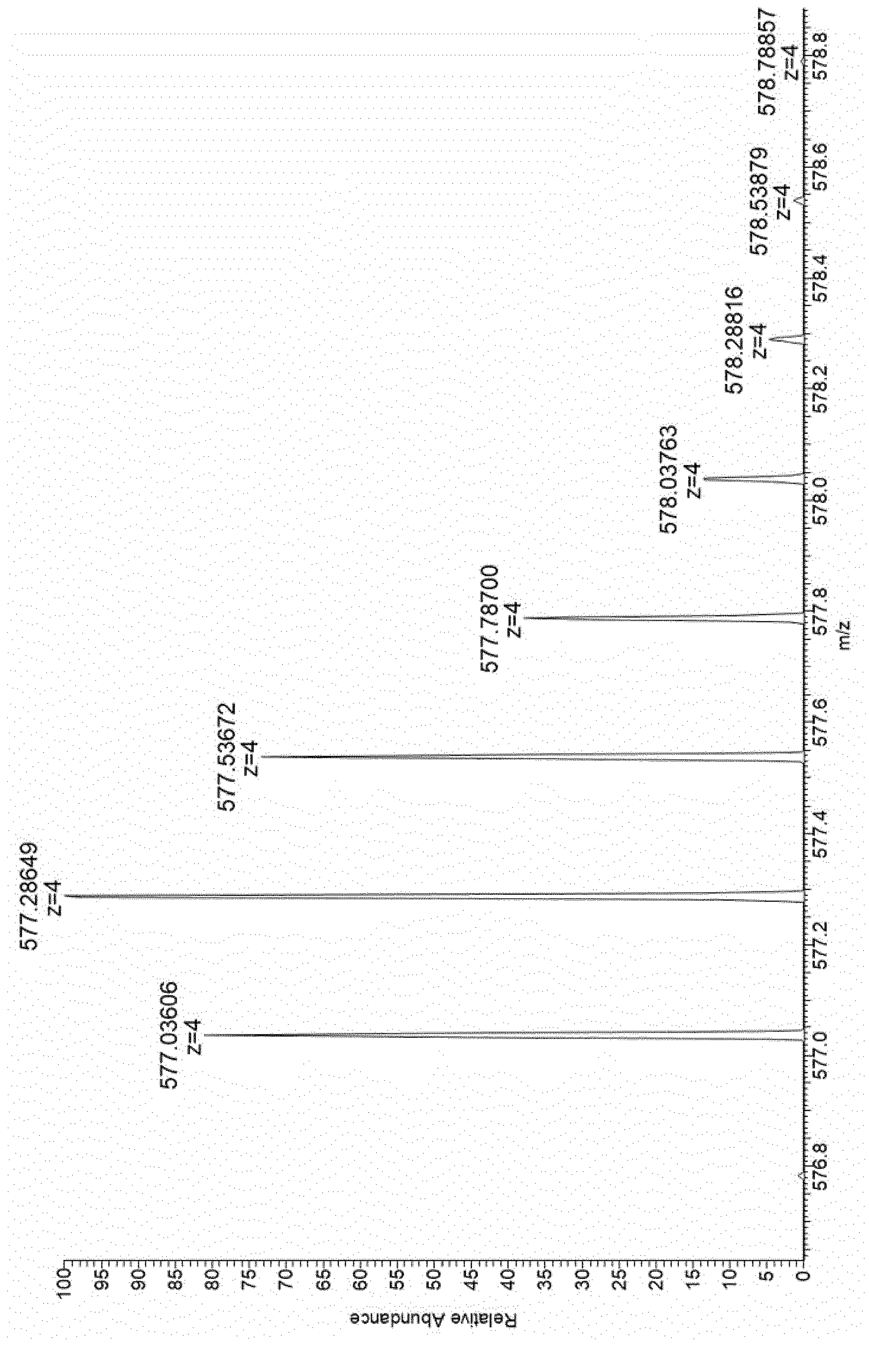
577.03606
z=4
577.28649
z=4
577.53672
z=4
577.78700
z=4
578.03763
z=4
578.28816
z=4
578.53879
z=4
578.78857
z=4
Relative Abundance
m/z
0
5
10
15
20
25
30
35
40
45
50
55
60
65
70
75
80
85
90
95
100
576.8
577.0
577.2
577.4
577.6
577.8
578.0
578.2
578.4
578.6
578.8

FIG. 8

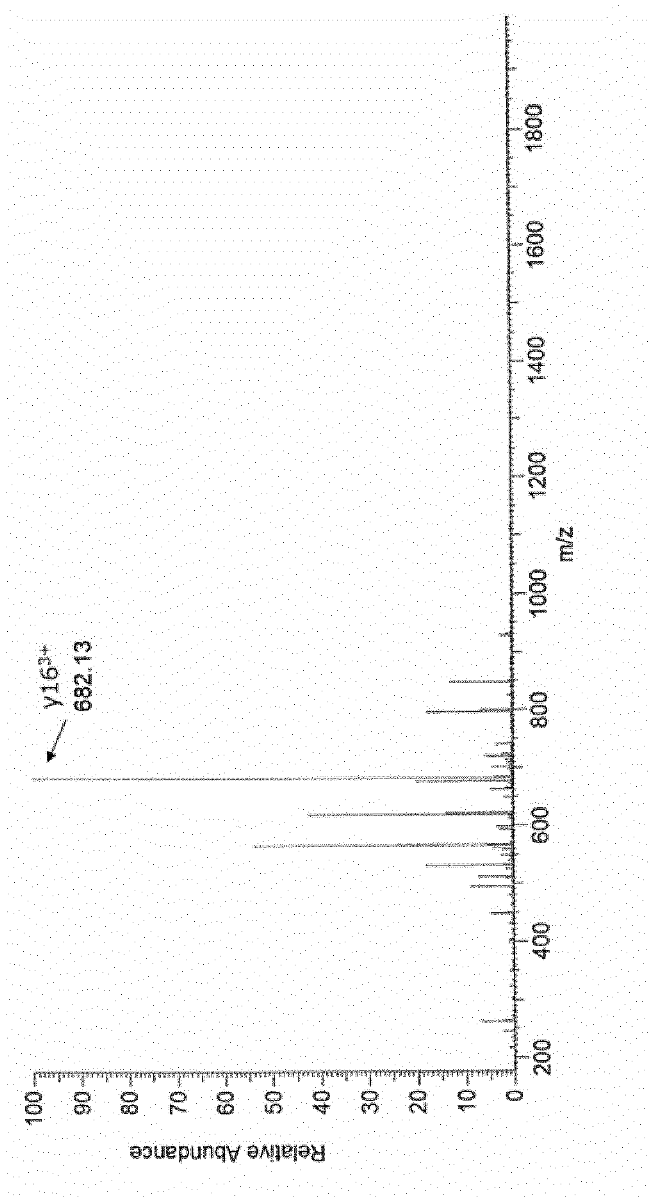
y16$^{3+}$
682.13
Relative Abundance
100
90
80
70
60
50
40
30
20
10
0
200
400
600
800
1000
1200
1400
1600
1800
m/z

FIG. 9

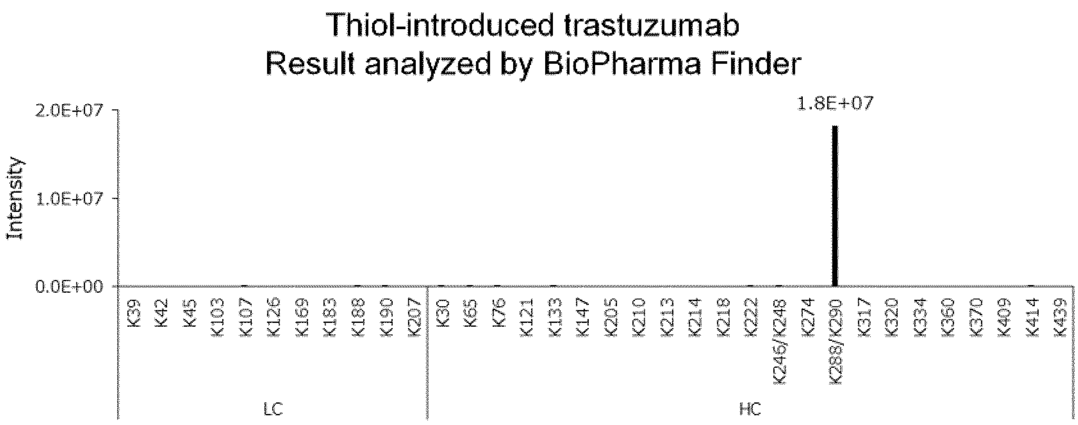
Thiol-introduced trastuzumab
Result analyzed by BioPharma Finder
1.8E+07
2.0E+07
1.0E+07
0.0E+00
Intensity
K39
K42
K45
K103
K107
K126
K169
K183
K188
K190
K207
K30
K65
K76
K121
K133
K147
K205
K210
K213
K214
K218
K222
K246/K248
K274
K288/K290
K317
K320
K334
K360
K370
K409
K414
K439
LC
HC

FIG. 10

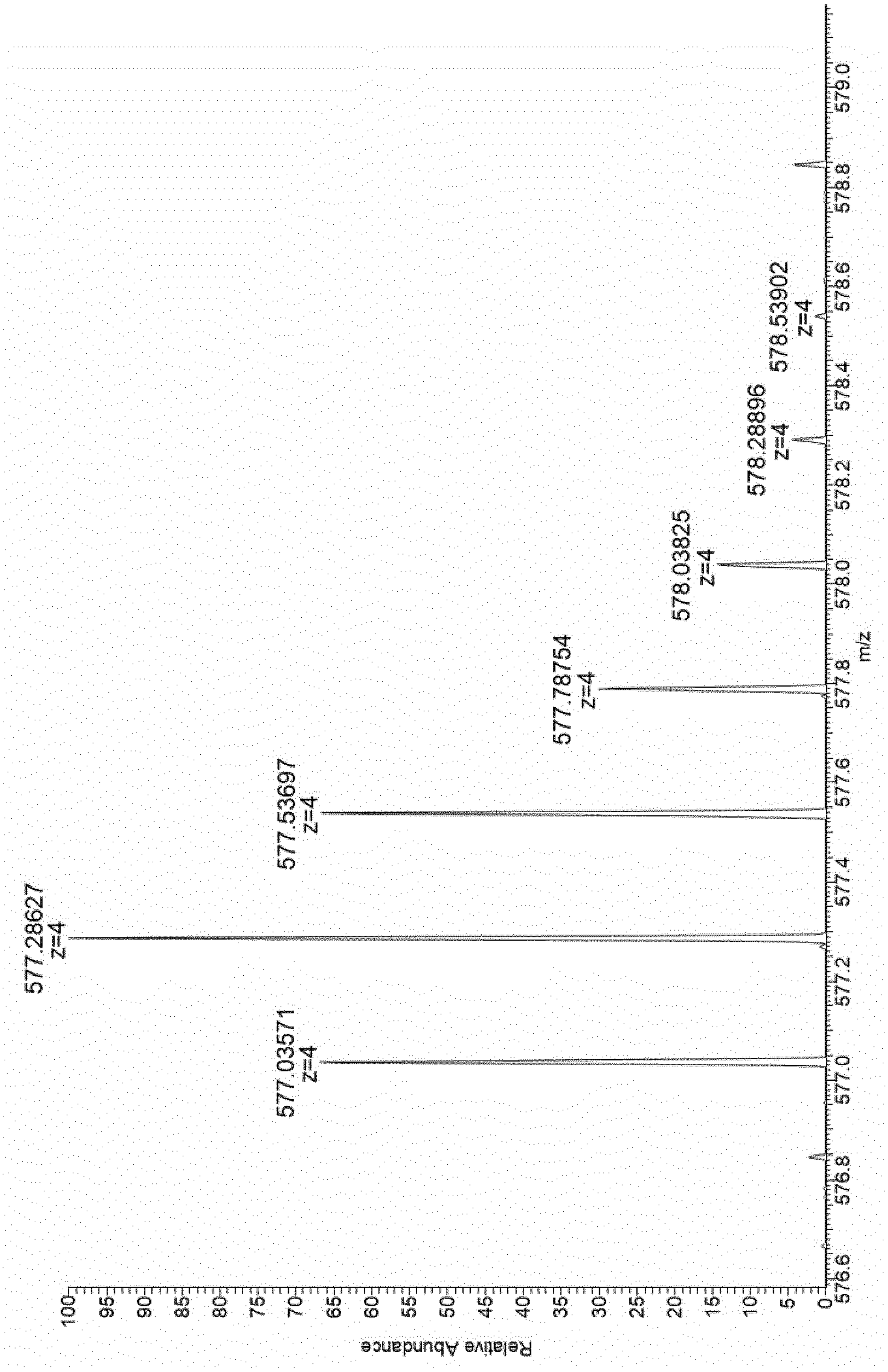
577.03571
z=4
577.28627
z=4
577.53697
z=4
577.78754
z=4
578.03825
z=4
578.28896
z=4
578.53902
z=4
Relative Abundance
m/z
100
95
90
85
80
75
70
65
60
55
50
45
40
35
30
25
20
15
10
5
0
576.6
576.8
577.0
577.2
577.4
577.6
577.8
578.0
578.2
578.4
578.6
578.8
579.0

FIG. 11

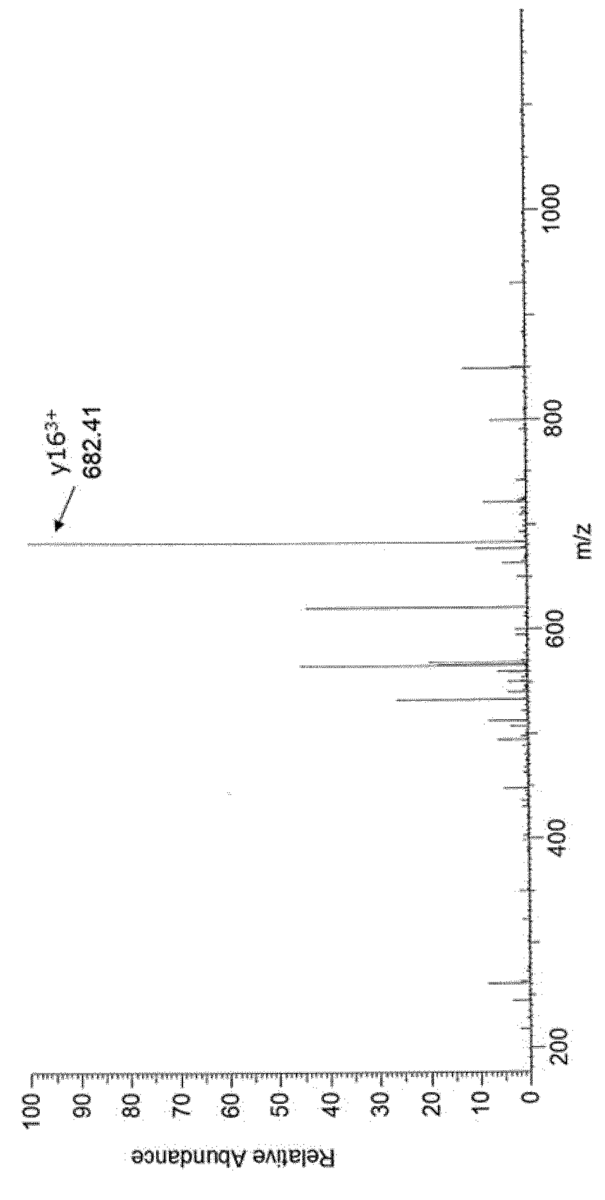
y16³⁺
682.41
Relative Abundance
m/z
0
10
20
30
40
50
60
70
80
90
100
200
400
600
800
1000

FIG. 12

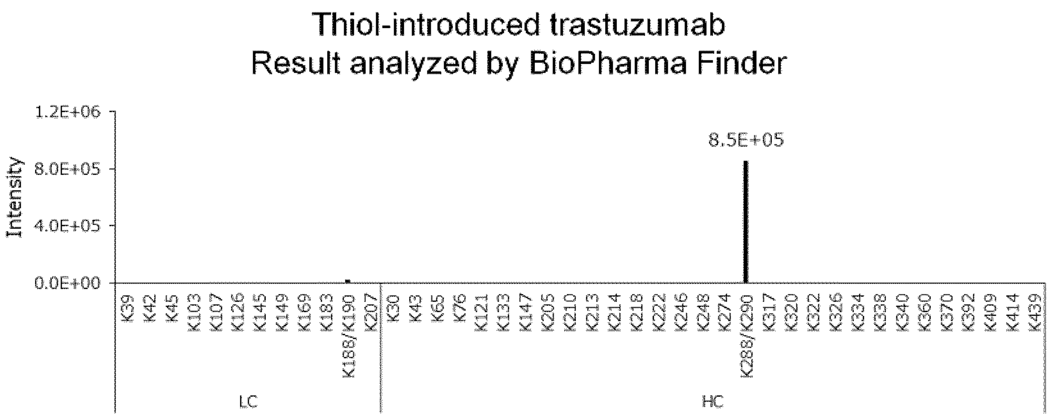
Thiol-introduced trastuzumab
Result analyzed by BioPharma Finder
Intensity
1.2E+06
8.0E+05
4.0E+05
0.0E+00
8.5E+05
K39
K42
K45
K103
K107
K126
K145
K149
K169
K183
K188/K190
K207
K30
K43
K65
K76
K121
K133
K147
K205
K210
K213
K214
K218
K222
K246
K248
K274
K288/K290
K317
K320
K322
K326
K334
K338
K340
K360
K370
K392
K409
K414
K439
LC
HC

FIG. 13

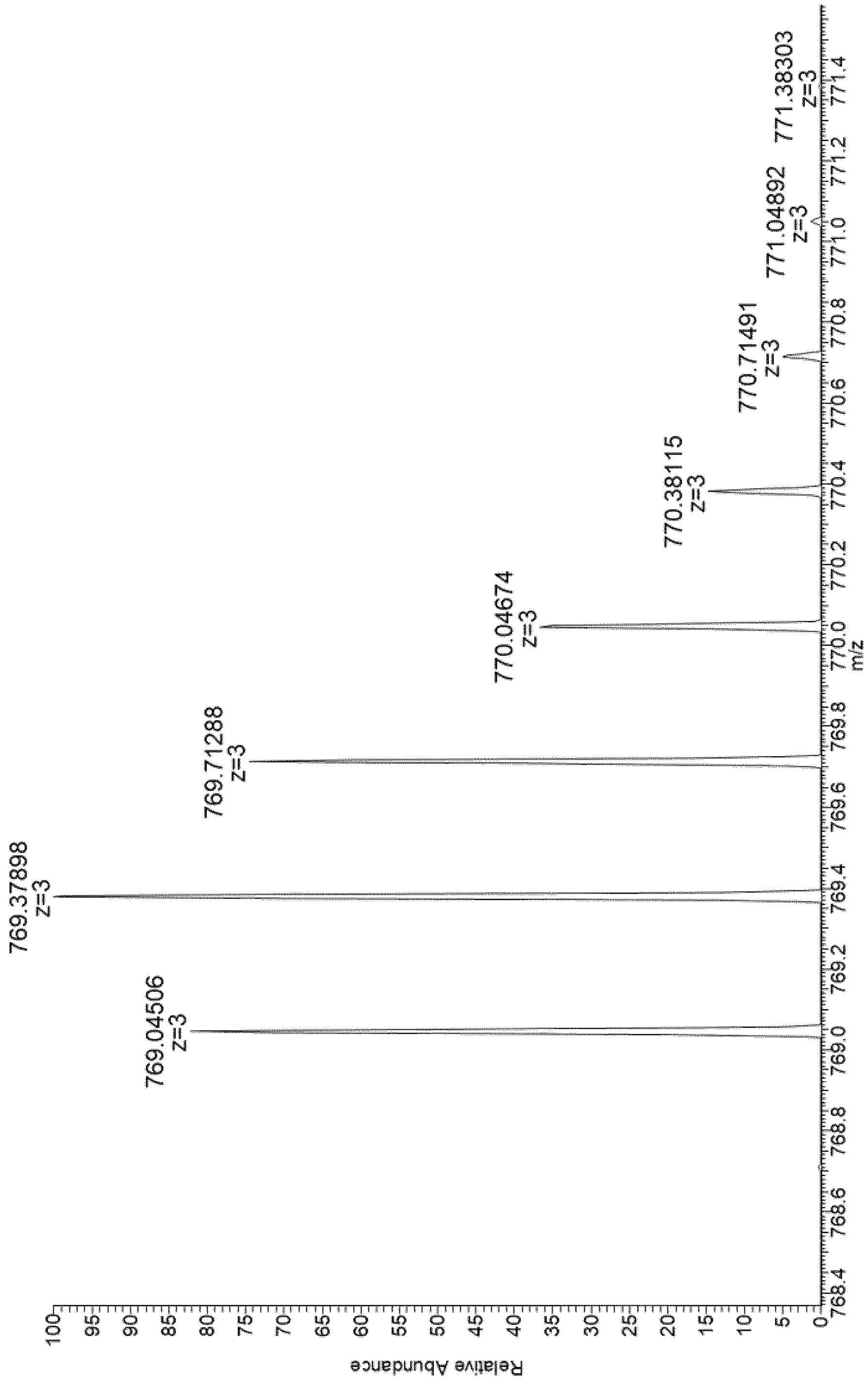
769.04506
z=3
769.37898
z=3
769.71288
z=3
770.04674
z=3
770.38115
z=3
770.71491
z=3
771.04892
z=3
771.38303
z=3
Relative Abundance
m/z

FIG. 14

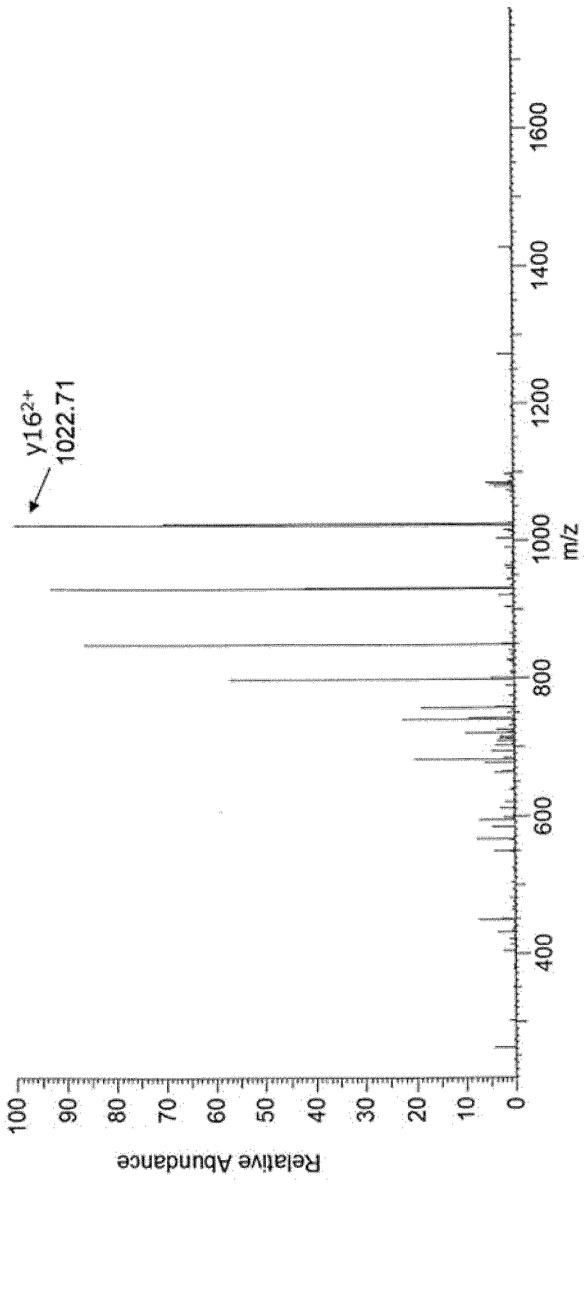
y16$^{2+}$
1022.71
Relative Abundance
100
90
80
70
60
50
40
30
20
10
0
400
600
800
1000
1200
1400
1600
m/z

FIG. 15

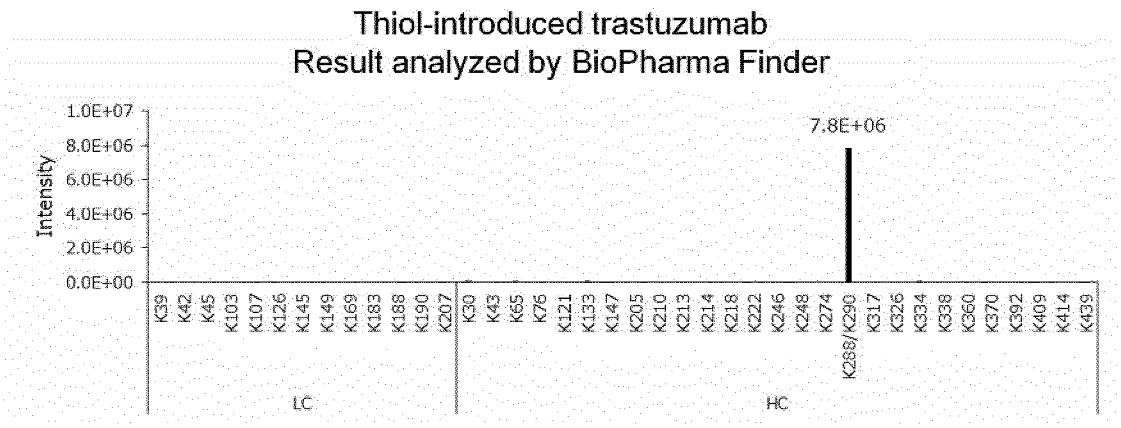
Thiol-introduced trastuzumab
Result analyzed by BioPharma Finder
Intensity
1.0E+07
8.0E+06
6.0E+06
4.0E+06
2.0E+06
0.0E+00
7.8E+06
K39
K42
K45
K103
K107
K126
K145
K149
K169
K183
K188
K190
K207
K30
K43
K65
K76
K121
K133
K147
K205
K210
K213
K214
K218
K222
K246
K248
K274
K288/K290
K317
K326
K334
K338
K360
K370
K392
K409
K414
K439
LC
HC

**INTERNATIONAL SEARCH REPORT**

International application No. **PCT/JP2022/001617**

**A. CLASSIFICATION OF SUBJECT MATTER**

***C07K 16/00***(2006.01)i; ***A61K 39/395***(2006.01)i; ***A61K 47/54***(2017.01)i; ***A61K 47/65***(2017.01)i; ***A61K 47/68***(2017.01)i; ***A61P 43/00***(2006.01)i; ***C07K 1/00***(2006.01)i; ***C07K 14/00***(2006.01)i; ***C07K 19/00***(2006.01)i; ***C12N 15/13***(2006.01)i; ***C12P 21/08***(2006.01)i

FI: C07K16/00 ZNA; A61K39/395 N; A61K47/54; A61K47/65; A61K47/68; A61P43/00 111; C07K1/00; C07K14/00; C07K19/00; C12N15/13; C12P21/08

According to International Patent Classification (IPC) or to both national classification and IPC

**B. FIELDS SEARCHED**

Minimum documentation searched (classification system followed by classification symbols)

C07K16/00; A61K39/395; A61K47/54; A61K47/65; A61K47/68; A61P43/00; C07K1/00; C07K14/00; C07K19/00; C12N15/13; C12P21/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | KONIEV, Oleksandr et al. MAPN: First-in-Class Reagent for Kinetically Resolved Thiol-to-Tiol Conjugation. Bioconjugate Chem. 2015, vol. 26, pp. 1863-1867<br>fig. 2 | 1-6, 8-15, 17, 19-23, 25-26, 28-29 |
| Y | fig. 2 | 1-29 |
| X | JEONG, Sin-Young et al. Highly robust and optimized conjugation of antibodies to nanoparticles using quantitatively validated protocols. Nanoscale. 2017, vol. 9, issue 7, pp. 2548-2555<br>fig. 1 | 1-2, 5-6, 8-15, 17, 19, 22-23, 25-26, 28-29 |
| Y | fig. 1 | 1-29 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 March 2022** | **22 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/001617**

**C. DOCUMENTS CONSIDERED TO BE RELEVANT**

| Category* | Citation of document, with indication, where appropriate, of the relevant passages | Relevant to claim No. |
|---|---|---|
| X | LEE, Byeong-Sung et al. Construction of an immunotoxin via site-specific conjugation of anti-Her2 IgG and engineered Pseudomonas exotoxin A. Journal of Biological Engineering. 2019, vol. 13, no. 56, pp. 1-13<br>scheme 1 | 1-2, 5-6, 8-15, 17, 19, 22-23, 25-26, 28-29 |
| Y | scheme 1 | 1-29 |
| X | JP 11-514223 A (IMMUNOMEDICS, INC) 07 December 1999 (1999-12-07)<br>B.2. | 1-2, 5-7, 10-17, 19, 22-29 |
| Y | B.2. | 1-29 |
| X | JP 2020-121981 A (ECOLE POLYTECHNIQUE FEDERALE DE LAUSANNE (EPFL)) 13 August 2020 (2020-08-13)<br>reaction formulas 9, 10 | 1-2, 5-6, 8-15, 17, 19, 22-23, 25-26, 28-29 |
| Y | reaction formulas 9, 10 | 1-29 |
| X | JP 2008-539270 A (VENTANA MEDICAL SYSTEMS, INC) 13 November 2008 (2008-11-13)<br>claims 7, 8, 18, example A | 1-2, 5-7, 10-19, 22-29 |
| Y | claims 7, 8, 18, example A | 1-29 |
| X | JP 2011-508194 A (GENERAL ELECTRIC COMPANY) 10 March 2011 (2011-03-10)<br>claims, paragraph [0031] | 10-17, 26 |
| Y | claims, paragraph [0031] | 1-29 |
| X | WO 2008/079897 A2 (THE REGENTS OF THE UNIVERSITY OF CALIFORNIA) 03 July 2008 (2008-07-03)<br>table 1 | 10-18, 26-27 |
| Y | table 1 | 1-29 |
| X | JP 2017-503777 A (NOVARTIS AG) 02 February 2017 (2017-02-02)<br>example 59 | 10-18, 26-27 |
| Y | example 59 | 1-29 |
| X | CN 112098640 A (ZHEJIANG ZHENGXI BIOLOGICAL MEDICINE CO LTD) 18 December 2020 (2020-12-18)<br>claims | 10-18 |
| Y | claims | 1-29 |
| X | WO 2014/144878 A2 (THE SCRIPPS RESEARCH INSTITUTE) 18 September 2014 (2014-09-18)<br>claims, paragraph [0353] | 10-18 |
| Y | claims, paragraph [0353] | 1-29 |
| Y | WO 2019/240287 A1 (AJINOMOTO KK) 19 December 2019 (2019-12-19)<br>claims, examples | 1-29 |

Form PCT/ISA/210 (second sheet) (January 2015)

**INTERNATIONAL SEARCH REPORT**

International application No.
**PCT/JP2022/001617**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/001617**

| Patent document cited in search report | Publication date (day/month/year) | Patent family member(s) | Publication date (day/month/year) |
|---|---|---|---|
| JP 11-514223 A | 07 December 1999 | WO 1997/011370 A1<br>B.2. | |
| JP 2020-121981 A | 13 August 2020 | WO 2015/140648 A2<br>reaction scheme 9, 10<br>US 2016/0015821 A1<br>US 2016/0243248 A1 | |
| JP 2008-539270 A | 13 November 2008 | US 2006/0246523 A1<br>WO 2006/116628 A2<br>claims 7, 8, 18, example A<br>US 2006/0246524 A1<br>US 2009/0176253 A1<br>US 2009/0181398 A1<br>US 2014/0147906 A1<br>US 2016/0187324 A1<br>WO 2006/116742 A2<br>EP 3144675 A1 | |
| JP 2011-508194 A | 10 March 2011 | JP 5566905 B2<br>WO 2009/080561 A1<br>claims, p. 7<br>US 2008/0161537 A1<br>EP 2591808 A2 | |
| WO 2008/079897 A2 | 03 July 2008 | US 2008/0171793 A1 | |
| JP 2017-503777 A | 02 February 2017 | US 2016/0311853 A1<br>example 59<br>US 2018/0244717 A1<br>US 2019/0382441 A1<br>WO 2015/095301 A2<br>EP 3753578 A1 | |
| CN 112098640 A | 18 December 2020 | (Family: none) | |
| WO 2014/144878 A2 | 18 September 2014 | (Family: none) | |
| WO 2019/240287 A1 | 19 December 2019 | US 2021/0139549 A1<br>claims, examples<br>EP 3808760 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015001117 A
- WO 2014144878 A
- WO 2004010957 A
- US 20060074008 A
- US 200502386649 A
- US 6214345 B
- US 5622929 A
- US 5122368 A
- US 5824805 A
- WO 02083180 A
- WO 04043493 A
- WO 051192919 A
- WO 2018199337 A
- WO 2019240288 A
- WO 2019240287 A
- WO 2020090979 A
- WO 2019240287 A1
- WO 2019240288 A1
- WO 2018218004 A1


### Non-patent literature cited in the description


- **SHARPLESS K. B et al.** *Angew. Chem. Int. Ed.,* 2015, vol. 40, 2004
- **BERTOZZI C. R. et al.** *Science,* 2001, vol. 291, 2357
- **BERTOZZI C. R. et al.** *Nature Chemical Biology,* 2005, vol. 1, 13
- **G. LERICHE ; L. CHISHOLM ; A. WAGNER.** *Bioorganic & Medicinal Chemistry.,* 2012, vol. 20, 571
- **FENG P. et al.** *Jounal of American Chemical Society,* 2010, vol. 132, 1500
- **BESSODES M. et al.** *Journal of Controlled Release,* 2004, vol. 99, 423
- **DESIMONE, J.M.** *Journal of American Chemical Society,* 2010, vol. 132, 17928
- **THOMPSON, D.H.** *Journal of Controlled Release,* 2003, vol. 91, 187
- **SCHOENMARKS, R.G.** *Journal of Controlled Release,* 2004, vol. 95, 291
- **DUBOWCHIK et al.** *Pharma. Therapeutics,* 1999, vol. 83, 67-123
- *ACS Omega,* 2020, vol. 5, 7193-7200
- **G.I.J.L. BERNARDES et al.** *Chem. Rev.,* 2015, vol. 115, 2174
- **J.L. BERNARDES et al.** *Chem. Asian. J.,* 2009, vol. 4, 630
- **B. G. DEVICES et al.** *Nat. Commun,* 2014, vol. 5, 4740
- **WAGNER et al.** *Bioconjugate. Chem.,* 2014, vol. 25, 825
- **B. G. DEVICES et al.** *Nat. Commun.,* 2014, vol. 5, 4740
- **WAGNER et al.** *Bioconjugate. Chem,* 2014, vol. 25, 825
- *Anal. Chem.,* 2019, vol. 91 (20), 12724-12732
- *Anal. Chem,* 2019, vol. 91 (20), 12724-12732
- *CHEMICAL ABSTRACTS,* 159857-81-5